# EUROPEAN PATENT APPLICATION

(11) **EP 4 450 098 A1**
(43) Date of publication of application: **23.10.2024**
(21) Application number: 23168342.6
(22) Date of filing: 17.04.2023
(51) Int. Cl.: A61M 5/20, A61M 5/315

(54) **MECHANISM FOR AN AUTOMATIC MEDICAMENT DELIVERY DEVICE**

(71) Applicant: medmix Switzerland AG, 9469 Haag (Rheintal) (CH)
(72) Inventor: Muenzer, Chris, 4051 Basel (CH)
(74) Representative: Manitz Finsterwald Patent- und Rechtsanwaltspartnerschaft mbB

(57) **Abstract**

A mechanism for an automatic medicament delivery device comprises a housing; a dose setting element; a button; a piston rod that is rotationally fixed and axially movable with respect to the housing; a nut; and a spring. The dose setting element is configured to be gripped by a user of the device to set a dose to be delivered by rotation of the dose setting element with respect to the housing when the mechanism is in a dose setting state, wherein the rotation of the dose setting element stores energy in the spring. Furthermore, the rotation of the dose setting element causes the nut to move by a dose distance in a proximal direction with respect to the piston rod, the dose distance being proportional to the dose. The mechanism further is configured to switch from the dose setting state into a dose delivery state upon moving the button, for example in an axial direction, with respect to the housing. A transfer into the dose delivery state couples the spring to the nut to cause automatic movement of the nut in a proximal direction by releasing the energy stored during dose setting. The piston rod is configured to move together with the nut by the dose distance in the proximal direction to deliver the dose when the mechanism is in the dose delivery state. Furthermore, the dose setting element is configured to remain axially stationary with respect to the housing when the dose is being delivered.

## Description

### FIELD

The present disclosure relates to a mechanism for an automatic medicament delivery device and a medicament delivery device having the mechanism.

### BACKGROUND

Medicament delivery devices, such as injection devices, are used to deliver a liquid medicament to a patient. Such medicament delivery devices usually comprise a medicament container holding the medicament and a mechanism configured to expel a predefined dose of medicament from that container. At a distal side facing away from the delivery site, the medicament container usually comprises a movable plunger, which plunger seals the medicament container and which plunger is moved in a proximal direction towards the injection site to expel the medicament from the container. To move the plunger, the mechanism usually comprises a piston rod that acts on the plunger by moving the plunger in the proximal direction. The dose to be delivered then is defined by the axial movement of the piston rod and the plunger within the medicament container.

When being configured as automatic medicament delivery devices, the force to cause axial movement of the plunger is entirely provided by an energy storage of the mechanism and the dose of medicament is automatically delivered once a user has triggered the mechanism for dose delivery. Usually, such mechanisms comprise a release element, such as a button, that is actuated by a user to trigger dose delivery.

Some types of automatic medicament delivery devices provide a dose setting functionality that prepares the device for automatic injection by storing energy in the energy storage and by defining the amount of medicament to be delivered during dose delivery. Such devices thereby may be configured as fixed dose devices that allow only a single dose or amount of medicament to be set, or they may be configured as variable dose devices that allow a user to select from a multitude of predefined doses, such as from two or more predefined doses.

Document WO 2020/015980 A1 describes, inter alia, an automatic medicament delivery device that has a knob provided at a distal end of the device, which knob is configured for dose setting and for triggering dose delivery. During dose setting, a user of the device rotates the knob. This causes the knob to move distally away from the housing of the device by a distance that is proportional to the set dose. Furthermore, rotation of the knob strains a torsion spring provided within the device.

To initiate dose delivery, the user pushes the knob in the proximal direction. This causes the spring to relax and to thereby drive dose delivery. During dose delivery, the knob moves back towards the housing by the distance it previously had been traveled away from the housing during dose setting.

The device described in document WO 2020/015980 A1 thus comprises movable components, such as the knob, that are accessible to a user of the device and that have to move during dose delivery. This entails the risk that a user may interfere with these components during dose delivery and thereby may disturb medicament delivery.

Furthermore, the device described in document WO 2020/015980 A1 comprises a single actuation member in the form of the knob that is used for both dose setting and for initiating automatic dose delivery. This may complicate the use of the device since the user has to avoid to inadvertently trigger dose delivery by pushing the knob when trying to change the dose setting.

It is thus an objective of the present disclosure to provide a mechanism for an automatic medicament delivery device and a medicament delivery device that allow for user-friendly and safe dose setting and dose delivery.

### SUMMARY

The present disclosure provides a mechanism for an automatic medicament delivery device and a medicament delivery device. Embodiments are given in the dependent claims, the description and the drawings.

In one aspect, the present disclosure is directed at a mechanism for an automatic medicament delivery device comprising a housing; a dose setting element; a button; a piston rod that is rotationally fixed and axially movable with respect to the housing; a nut; and a spring. The dose setting element is configured to be gripped by a user of the device to set a dose to be delivered by rotation of the dose setting element with respect to the housing when the mechanism is in a dose setting state, wherein the rotation of the dose setting element stores energy in the spring. Furthermore, the rotation of the dose setting element causes the nut to move by a dose distance in a proximal direction with respect to the piston rod, the dose distance being proportional to the dose. The mechanism further is configured to switch from the dose setting state into a dose delivery state upon moving the button, for example in an axial direction, with respect to the housing. A transfer into the dose delivery state couples the spring to the nut to cause automatic movement of the nut in a proximal direction by releasing the energy stored during dose setting. The piston rod thereby is configured to move together with the nut by the dose distance in the proximal direction to deliver the dose when the mechanism is in the dose delivery state. Furthermore, the dose setting element is configured to remain axially stationary with respect to the housing when the dose is being delivered.

The mechanism for an automatic medicament delivery device provides a dose setting element that is not required to axially move during dose delivery and therefore reduces the risk of a user of the device interfering with components that move during automatic medicament delivery. This provides a user-friendly and safe mechanism.

With the mechanism, delivery of the medicament may not require axial movement of the dose setting element, at least after having transferred the mechanism to the dose delivery state. In general, the dose setting element may however be axially movable with respect to the housing. For example, the dose setting element may be configured to axially move upon triggering automatic injection and/or upon interrupting automatic injection. Furthermore, delivery of the dose may already start while the dose setting element is still moved into its fully triggered position and/or delivery of the dose may still last while the dose setting element is starting to move away from the fully triggered position to interrupt automatic injection.

The dose setting element being configured to remain axially stationary with respect to the housing when the piston rod moves in the proximal direction to deliver the dose thus encompasses that the dose setting element is configured to remain axially stationary with respect to the housing during at least a part of the movement of the piston rod in the proximal direction during dose delivery.

The mechanism having a nut that is moved with respect to the piston rod in a distal direction during dose setting and moves with the piston rod in the proximal direction during dose delivery provides a mechanically simple and compact actuation arrangement for the piston rod.

The dose setting element may comprise a gripping surface that is configured to be gripped by the user. The gripping surface may be an outer surface of the dose setting element. Furthermore, the gripping surface may be configured as a structured surface to enhance friction upon gripping the dose setting element. For example, the gripping surface may comprise grooves and/or ridges. The dose setting element may comprise a cylindrical shape. The gripping surface may be an outer cylindrical surface and/or an outer circumferential surface of the dose setting element.

The dose setting element may be configured to be rotated around a longitudinal axis of the mechanism during dose setting.

The mechanism may be configured to provide a force from the spring during dose delivery that is sufficient to automatically deliver the set dose. Such a mechanism does not rely on a force that is provided by a user of the mechanism to advance the piston rod. For example, the mechanism may be configured to prevent a force exerted by a user onto the button during dose delivery from being transferred to the piston rod.

The spring may be indirectly coupled to the nut in the dose delivery state, for example by one or more intermediate members. These intermediate members may be configured to convert a torque provided by the spring to axial movement of the nut. For example, the intermediate member or one of the intermediate members may be configured to axially push upon the nut during dose delivery. The intermediate member may be formed by a driver of the mechanism.

The housing may form an outer housing or outer enclosure of the mechanism. It may comprise a single component or several components that are permanently fixed to each other. The housing may surround at least parts of the mechanism.

The housing may have an elongated shape that extends along the longitudinal axis. It may have a generally cylindrical shape. The piston rod may also extend along the longitudinal axis.

The mechanism may comprise a dose definition mechanism that allows a user of the device to set at least one dose of medicament for delivery. For example, the dose definition mechanism may be configured to allow only a single predetermined dose to be set. Alternatively, the dose definition mechanism may also be configured to allow a multitude of differing predetermined doses to be set by the user, such as two or more differing doses.

The mechanism may be configured as a single-use mechanism that allows to set a dose only once and subsequently prevents a user from setting and delivering further doses. The mechanism may also be configured as a multi-use mechanism that allows to repeatedly set doses for delivery.

The housing may be configured to connect to a medicament container that contains the medicament to be delivered. For example, the housing may have a connector that allows attachment of a separate container holder comprising the medicament container to the housing. The connector may be, for example, configured as a form fit, such as a snap-fit connector or a threaded connector, or as an adhesive bond, such as a welded or glued connection. Alternatively, the housing may also connect to the medicament container by comprising a container holder that is integrally formed with housing sections that comprise other components of the mechanism, such as a piston rod guide.

The dose delivery mechanism may comprise a bearing that is configured to directly contact the plunger of the attached medicament container. The bearing may be located at the piston rod. Thereby, it may be integrally formed with the piston rod. Alternatively, it may be configured as a separate component, like a disc, that is located in between the piston rod and the plunger. For example, the bearing may be attached to the piston rod.

The mechanism may be configured as a disposable mechanism that is disposed of after ejecting a last dose from the medicament container attached to the mechanism. Alternatively, the mechanism may also be configured as a reusable mechanism that allows attachment of a new medicament container after having ejected a last dose from a previous container.

The dose setting element and the button may form elements of an actuation unit of the mechanism. The dose setting element and the button may be placed next to each other. For example, the dose setting element and the button may be located at a distal end of the mechanism. The button may terminate the mechanism at its distal end.

The button may be axially movable with respect to the housing. The button thereby may be biased in a distal direction with respect to the housing. For example, the mechanism may comprise a biasing element, such as a spring, for example a compression spring, that biases the button in the distal direction with respect to the housing.

With some embodiments, the dose setting element and the button are fixed with respect to each other during dose setting and dose delivery. This provides a simple and compact construction of the button and the dose setting element. For example, the button and the dose setting element may be integrally formed. Alternatively, the button and the dose setting element may be configured as separate components that are fixed to each other, either directly or via one or more intermediate components.

With other embodiments, the dose setting element and the button are movable with respect to each other. This allows to clearly separate the rotation of the dose setting element for setting the dose during dose setting from the movement of the button to transfer the mechanism to the dose delivery state. For example, the button may be configured to axially move with respect to the dose setting element upon transfer from the dose setting state to the dose delivery state.

With some embodiments, the button is axially movable with respect to the dose setting element and rotationally fixed with respect to the dose setting element. The button may be configured to follow the rotation of the dose setting element during dose setting and at the same time it may be configured to axially move with respect to the dose setting element to transfer the mechanism into the dose delivery state. Following the rotation of the dose setting element during dose setting facilitates setting of the dose, for example with embodiments in which the dose setting element and the button are placed next to each other.

With some embodiments, the dose setting element is rotationally fixed with respect to the housing when the dose is being delivered. This reduces the number of movable external parts of the mechanism during dose delivery and thus provides a user-friendly mechanism. The mechanism may, for example, comprise a clutch that rotationally fixes the dose setting element to the housing during dose delivery and rotationally releases the dose setting element from the housing during dose setting. The clutch may, for example, be configured to close upon movement of the button, such as upon axial movement of the button.

With some embodiments, the dose setting element is axially fixed with respect to the housing during dose setting and during dose delivery. This provides a user-friendly design since such a dose setting element has a reduced number of motional degrees of freedom.

With some embodiments, the button is configured to remain axially stationary with respect to the housing when the piston rod moves in the proximal direction to deliver the dose. This reduces the risk of a user of the device interfering with components that move during automatic medicament delivery and provides a user-friendly and safe mechanism.

Delivery of the medicament may not require axial movement of the button after having transferred the mechanism to the dose delivery state. Furthermore, delivery of the dose may already start while the button is still moved into its fully triggered position and/or delivery of the dose may still last while the button is starting to move back from its fully triggered position, for example to interrupt automatic injection.

The button being configured to remain axially stationary with respect to the housing when the piston rod moves in the proximal direction to deliver the dose thus encompasses that the button is configured to remain axially stationary with respect to the housing during at least a part of the movement of the piston rod in the proximal direction during dose delivery.

According to an embodiment, the mechanism further comprises a dosing member, wherein the dosing member is configured to rotate with respect to the housing during dose setting and to rotate with respect to the housing during dose delivery and wherein the dosing member is configured to remain axially stationary with respect to the housing during dose delivery. Compared to a dosing member that is configured to axially move during dose delivery, a dosing member that is axially fixed during dose delivery provides for a compact construction of the mechanism.

The button and/or the dose setting element may be coupled to the housing via the dosing member. For example, the button and/or the dose setting element may be axially coupled to the housing via the dosing member. The dosing member then may limit axial movement of the button and/or the dose setting element with respect to the housing.

The dosing member may comprise a dose sleeve that at least partially encloses the piston rod and/or the nut.

With some embodiments, the dosing member comprises a first part and a second part, wherein the first part is axially movable and rotationally fixed with respect to the second part. This allows to axially fix the first part and the second part to different components of the mechanism that are axially movable with respect to each other.

The first part and the second part are configured to both remain axially stationary with respect to the housing during dose delivery so that the dosing member as a whole remains axially stationary during dose delivery. One of the first part and the second part may be configured to move axially with respect to the housing upon transfer of the mechanism between the dose setting state and the dose delivery state. The first part and the second part may be configured to move axially with respect to each other upon transfer of the mechanism between the dose setting state and the dose delivery state.

With some embodiments, the first part is axially fixed with respect to the button and/or the second part is axially fixed with respect to the housing. The first part may be a coupling member that couples the dosing member to the dose setting element during dose setting. Additionally or alternatively, the first part may be a carrier that comprises elements of a dose definition mechanism, such as one of a dose stop or a counter element for engagement with a dose stop. The second part may be the dose sleeve.

With some embodiments, a rotational position of the dosing member defines a dose to be delivered in the dose delivery state. For example, each rotational position of the dosing member may uniquely define a dose to be delivered. The dosing members then may be configured to perform less than a full rotation during dose setting. The rotational position may be defined with respect to the housing of the mechanism.

With some embodiments, a rotational position of the dosing member defines a dose to be delivered in the dose delivery state and the first part comprises one of a dose stop and a counter element of a dose definition mechanism. The dose definition mechanism than may act between the dosing member and the housing of the mechanism.

According to an embodiment, the dosing member is configured to remain axially stationary with respect to the housing while storing the energy in the spring during dose setting. Such a dosing member provides for a compact construction of the mechanism.

According to an embodiment, the dosing member is coupled between the spring and the dose setting element during dose setting to transfer energy from the dose setting element to the spring. For example, the dosing member may be rotationally fixed with respect to the dose setting element during dose setting. A torque that is supplied by a user to the dose setting element during dose setting may be transferred to the spring via the dosing member.

According to an embodiment, the dosing member comprises a label to visually indicate setting of the dose. The label then rotates with respect to the housing during dose setting and dose delivery. This allows to reset the indication of a set dose during dose delivery.

The label may be configured to be visible from an outside of the housing when the dose has been set. For example, the label may be visible within a window provided in the housing. The label may be provided at a component of the dosing member that is axially fixed with respect to the housing, such as at the dose sleeve.

With some embodiments, the dose setting element is configured to remain axially stationary with respect to the dosing member during dose delivery. Additionally or alternatively, the dose setting element may be configured to remain axially stationary with respect to the dosing member during dose setting.

According to an embodiment, the mechanism further comprises a blocker, wherein the blocker acts between the button and the housing and wherein the blocker blocks the button from moving axially with respect to the housing in the distal direction. Such a blocker may prevent detachment of the button from the housing. Additionally or alternatively, the blocker may be configured to prevent axial movement of the dose setting element in the distal direction with respect to the housing.

According to an embodiment, the blocker acts between the dosing member and the housing. This fixes the button and/or the dose setting element via the dosing member to the housing. The blocker may directly act between the housing and the dosing member so that the dosing member engages with the housing via the blocker. Alternatively, the blocker may also act via one or more intermediate members between the housing and the dosing member.

According to an embodiment, the blocker is provided at one of the housing and the dosing member and engages with the other one of the housing and the dosing member. This provides for a blocker that directly acts between the housing and the dosing member. The blocker may be fixed to the one of the housing and the dosing member.

With some embodiments the blocker is configured as an axial stop. This precisely defines a limit for the axial position of the button and/or the dose setting element in the distal direction.

In general, an axial stop may comprise two blocking surfaces that move towards each other along the longitudinal axis of the mechanism and engage with each other to stop further axial movement. The blocking surfaces may be orientated parallel to each other.

Each blocking surface may be orientated essentially perpendicular, such as perpendicular, to the longitudinal axis of the mechanism and essentially parallel, such as parallel, to a radial plane that is orientated perpendicular to the longitudinal axis. Alternatively, each blocking surface may be tilted with respect to the radial plane. Such tilting may provide an undercut. The undercut may be configured to press the blocking surfaces against each other upon mutual engagement.

According to an embodiment, the blocker is configured as a one-way blocker and the blocker allows relative axial movement between the housing and a counter member in a first direction and blocks relative axial movement between the housing and the counter member in a second direction opposite to the first direction. This facilitates assembly of the mechanism since the housing and the counter member may be moved with respect to each other in the first direction during assembly and the housing and the counter member may be restrained with respect to each other in the second direction after assembly.

For example, the blocker may allow axial movement of the counter member with respect to the housing in the proximal direction and block axial movement of the counter member with respect to the housing in the distal direction. This allows to insert the counter member into the housing from the distal end of the housing during assembly.

According to an embodiment, the blocker is configured as a flexible element that is configured to snap into a blocking position upon assembly of the counter member to the housing. This provides for a simple assembly of the mechanism.

For example, the blocker may be configured to deflect during assembly of the counter member to the housing and to snap into the blocking position when the counter member has been positioned with respect to the housing. With such a configuration, the blocker may be fixed to one of the housing and the counter member during assembly.

With other embodiments, the blocker may be configured as a separate element that is configured to be attached and/or fixed to one of the housing and the counter member after assembly of the counter member to the housing. The blocker may, for example, be configured to become attached and/or fixed to the one of the housing and the counter member by a form-fit, such as a threaded connection or a snap fit. Exemplarily, the blocker may be configured to be fixed and/or attached to the housing during assembly.

According to an embodiment, the counter member is part of the dosing member. This allows to fix and/or attach the blocker to the housing during assembly.

With other embodiments, the counter member is part of the housing. This allows to fix and/or attach the blocker to the counter member.

According to an embodiment, the mechanism comprises a stop to limit proximal movement of the button during dose delivery. This precisely defines the position of the button after transfer of the mechanism to the dose delivery state. The button may be configured to interact with the stop, either directly or via one or more intermediate members. The stop may limit proximal movement of the button with respect to the housing.

The stop may comprise a first stop part and a second stop part. The first stop part and the second stop part may be configured to move towards each other during proximal movement of the button and to engage with each other to limit further proximal movement of the button during dose delivery. Furthermore, the first stop part and the second stop part may be configured to move away from each other upon distal movement of the button.

According to an embodiment, the dosing member is coupled in between the stop and the housing. The stop then limits proximal movement of the button with respect to the dosing member. In addition, the dosing member may limit proximal movement of the button with respect to the housing. For example, the dosing member may be axially fixed with respect to the housing.

With some embodiments, a proximal force exerted on the button during dose delivery may be transferred to the housing only via components that are static during dose delivery.

According to an embodiment, the stop is provided at the housing, such as an inside surface of the housing. This allows to absorb a proximal force exerted on the button directly at the housing and to thus direct this force away from components that move during dose delivery. This may reduce friction within the mechanism.

According to an embodiment, the stop acts between the housing and a housing connector that is axially fixed to the button. For example, the first stop part may be fixed to the housing and the second stop part may be fixed to the housing connector. The housing and the housing connector may engage with each other via the stop.

The button may be rotationally movable with respect to the housing connector. The housing connector may be configured, for example, as a dose selector of the mechanism.

With other embodiments, the stop may be act between the dose setting element and the button. This provides a simple construction of the stop. With these embodiments, the button may be axially movable with respect to the dose setting element. The dose setting element may be axially restrained, such as axially fixed, with respect to the housing. The first stop part then may be fixed to the dose setting element and the second stop part may be fixed to the button.

According to an embodiment, the stop is configured as an axial stop. This precisely defines a limit for the axial position of the button in the distal direction. The axial stop may be configured as it is disclosed in connection with the axial stop of the blocker above.

With some embodiments, the mechanism further comprises a retainer to prevent detachment of the button and/or the dose setting element from the housing, wherein the retainer comprises a first retainer element and a second retainer element, wherein the first retainer element and the second retainer element are configured to engage with each other to prevent the detachment of the button and/or the dose setting element from the housing.

With some embodiments, the retainer acts between, on the one hand, the dosing member and, on the other hand, the button and/or the dose setting element. For example, the retainer may act via the dosing member between, on the one hand, the housing and, on the other hand, the button and/or the dose setting element.

With some embodiments, the dose setting element is axially fixed with respect to the housing, wherein the retainer is configured to prevent detachment of the button from the housing and wherein the retainer acts between the dose setting element and the button.

With some embodiments, the first retainer element and the second retainer element are configured to move away from each other to allow axial movement of the button and/or the dose setting element with respect to the housing in the proximal direction.

With some embodiments, the retainer is configured as an axial stop. This allows to securely fix the button and/or the dose setting element to the housing. The axial stop may be configured as it is disclosed above in connection with the axial stop of the blocker.

With some embodiments, the mechanism further comprises a driver, wherein the driver is coupled between the spring and the nut to transfer the energy stored in the spring to the nut when the mechanism is in the dose delivery state. Furthermore, the driver is configured to move in the distal direction when the mechanism is in the dose setting state and to move in the proximal direction when the mechanism is in the dose delivery state.

The driver may be configured to engage with the knob to transfer the energy stored in the spring in the dose delivery state. For example, the driver may be configured to axially push onto the nut.

With some embodiments, the spring is coupled between the driver and the housing. The spring then may be strained by moving the driver, for example by rotationally moving the driver during dose setting. Furthermore, the spring may release by moving the driver, for example by rotationally moving the driver with respect to the housing. The driver then may transfer the energy stored in the spring during dose setting to the nut and the piston rod. The spring may be directly coupled to the driver and/or directly coupled to the housing. This provides for a compact construction of the mechanism.

With some embodiments, the driver is rotationally driven by the spring during dose delivery. The driver then may be configured to convert rotational movement into axial movement, for example via a threaded connection.

With some embodiments, the driver is coupled between the spring and the dose setting element during dose setting to transfer the energy from the dose setting element to the spring. The driver then is configured to couple the spring to the mechanism both during dose setting and dose delivery. For example, the driver may be permanently coupled to one end of the spring both during dose setting and dose delivery.

With some embodiments, the driver is rotationally fixed with respect to the nut when the mechanism is in the dose setting state and rotationally movable with respect to the nut when the mechanism is in the dose delivery state. The driver rotating together with the nut allows to simultaneously move the nut and the driver in the axial direction, for example via respective threaded connections to the housing. Rotationally decoupling the nut from the driver during dose delivery then may allow to axially lock the nut to the piston rod.

With some embodiments, the driver is rotationally fixed with respect to the dose setting element when the mechanism is in the dose setting state and rotationally movable with respect to the dose setting element when the mechanism is in the dose delivery state. This decouples the dose setting element from the driver during dose delivery so that the dose setting element may be rotationally stationary during dose delivery.

With some embodiments, the driver is rotationally movable with respect to the housing during dose setting and dose delivery. The driver then may rotationally couple the spring to other movable parts of the mechanism both during dose setting and dose delivery.

With some embodiments, the mechanism comprises a drive thread that couples the driver to the housing, wherein the drive thread converts a torque provided by the spring into axial movement of the driver. For example, the driver may threadedly engage the housing via the drive thread.

With some embodiments, the driver is coupled between the nut and the dosing member, wherein, for example, the driver engages with the dosing member. Engagement with the dosing member may, for example, rotationally fix the driver to the dosing member.

With some embodiments, the driver is rotationally fixed and/or axially movable with respect to the dosing member. For example, the driver may be connected to the dosing member by a rotation lock, such as a splined connection. The driver may directly engage the dosing member. It may, for example, directly engage with the second part of the dosing member that is axially fixed with respect to the housing, such as with the dose sleeve.

With some embodiments, the mechanism further comprises a dose definition mechanism, wherein the dose definition mechanism acts between the dose setting element and the housing during dose setting. The dose definition mechanism thereby has at least one dose stop and a counter element, wherein the counter element is configured to rotate with respect to the dose stop when the dose setting element rotates during dose setting and wherein the counter element is configured to engage the dose stop when the dose has been set. The dose definition mechanism may define the rotational positions of the dose setting element with respect to the housing that correspond to settable doses. For each settable dose, the dose definition mechanism may comprise a separate dose stop.

The counter element may be configured as a flexible element that snaps over the dose stop upon setting the dose. For example, the counter element may be configured as a flexible protrusion at a component of the mechanism. The counter element may, for example, be integrally formed with the component of the mechanism it is fixed to.

With some embodiments, engagement of the counter element with the dose stop prevents the spring from releasing the energy stored upon rotation of the dose setting element. The dose definition mechanism thereby may provide a latching function that keeps the spring in a tensioned state until the dose is delivered by transferring the mechanism from the dose setting state to the dose delivery state.

With some embodiments, the counter element is configured to disengage from the dose stop upon transfer of the mechanism from the dose setting state into the dose delivery state. This prevents the dose definition mechanism from interfering with the delivery of the set dose. Furthermore, it may allow the spring to release the energy stored upon rotation during dose setting. With embodiments having more than a single dose stop, the counter element may be configured to disengage from all dose stops upon transfer of the mechanism into the dose delivery state. This may allow the counter element to rotate back to its initial position during dose delivery without interfering with the dose stops. The initial position may correspond to a zero dose position in which no dose has been set.

For example, the counter element may be configured to disengage from the dose stop by axially moving with respect to the dose stop.

With some embodiments, one of the dose stop and the counter element, such as the dose stop, is rotationally fixed with respect to the housing. The one of the dose stop and the counter element then may be axially movable with respect to the dose setting element.

With some embodiments, the one of the dose stop and the counter element, such as the dose stop, is axially fixed with respect to the button. This allows to move the one of the dose stop and the counter element together with the button upon transfer of the mechanism from the dose setting state into the dose delivery state. The one of the dose stop and the counter element than may disengage from the other one of the dose stop and the counter element by this movement. For example, the one of the dose stop and the counter element may be linearly guided at the housing.

With other embodiments, the one of the dose stop and the counter element is axially fixed with respect to the housing. The other one of the dose stop and the counter element than may be axially movable with respect to the housing, for example by axially moving the button.

With some embodiments, the one of the dose stop and the counter element is fixed to an outer housing part of the mechanism. The outer housing part may be fixed to a connection for coupling a medicament container to the mechanism. Alternatively, the outer housing part may also be movable with respect to the connection, such as axially movable. For example, the outer housing part may be a housing connector, such as the housing connector that engages with the housing via the stop upon relative axial movement between the housing in the housing connector.

The dose definition mechanism may also act between the dosing member and the housing. It then may define rotational positions of the dosing member with respect to the housing that correspond to settable doses.

With some embodiments, the other one of the dose stop and the counter element, such as the counter element, is rotationally fixed with respect to the dosing member. For example, the other one of the dose stop and the counter element may be permanently rotationally fixed with respect to the dosing member. The other one of the dose stop and the counter element may additionally be axially fixed to the dosing member, such as to the first part of the dosing member that is movable with respect to the housing.

The other one of the dose stop and the counter element may be axially fixed to the housing. For example, the other one of the dose stop and the counter element may be provided at an embodiment of the dosing member that comprises a single part that is axially fixed to the housing.

With some embodiments, the other one of the dose stop and the counter element, such as the counter element, is axially movable with respect to the button. The one of the dose stop and the counter element, such as the dose stop, then may be axially fixed with respect to the button. This allows to disengage the dose stop from the counter element by moving the button with respect to the housing.

With some embodiments, the other one of the dose stop and the counter element, such as the counter element, is axially movable with respect to the housing. With embodiments of the dosing member that have the first part that is axially movable with respect to the second part, the other one of the dose stop and the counter element may be fixed to the first part of the dosing member. For example, the other one of the dose stop and the counter element may be formed at the dosing member, such as at the first part of the dosing member.

With some embodiments, the other one of the dose stop and the counter element is axially fixed with respect to the button. The one of the dose stop and the counter element, such as the dose stop, then may be axially movable with respect to the button. This allows to disengage the dose stop from the counter element by moving the button with respect to the housing.

With some embodiments, the mechanism comprises a blocking mechanism having a first element and a second element, wherein the first element engages the second element upon release of the button during dose delivery to prevent a transfer of the mechanism from the dose delivery state to the dose setting state. This keeps up dose delivery even if a user releases the button during dose delivery. The blocking mechanism may block distal movement of the button against a biasing force biasing the button in the distal direction.

The blocking mechanism may be configured to disengage the first element from the second element at a zero dose position at which a set dose has been fully delivered. This allows the mechanism to return to the dose setting state so that a subsequent dose can be set after having completed a previous medicament delivery.

With some embodiments, the first element rotates with respect to the second element in a first direction during dose setting and rotates in a second direction opposite the first direction during dose delivery. Relative movement between the first and second element may bring the first and second element in relative positions that prevent mutual engagement at the end of dose delivery and/or when a dose has been set.

With some embodiments, the first element is configured as a circumferential rib that longitudinally extends around an axis of the housing and the second element is configured as a stop or counter element that travels along the circumferential rib during dose delivery.

With some embodiments, the second element passes the first element upon release of the button at the end of dose delivery. For example, the second element may pass through an opening within the first element. The second element may rotate into alignment with the opening at the end of dose delivery.

With some embodiments, the second element passes the first element upon transfer of the mechanism from the dose setting state into the dose delivery state. For example, the second element may pass through an opening within the first element. The second element may rotate into alignment with the opening when a dose has been set.

With some embodiments, the second element passes through an opening within the first element upon transfer of the mechanism from the dose setting state into the dose delivery state. Such an opening prevents blocking and thus allows axial movement of the button to initiate dose delivery.

With some embodiments, the first element comprises a recessed section at the opening, wherein the second element engages with the recessed section upon release of the button after having passed through the opening during transferring the mechanism from the dose setting state into the dose delivery state and wherein engagement of the second element with the recessed section prevents transition of the mechanism back into the dose setting state. The recessed section may form a one-way passage for the second element that allows movement of the second element with respect to the first element in a first direction and blocks movement in a second direction opposite the first direction.

The first element may comprises several openings. At each opening, a recessed section may be formed. This prevents passage of the second element through the openings in the dose delivery state. For example, the second element may pass along at least one of the openings, such as along a multitude of the openings, during dose delivery. The recessed sections then prevent a transition of the mechanism into the dose setting state at the respective openings.

The recessed sections may have chamfered edges that deflect the second element during passage in the first direction. The recessed sections then may still interfere with the second element upon movement in the second direction opposite the first direction. The second element may engage the chamfered edges at a shallow angle when moving in the first direction. Furthermore, the second element may engage the recessed section at a steep angle that is larger than the shallow angle when moving in the second direction.

With some embodiments, the first element of the blocking mechanism and one of the dose stop and the counter element, such as the dose stop, are fixed to the same member of the mechanism. Furthermore, the second element of the blocking mechanism and the other one of the dose stop and the counter element, such as the counter element, are fixed to the same further member of the mechanism. This allows to precisely align the elements of the blocking mechanism and the elements of the dose definition mechanism and enhances reliability of the mechanism.

The member of the mechanism that comprises the first element of the blocking mechanism and the one of the dose stop and the counter element may, for example, be a dose selector of the mechanism. The further member of the mechanism that comprises the second element of the blocking mechanism and the other one of the dose stop and the counter element may, for example, be a carrier that is rotationally movable with respect to the dose selector. The carrier may, for example, be a part of the dosing member.

The dose selector may at least partly the located within an outer housing of the mechanism. The dose selector may be configured to protrude from the outer housing. With other embodiments, the dose selector may be entirely located within the outer housing.

With some embodiments, one of the first element and second element of the blocking mechanism and one of the dose stop and the counter element are formed by a single element. For example, the second element of the blocking mechanism and the counter element of the dose definition mechanism may be formed by the single element. This facilitates alignment of the components of the blocking mechanism with respect to the components of the dose definition mechanism.

The single element may be a flexible element that is configured to snap over the dose stop upon rotation with respect to the dose stop. Furthermore, the single element may be configured to snap over the recessed section provided in the first element of the blocking mechanism.

With some embodiments, the mechanism comprises a maximum dose mechanism that restrains further rotation of the dose setting element upon dialing past a maximum dose setting, wherein the maximum dose mechanism comprises a maximum dose stop and a blocking part and wherein the blocking part is configured to engage the maximum dose stop upon dialing past the maximum dose setting. This provides a well-defined rotational end position of the dose setting element. The maximum dose stop may also absorb a torque provided by a user and direct the torque to the housing of the mechanism.

The blocking part may engage the maximum dose stop right at the maximum dose setting. With other embodiments, the blocking part may only engage the maximum dose stop after having dialed past the maximum dose setting by a predefined amount.

The blocking part may be configured as a hard stop that is rigidly connected, such as integrally formed, with a component of the mechanism. Likewise, the maximum dose stop may be configured as such a hard stop.

With some embodiments, the maximum dose stop and the blocking part are configured to rotate with respect to each other during dose setting. For example, one of the maximum dose stop and the blocking part, such as the maximum dose stop, may be rotationally fixed with respect to the housing during dose setting and the other one of the maximum dose stop and the blocking part, such as the blocking part, may be rotationally fixed with respect to the dose setting element during dose setting. The other one of the maximum dose stop and the blocking part may be rotationally movable with respect to the dose setting element during dose delivery.

With some embodiments, the maximum dose stop is configured as a radial stop and the blocking part is configured to rotate against the maximum dose stop upon dialing past the maximum dose. Such a radial stop provides a well-defined rotational position in which the blocking part and the maximum dose stop get into engagement.

The maximum dose stop and the blocking part may comprise engagement surfaces that engage with each other. The engagement surfaces may be orientated essentially perpendicular, such as perpendicular, to a circumferential direction around the longitudinal axis of the mechanism.

With some embodiments, one of the maximum dose stop and the blocking part, such as the maximum dose stop, is rotationally fixed with respect to the housing. The one of the maximum dose stop and the blocking part may be permanently rotationally fixed with respect to the housing, both during dose setting and dose delivery.

With some embodiments, the one of the maximum dose stop and the blocking part is fixed to an outer housing part of the mechanism. The outer housing part may, for example, be configured as a housing connector that is located between the dose setting element and the housing of the device. Additionally or alternatively, the outer housing part may be configured as the dose selector.

With some embodiments, the other one of the maximum dose stop and the blocking part, such as the blocking part, is rotationally fixed with respect to the dosing member. The other one of the maximum dose stop and the blocking part then rotates with respect to the housing during both dose setting and dose delivery. This allows to reset the maximum dose mechanism during dose delivery. With embodiments, in which a rotational position of the dosing member defines the dose that has been set, rotationally fixing the one of the maximum dose stop and the blocking part to the dosing member precisely defines a maximum dose position in which the maximum dose stop and the blocking part engage with each other.

With some embodiments, the other one of the maximum dose stop and the blocking part is fixed to a coupling member that rotationally couples the dosing member to the dose setting element during dose setting.

With some embodiments, one of the dose stop and the counter element, such as the dose stop, and one of the maximum dose stop and the blocking part, such as the maximum dose stop, are fixed to the same member of the mechanism. This allows to precisely define the relative positions of the components of the dose definition mechanism with respect to the components of the maximum dose mechanism. The member of the mechanism may, for example, be the dose selector.

With some embodiments, the other one of the dose stop and the counter element, such as the counter element, and the other one of the maximum dose stop and the blocking part, such as the blocking part, are fixed to the same further member of the mechanism. This also allows to precisely define the relative positions of the components of the dose definition mechanism with respect to the components of the maximum dose mechanism. The further member may, for example, be the carrier that is rotationally movable with respect to the dose selector.

With some embodiments, the mechanism comprises a zero dose mechanism that prevents further axial movement of the nut at the end of dose delivery, wherein the zero dose mechanism comprises a zero dose stop and a further blocking part and wherein the further blocking part is configured to engage the zero dose stop at the end of dose delivery. This provides a well-defined end position for the piston rod at the end of dose delivery and thus contributes to precisely define the amount of medicament delivered.

The further blocking part may be configured as a hard stop that is rigidly connected, such as integrally formed, with a component of the mechanism. Likewise, the zero dose stop may be configured as such a hard stop.

With some embodiments, the zero dose stop and the further blocking part are configured to rotate with respect to each other during dose delivery. For example, one of the zero dose stop and the further blocking part, such as the zero dose stop, may be rotationally fixed with respect to the housing during dose setting and the other one of the zero dose stop and the further blocking part, such as the further blocking part, may be rotationally fixed with respect to the dose setting element during dose setting. The other one of the zero dose stop and the further blocking part may be rotationally movable with respect to the dose setting element during dose delivery.

With some embodiments, the zero dose stop is configured as a radial stop and the further blocking part is configured to rotate against the zero dose stop at the end of dose delivery. Such a radial stop provides a well-defined rotational position in which the further blocking part and the zero dose stop become engaged with each other.

The zero dose stop and the further blocking part may comprise engagement surfaces that engage with each other. The engagement surfaces may be orientated essentially perpendicular, such as perpendicular, to a circumferential direction around the longitudinal axis of the mechanism.

With some embodiments, one of the zero dose stop and the further blocking part, such as the zero dose stop, is rotationally fixed with respect to the housing. The one of the zero dose stop and the further blocking part may be permanently rotationally fixed with respect to the housing, both during dose setting and dose delivery.

With some embodiments, the one of the zero dose stop and the further blocking part is fixed to an outer housing part of the mechanism. The outer housing part may, for example, be configured as a housing connector that is located between the dose setting element and the housing of the device. Additionally or alternatively, the outer housing part may be configured as the dose selector.

With some embodiments, the other one of the zero dose stop and the further blocking part is rotationally fixed with respect to the dosing member. The other one of the zero dose stop and the further blocking part then rotates with respect to the housing during both dose setting and dose delivery. This allows to reset the zero dose mechanism during dose setting. With embodiments, in which a rotational position of the dosing member defines the dose that has been set, rotationally fixing the one of the zero dose stop and the further blocking part to the dosing member precisely defines a zero dose position in which the zero dose stop and the further blocking part engage with each other.

With some embodiments, the other one of the zero dose stop and the further blocking part is fixed to a coupling member that rotationally couples the dosing member to the dose setting member during dose setting.

With some embodiments, one of the dose stop and the counter element, such as the dose stop, and one of the zero dose stop and the further blocking part, such as the zero dose stop, are fixed to the same member of the mechanism. This allows to precisely define the relative positions of the components of the dose definition mechanism with respect to the components of the zero dose mechanism. The member of the mechanism may, for example, be the dose selector.

With some embodiments, the other one of the dose stop and the counter element, such as the counter element, and the other one of the zero dose stop and the further blocking part, such as the further blocking part, are fixed to the same further member of the mechanism. This also allows to precisely define the relative positions of the components of the dose definition mechanism with respect to the components of the zero dose mechanism. The further member may, for example, be the carrier that is rotationally movable with respect to the dose selector.

With some embodiments, one of the maximum dose stop and the blocking part of the maximum dose mechanism and one of the zero dose stop and the further blocking part of the zero dose mechanism, such as the maximum dose stop and the zero dose stop, are fixed to the same member of the mechanism. Furthermore, the other one of the maximum dose stop and the blocking part of the maximum dose mechanism and the other one of the zero dose stop and the further blocking part of the zero dose mechanism, such as the blocking part and the further blocking part, are fixed to the same further member of the mechanism. This provides for a precise alignment between the components of the maximum dose mechanism and the components of the zero dose mechanism.

Irrespective of whether they are fixed to the same member of the mechanism or not, the maximum dose stop and/or the minimum dose stop may be integrally formed with the member they are fixed to. Analogously, the blocking part and/or the further blocking part may be integrally formed with the member they are fixed to.

With some embodiments, the blocking part of the maximum dose mechanism forms the further blocking part of the minimum dose mechanism. This allows to precisely define the distance between the maximum dose position and the zero dose position.

With some embodiments, the mechanism comprises a break for stopping movement of the nut upon release of the button during dose delivery. This provides a possibility for the user to interrupt dose delivery by releasing the button. The break may be configured to disengage when the user again actuates the button.

The break may comprise a first break part and a second break part that engages the first break part upon release of the button during dose delivery. The first break part may axially engage the second break part by moving along a longitudinal axis of the mechanism with respect to the second break part.

The first break part may be biased towards the second break part, for example by a spring.

With some embodiments, the first break part rotates with respect to the second break part in a first direction during dose setting and rotates in a second direction opposite the first direction during dose delivery. This allows to reset the brake parts during dose setting. Furthermore, the first break part may be prevented from engaging with the second break part at predefined rotational positions, such as at an end of dose position.

With some embodiments, the first break part is configured as a circumferential rib that longitudinally extends around an axis of the housing and the second break part is configured as a stop or counter element that travels along the circumferential rib during dose delivery. This provides a simple configuration in which the first break part may be prevented from engaging with the second break part at specific positions along the circumferential rib.

With some embodiments, the second break part frictionally locks to the first break part upon release of the button during dose delivery. The first break part then may travel along the second break part and it may be pushed against the second break part upon release of the button during dose delivery. The mechanism may be configured to counter the force provided by the spring during dose delivery by the friction between the first break part and the second break part.

With some embodiments, the first break part comprises a plurality of grooves, wherein the second break part is configured to engage with at least one of the grooves upon release of the button during dose delivery. This provides a strong and reliable break mechanism.

With some embodiments, the first element of the blocking mechanism forms the first break part of the break and/or the second element of the blocking mechanism forms the second break part of the break. This provides a compact configuration of the blocking mechanism and the break.

In general, the mechanism may comprise a clutch having a first engaging part and a second engaging part, wherein the clutch is closed during one of dose setting and dose delivery and opened during the other one of dose setting and dose delivery. The clutch is in the opened state when the first engaging part and the second engaging part do not engage with each other and the clutch is in the closed state when the first engaging part and the second engaging part engage with each other.

With some embodiments, the mechanism comprises a clutch, wherein the clutch rotationally locks the nut to the piston rod during dose delivery and rotationally releases the nut from the piston rod during dose setting. By rotationally locking the nut to the piston rod, the clutch may force simultaneous proximal movement of the nut together with the piston rod. For example, the clutch may rotationally lock a thread that couples the nut to the piston rod. The clutch may lock the nut to the piston rod in a closed state and rotationally release the nut from the piston rod in an opened state.

The clutch may comprise a first engaging part and a second engaging part and the first and second engaging parts may be configured to engage with each other to rotationally lock the nut to the piston rod. The first engaging part and the second engaging part may be configured to become disengaged from each other by relative axial movement with respect to each other.

The clutch may be configured to be transferred from the opened state into the closed state upon movement of the button and transfer of the mechanism from the dose setting state to the dose delivery state. One of the first engaging part and the second engaging part, such as the second engaging part, may be axially fixed with respect to the button and the other one of the first engaging part and the second engaging part, such as the first engaging part, may be axially fixed with respect to the housing. Additionally or alternatively, the first engaging part may be axially fixed with respect to the dosing member.

With some embodiments, the first engaging part is rotationally fixed to the housing and the second engaging part is rotationally fixed to the nut.

The second engaging part may be rotationally fixed to the button and/or the dose setting element.

With some embodiments, the clutch rotationally fixes the nut to the piston rod during dose delivery via the housing and, for example, via the dose setting element and/or the button.

With some embodiments, the clutch acts between the button and the housing and/or between the dose setting element and the housing.

With some embodiments the button is rotationally coupled, such as permanently rotationally coupled, to one of the first engaging part and the second engaging part. For example, the button may constitute the one of the first engaging part and the second engaging part.

With some embodiments, the mechanism comprises a further clutch, wherein the further clutch rotationally locks the dose setting element to one end of the spring during dose setting and decouples the dose setting element from the one end of the spring during dose delivery. Furthermore, the further clutch has a further first engaging part and a further second engaging part, wherein the further first engaging part is configured to move into engagement with the further second engaging part to rotationally lock the dose setting element to the one end of the spring. The further clutch allows to tension the spring during dose setting and at the same time prevents the dose setting element from rotating during dose delivery when the spring relaxes again.

The further clutch may be transferred from a closed state, in which the further first engaging part engages with the further second engaging part, into an opened state, in which the further first engaging part is disengaged from the further second engaging part, by movement of the button. The movement of the button may be the movement that transfers the mechanism from the dose setting state into the dose delivery state.

With some embodiments, one of the further first engaging part and the further second engaging part is rotationally and axially fixed to the dose setting element. This allows to open and close the further clutch by relative movement of the dose setting element with respect to the other one of the further first engaging part and the further second engaging part.

With some embodiments, the one of the further first engaging part and the further second engaging part is rotationally and axially fixed to the button. The dose setting element then may be at least rotationally fixed to the button. For example, the dose setting element and the button may be rotationally fixed and axially movable with respect to each other. This allows to axially fix the dose setting element with respect to the other one of the further first engaging part and the further second engaging part. For example, the dose setting element and the other one of the further first engaging part and the further second engaging part may be axially fixed with respect to the housing.

The dose setting element may be coupled to the further clutch via the button. The dose setting element and the button may be rotationally fixed with respect to each other. Furthermore, they may be rotationally fixed to one of the further first and second engaging parts, such as rotationally fixed to the further second engaging part.

With some embodiments, the further clutch acts between the dosing member and the dose setting element. The dosing member then may rotationally couple the dose setting element to the one end of the spring.

With some embodiments, one of the further first engaging part and the further second engaging part is rotationally fixed to the dosing member. Additionally or alternatively, the one of the further first engaging part and the further second engaging part may be axially fixed to the dosing member.

With some embodiments, the further clutch is located within the dosing member. For example, the further clutch may be located within the dose sleeve or dosing element of the dosing member.

With some embodiments, the clutch comprises the first engaging part that engages the second engaging part to rotationally fix the nut to the piston rod during dose delivery, wherein the second engaging part of the clutch forms the further first engaging part of the further clutch. This provides a compact construction of the clutches.

The nut may be rotationally fixed with respect to the button and/or the dose setting element. For example, the nut may be rotationally fixed and axially movable with respect to the button and/or the dose setting element. It may be coupled to the button and/or the dose setting element by a rotation lock. The rotation lock may be formed by the nut and one of the button and the dose setting element, such as the button.

With some embodiments, the nut is threadedly connected to the piston rod, such as threadedly engaged with the piston rod.

The nut may be rotationally movable with respect to the piston rod when the mechanism is in the dose setting state and the nut may be rotationally fixed with respect to the piston rod when the mechanism is in the dose delivery state. Rotation of the nut with respect to the piston rod during dose setting then may lead to axial movement due to the threaded connection between the nut and the piston rod. By rotationally locking the nut to the piston rod during dose delivery, the threaded connection between the nut and the piston rod is blocked and the nut and the piston rod may become axially fixed with respect to each other.

The nut may be turned by the dose setting element during dose setting and may perform an axial movement due to the threaded connection to the piston rod. The rotation of the nut may cause the nut to translate axially in a distal direction along the thread located on the piston rod during dose setting and to translate in the proximal direction during dose cancellation. Axial movement of the nut with respect to the piston rod then may define the axial movement of the piston rod during dose delivery and thus the amount of medicament expelled during dose delivery.

With some embodiments, the spring is configured as a torsion spring.

The present disclosure is also directed at a medicament delivery device having a mechanism according to the present disclosure and a medicament container attached to the mechanism. The medicament container comprises a plunger and a bearing located at the piston rod is configured to engage with the plunger for dose delivery.

All embodiments and technical effects that are disclosed in connection with the mechanism according to the present disclosure also apply to the medicament delivery device and vice versa.

The medicament delivery device may be configured as an injection device, such as a pen injection device. The medicament container may be configured to receive a cannula at its proximal end to deliver the medicament through the cannula.

### DRAWINGS

Exemplary embodiments and functions of the present disclosure are described herein in conjunction with the following drawings, showing schematically:
- Figure 1: a perspective view of a medicament delivery device according to the present disclosure;
- Figure 2: a first side view of the medicament delivery device;
- Figure 3: a second side view of the medicament delivery device;
- Figure 4: an exploded view of the medicament delivery device with a first mechanism according to the present disclosure;
- Figure 5: a cross-sectional cut through the medicament delivery device parallel to a longitudinal axis;
- Figure 6: a first cross-sectional cut through the medicament delivery device in a dose setting state prior to setting a dose;
- Figure 7: a second cross-sectional cut through the medicament delivery device in the dose setting state prior to the setting of the dose;
- Figure 8: a first cross-sectional cut through the medicament delivery device in the dose setting state after setting of the dose;
- Figure 9: a second cross-sectional cut through the medicament delivery device in the dose setting state after setting of the dose;
- Figure 10: a first cross-sectional cut through the medicament delivery device in a dose delivery state prior to delivery of the set dose;
- Figure 11: a second cross-sectional cut through the medicament delivery device in the dose delivery state prior to the delivery of a set dose;
- Figure 12: a first cross-sectional cut through the medicament delivery device in the dose delivery state after delivery of the set dose;
- Figure 13: a second cross-sectional cut through the medicament delivery device in the dose delivery state after delivery of the set dose;
- Figure 14: a perspective view of an outer housing of the first mechanism from a distal end;
- Figure 15: a perspective view of the outer housing from a proximal end;
- Figure 16: a perspective view of a piston rod guide of the first mechanism from a distal end;
- Figure 17: a perspective view of the piston rod guide from a proximal end;
- Figure 18: a perspective view of a piston rod of the first mechanism;
- Figure 19: a perspective view of a piston disc of the first mechanism;
- Figure 20: a top view of the piston disc;
- Figure 21: a section view of the piston disc along a line A-A shown in Figure 20;
- Figure 22: a first perspective view of a nut of the first mechanism;
- Figure 23: a second perspective view of the nut;
- Figure 24: a side view of the nut;
- Figure 25: a first section view of the nut along a line A-A shown in Figure 24;
- Figure 26: a second section view of the nut along a line B-B shown in Figure 24;
- Figure 27: a perspective view of a driver of the first mechanism;
- Figure 28: a top view of the driver;
- Figure 29: a perspective view of a dosing element of a dosing member of the first mechanism from a distal end;
- Figure 30: a perspective view of the dosing element from a proximal end;
- Figure 31: a perspective view of a carrier of the dosing member of the first mechanism;
- Figure 32: a side view of the carrier:
- Figure 33: a section view of the carrier along a line A-A shown in Figure 32;
- Figure 34: a perspective view of a connector of the first mechanism;
- Figure 35: a bottom view of the connector;
- Figure 36: a side view of the connector;
- Figure 37: a top view of the connector;
- Figure 38: a first perspective view of a dose setting element of first mechanism from a distal end;
- Figure 39: a second perspective view of the dose setting element from a proximal end;
- Figure 40: a section side view of the dose setting element;
- Figure 41: a perspective section view of the dose setting element;
- Figure 42: a perspective view of an intermediate member of the first mechanism;
- Figure 43: a top view of the intermediate member;
- Figure 44: a side view of the intermediate member;
- Figure 45: a bottom view of the intermediate member;
- Figure 46: a perspective view of a button of the first mechanism;
- Figure 47: a side view of the button;
- Figure 48: a section view of the button along a line A-A shown in Figure 47;
- Figure 49: a section view of the button along a line B-B shown in Figure 47;
- Figure 50: a section view of the button along a line C-C shown in Figure 47;
- Figure 51: a subassembly of the first mechanism;
- Figure 52: a perspective view of a first embodiment of a dose selector of the first mechanism from a distal end;
- Figure 53: a perspective view of the first embodiment of the dose selector from a proximal end;
- Figure 54: a longitudinal cut through the first embodiment of the dose selector and the carrier of the dosing member in a plane parallel to a longitudinal axis of the first mechanism in the dose setting state;
- Figure 55: a longitudinal cut through the first embodiment of the dose selector and the carrier of the dosing member in a plane parallel to a longitudinal axis of the first mechanism in the dose delivery state;
- Figure 56: a radial cut through the first embodiment of the dose selector;
- Figure 57: a bottom view of a second embodiment of the dose selector from a proximal end;
- Figure 58: a bottom view of a third embodiment of the dose selector from a proximal end;
- Figure 59: a top view of the first embodiment of the dose selector from a distal end;
- Figure 60: a section view of a second mechanism for an automatic medicament delivery device according to the present disclosure in a dose setting state prior to setting a dose;
- Figure 61: a section view of the second mechanism in the dose setting state after setting the dose;
- Figure 62: a section view of the second mechanism in a dose delivery state prior to delivering the dose;
- Figure 63: a section view of the second mechanism in the dose delivery state after delivery of the dose;
- Figure 64: a section view of a third mechanism for an automatic medicament delivery device according to the present disclosure in a dose setting state prior to setting a dose;
- Figure 65: a section view of the third mechanism in a dose delivery state after delivering the dose;
- Figure 66: a section view of a fourth mechanism for an automatic medicament delivery device according to the present disclosure in a dose setting state prior to setting a dose;
- Figure 67: a section view of the fourth mechanism in a dose delivery state after delivering the dose;
- Figure 68: a section view of a fifth mechanism for an automatic medicament delivery device according to the present disclosure in a dose setting state prior to setting a dose;

- Figure 69: a section view of the fifth mechanism in a dose delivery state after delivering the dose.

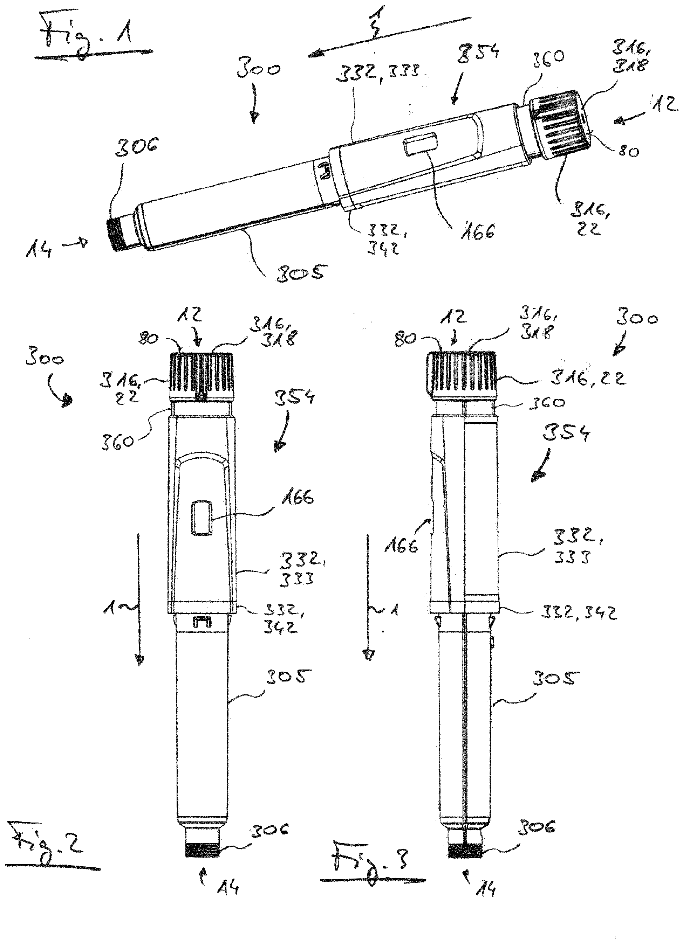

**Figs. 1** to **3** depict a medicament delivery device 300 according to the present disclosure. The medicament delivery device 300 is configured as a pen-type injection device suited to deliver a dose of medicament by injection through the skin of a patient. The medicament delivery device 300 has a generally cylindrical shape that extends from a distal end 12 facing away from the injection site to a proximal end 14 that is located at the injection site. A proximal direction 1 points towards the injection site, that is from the distal end 12 to the proximal end 14. A distal direction is orientated opposite the proximal direction 1 and points from the proximal end 14 to the distal end 12.

The medicament delivery device 300 comprises a first mechanism 354 for setting the dose of medicament to be delivered to the injection site and for delivering the set dose. A container holder 305 that is configured to receive a medicament container with the medicament to be delivered is connected to the proximal end of the first mechanism 354. At the proximal end of the container holder 305, a needle connector 306 is located for connecting a cannula to the medicament delivery device 300. The needle connector 306 is configured as a form-fit connector. It is exemplarily configured as a threaded connector, it may also be configured as a bayonet lock, a Luer lock or the like.

The first mechanism 354 is configured as an automatic mechanism that automatically delivers a set dose after a user has triggered dose delivery. A force that is sufficient to deliver the set dose thereby is provided by a spring of the mechanism so that it is not necessary for a user of the device to provide a force that drives the injection.

At the distal end 12, the first mechanism 354 comprises an actuation unit 316 for setting the dose and for triggering injection of the set dose. The actuation unit 316 comprises a dose setting element 22 and a button 318.

The dose setting element 22 has a cylindrical outer surface that is configured to be gripped by a user of the medicament delivery device 300 to set the dose to be delivered by rotating the dose setting element 22 around the longitudinal axis of the first mechanism 354. Thereby, the rotation in one circumferential direction increases the set dose and rotation in the opposite circumferential direction decreases the set dose. The set dose is indicated in a window 166 that is formed within a housing 332 of the first mechanism 354.

The button 318 is configured as a push button. It has an end surface 80 that is orientated generally perpendicular, such as perpendicular, to the longitudinal axis and that is located at the distal end 12 of the first mechanism 354. The button 318 is configured to be pushed by the user of the device in the proximal direction 1 to transfer the first mechanism 354 from a dose setting state into a dose delivery state. The user thereby pushes upon the end surface 80 of the button 318. Proximal movement of the button 318 initiates automatic dose delivery.

With the embodiment shown in Figures 1 to 3, the dose setting element 22 and the button 318 of the actuation unit 316 are rigidly connected with each other so that they are axially and rotationally fixed to each other. Therefore, proximal movement of the button 318 to also leads to proximal movement of the dose setting element 22 and rotation of the dose setting element 22 also leads to a rotation of the button 318.

The first mechanism 354 comprises a housing connector that is located in between the actuation unit 316 and the housing 332 and forms an outer housing component. The housing connector is exemplarily configured as a dose selector 360.

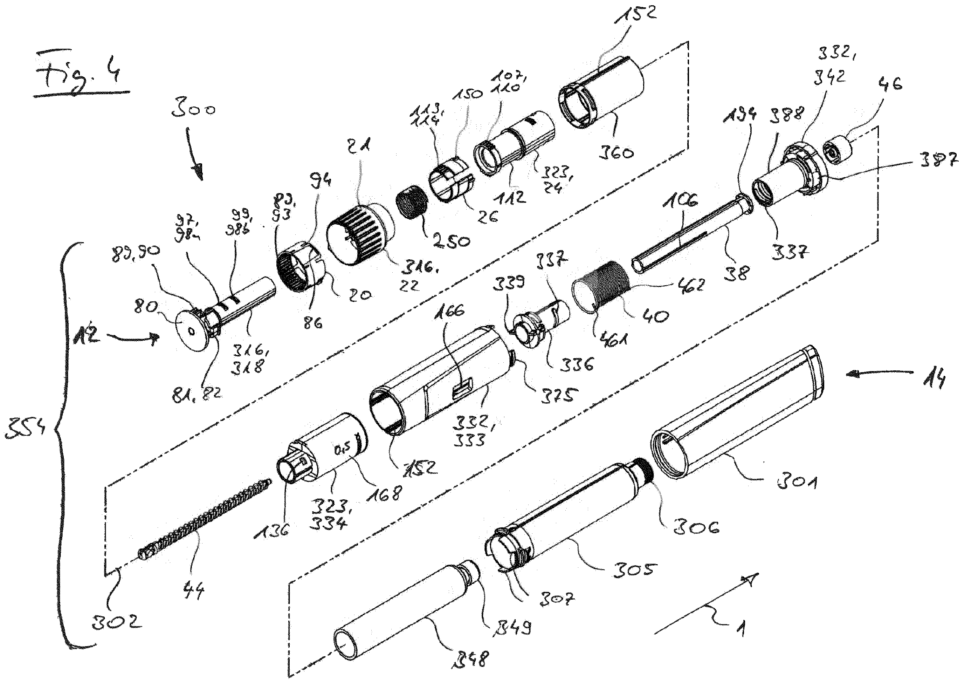

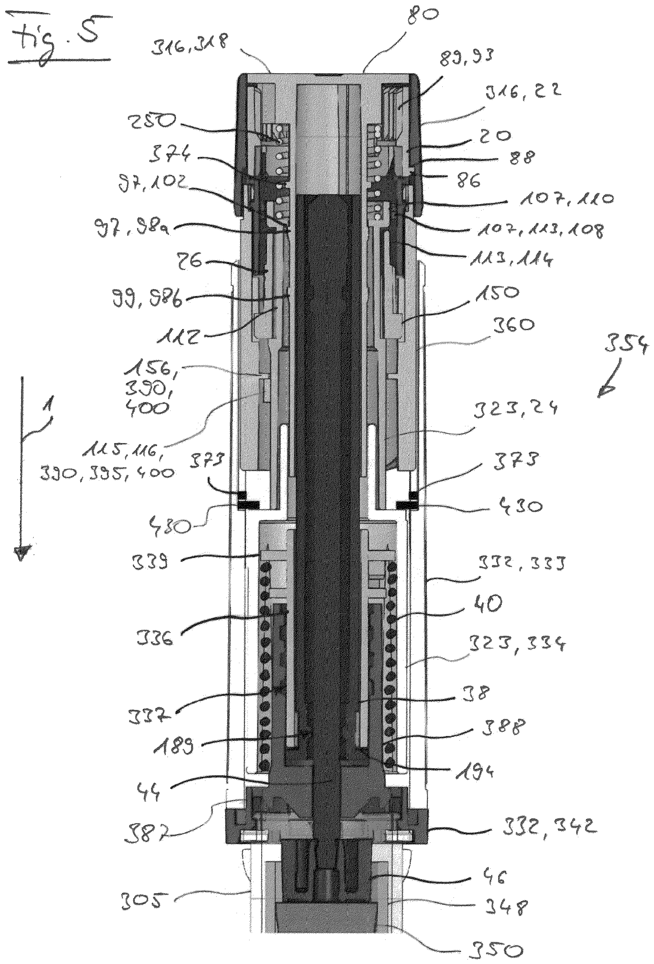

**Fig. 4** shows an exploded view of the medicament delivery device 300 and **Fig. 5** shows a cross-sectional cut through the medicament delivery device 300 parallel to the longitudinal axis, whereby the first mechanism 354 is in the dose setting state.

The housing 332 comprises an outer housing 333 that is rigidly connected to a piston rod guide 342. The outer housing 333 is configured as a hollow generally cylindrical member. It thereby has the shape of a sleeve. The outer housing 333 is axially and rotationally fixed with respect to the piston rod guide 342 by a connector 375. The outer housing 333 and the piston rod guide 342 thereby form a single functional component of the medicament delivery device 300.

The connector 375 is located at a proximal end of the outer housing 333. It exemplarily is configured as a snap-fit connector that irreversibly snaps the outer housing 333 to the piston rod guide 342. With other embodiments, the connector may also be configured as another form-fit connector, such as a screwed connection. It may also be configured as an adhesive bond, such as a glued connection. The connector 375 engages with an outer body 387 of the piston rod guide 342. The outer body 387 is configured as a ring-shaped part of the piston rod guide 342 and located at the proximal end of the piston rod guide 342. The connector 375 engages a distal side of the outer body 387.

The container holder 305 has a generally cylindrical hollow shape and receives the medicament container 348 in its interior. It is coupled to the housing 332 by a connector 307 that is located at the distal end of the container holder 305. The connector 307 engages with the piston rod guide 342 at the proximal side of the outer body 387 of the piston rod guide 342. The connector 307 is configured as a form-fit connector, namely as a snap-fit connector. With other embodiments, the connector 307 may also be configured as another form-fit connector, such as a screwed connection, or as an adhesive bond.

At a needle end 349 of the medicament container 348, which needle and 349 is located at the proximal end of the medicament container 348, the medicament container 348 comprises a septum that seals the interior of the medicament container 348. The septum is configured to be pierced by a double-ended cannula upon mounting the cannula onto the needle connector 306 of the container holder 305.

With the embodiment shown in Figures 1 to 5, the medicament delivery device 300 is configured as a disposable device that is disposed of after delivery of a last dose from the medicament container 348. The container holder 305 is non-releasably connected to the housing 332 by the connector 307. The container holder 305 then may not be removed from the housing 332 without breaking the first mechanism 354 and/or the container holder 305. With other embodiments, the medicament delivery device 300 may also be configured as a reusable device that allows to attach a new medicament container 348 after having delivered a last dose from the medicament container 348 attached to the first mechanism 354. With such embodiments, the connector 307 may be configured as a releasable connector, such as a screwed connection or a bayonet lock.

The medicament delivery device 300 comprises a cap 301 that is configured to be releasably attachable to the proximal end 14 of the medicament delivery device 300 to cover the container holder 305 and the needle connector 306.

To expel a medicament from the medicament container 348, the first mechanism 354 comprises a piston rod 44. The piston rod 44 protrudes from the first mechanism 354 at its proximal end. It thereby protrudes through the piston rod guide 342. At the proximal end of the piston rod 44, a bearing 46 is located that pushes upon a movable plunger 350 (see Fig. 5) that seals the medicament container 348 towards its distal end.

The piston rod 44 is rotationally fixed with respect to the piston rod guide 342 and the outer body 333. The piston rod 44 has an outer thread that engages with an inner thread of a nut 38. The nut 38 is configured as a hollow generally cylindrical member that is disposed around the piston rod 44.

The nut 38 is connected to the button 316 of the actuation unit 318, whereby the nut 38 is rotationally fixed and axially movable with respect to the button 316. With the embodiment shown in Fig. 4, the first mechanism 354 exemplary comprises a splined connection between the nut 38 and a cylindrical proximal part of the button 316. A distal portion of the nut 38 is received within the button 316. The splined connection comprises longitudinal grooves 106 at one of the nut 38 and the button 316 and corresponding longitudinal ridges that engage with the grooves 106 and that are provided at the other one of the nut 38 and the body 316. Exemplarily, the longitudinal grooves 106 are provided at an outer surface of the nut 38 and the longitudinal ridges are provided on an inner surface of the button 316.

The button 316 is rigidly connected to the dose setting element 22 by an intermediate member 20. The intermediate member 20 is located within the cylindrical portion of the dose setting element 22 and is rotationally and axially fixed to both the dose setting element 22 and the button 316.

The first mechanism 354 further comprises a driver 336 that has a generally cylindrical hollow body and that is disposed around the nut 38. The driver 336 is threadedly connected to the housing 323, namely to the piston rod guide 342, via a drive thread 337. It engages the piston rod guide 342 via the drive thread 337. The drive thread 337 is formed between a distal inner body 388 of the housing 332 and the driver 336. The inner body 388 forms a distal portion of the piston rod guide 342 that is located distally from the outer body 387.

An inner thread of the drive thread 337 is formed at an inside surface of the inner body 388 and an outer thread of the drive thread 337 is formed on an outside surface of the body of the driver 336.

The first mechanism 354 further comprises a dosing member 323 having a carrier 24 that forms a first part of the dosing member 323 and a dosing element 334 that forms a second part of the dosing member 323. The carrier 24 forms a coupling member that rotationally couples the dosing member 323 to the dose setting element 22 during dose setting.

With the embodiment shown in Figures 1 to 5, the dosing element 334 and the carrier 24 are rigidly connected with each other so that they are axially and rotationally fixed with respect to each other. With the embodiment shown, the dosing element 334 and the carrier 24 are configured as two separate parts that are joined together. With other embodiments, the dosing element 334 and the carrier 24 may also be configured as a single materially uniform element.

The dosing member 323 is held axially fixed and rotationally movable within the housing 332. Both the dosing element 334 and the carrier 24 are configured as generally cylindrical hollow members. The dosing element 334 is located in the proximal direction 1 with respect to the carrier 24.

The dosing element 334 forms a dose sleeve. The dosing element 334 surrounds the piston rod 44 and the nut 38. Furthermore, it surrounds the inner body 388 of the housing 332 and the driver 336.

The driver 336 is rotationally movable and axially fixed with respect to the dosing member 323 and connected to the dosing member 323 by a connector 339. It thereby directly engages with the dosing member 323, namely with the dosing element 334 of the dosing member 323. The connector 339 is configured as a rotation lock. It is exemplarily a splined connection and comprises a longitudinal ridge that engages with a corresponding longitudinal groove. With the present embodiment, the longitudinal ridge is exemplarily formed at an outside surface of the driver 336 and the dosing member 323 comprises the corresponding longitudinal groove on an inside surface, namely on an inside surface of the dosing element 334. With other embodiments, the connector 339 may also rotationally fix the driver 336 to the dosing member 323 in other ways.

A spring 40 is coupled between the driver 336 and the housing 332. The spring 40 thereby is directly coupled between the driver 336 and the housing 332. A first end 461 of the spring 40 is attached to the driver 336 and a second end 462 of the spring 40 is attached to the housing 332. The spring 40 surrounds the inner body 388 of the housing 332.

The dose selector 360 is configured as a hollow generally cylindrical member. It surrounds the dosing member 323, namely the carrier 24 of the dosing member 323. Furthermore, the dose selector 360 is located inside the housing 332 and protrudes from the distal end of the housing 332.

The dose selector 360 is connected axially fixed and rotationally movable to the actuation unit 318. A distal end of the dose selector 360 thereby engages with a proximal end of the cylindrical body of the dose setting element 22. The dose selector 360 further is rotationally fixed and axially movable with respect to the housing 332. A rotation lock 152 between the dose selector 360 and the housing 332 is configured as a splined connection. The splined connection comprises longitudinal ridges that engage with corresponding longitudinal grooves. Exemplarily, the splined connection comprises longitudinal grooves formed at an inside surface of the housing 332 and corresponding longitudinal ridges that are formed at an outside surface of the dose selector 360. With other embodiments, the rotation lock 152 may also be configured in other ways. For example, the ridges may be provided at the housing 332 and the grooves may be provided at the dose selector 360.

A connector 26 is disposed around the dosing member 323, namely around the carrier 24. The connector 26 is axially fixed and rotationally movable with respect to the dosing member 323. It is disposed around a coupling section 112 of the carrier 24. The coupling section 112 is located at an outside surface of the dosing member 323 and is recessed with respect to the outer surface. The connector 26 is axially fixed with respect to the dosing member 323 by abutting against both longitudinal ends of the recessed section 112.

The connector 26 is rotationally fixed with respect to the housing 332. It is thereby rotationally fixed to the housing 332 via the dose selector 360. A rotation lock 150 between the connector 26 and the dose selector 360 rotationally fixes the connector 26 to the dose selector 360 while allowing axial movement. The rotation lock 150 is exemplarily configured as a splined connection having a longitudinal rib provided at an outside surface of the connector 26 and a corresponding longitudinal groove provided at an inside surface of the dose selector 360. With other embodiments, the rotation lock 150 may be configured in different ways, for example the groove may be provided at the connector 26 and the rib may be provided at the dose selector 360.

The proximal portion of the button 318 protrudes into the distal portion of the dosing member 323, as can be seen from Fig. 5. The button 318 thereby protrudes into the carrier 24 of the dosing member 323.

The button 318 is biased with respect to the housing 332 and the dosing member 323 by a biasing member 250 that is exemplarily configured as a compression spring. The biasing member 250 is located between the dosing member 323 and the button 318. Since, with the present embodiment, the button 318 is axially fixed with respect to the dose setting element 22 and the dose selector 360, the biasing member 250 also biases the dose setting element 22 and the dose selector 360 in the distal direction.

A retainer 97 prevents detachment of the button 318 and the dose setting element 22 from the housing in the distal direction. The retainer 97 acts between the button 318 and the dosing member 323. It restrains distal movement of the button 318 and the dose setting element 22 with respect to the housing 332 but allows for distal movement.

The retainer 97 has a first retainer element 98a that engages with a second retainer element 102 to block distal movement of the button 318 and the dose setting element 22. The first retainer element 98a is exemplarily provided at the button 318, namely at an outside surface of the proximal portion of the button 318. The second retainer element 102 is exemplarily provided at the dosing member 323. The first retainer element 98a is configured as a protrusion located at the outside surface of the button 318 and the second retainer element 102 is configured as a radially inwardly extending ledge.

The biasing member 250 biases the button 318 in the distal direction towards the second retainer element 102. Movement of the button 318 in the proximal direction 1 is possible against the force provided by the biasing member 250 and disengages the first retainer element 98a from the second retainer element 102.

The first mechanism 354 comprises a clutch 113 that acts between the nut 38 and the piston rod 44 and that is exemplarily formed between the dose setting element 22 and the connector 26. The first mechanism 354 additionally comprises a further clutch 107 that acts between, on the one hand, the dose setting element 22 and, on the other hand the dosing member 323 and the driver 336. The further clutch 107 is exemplarily formed between the dose setting element 22 and the dosing member 323.

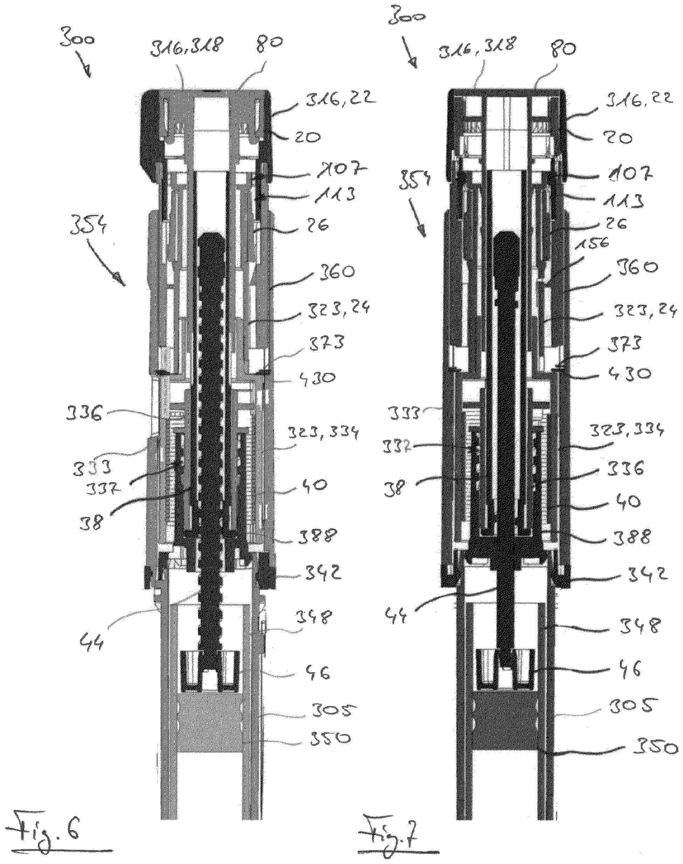

**Fig. 6** depicts a first cross-sectional cut through the medicament delivery device 300 in a first cut plane and **Fig. 7** shows a corresponding second cross-sectional cut through the medicament delivery device 300 in a second cut plane perpendicular to the first cut plane, whereby the medicament delivery device 300 is in a dose setting state prior to setting a dose.

In the dose setting state, the button 318 and the dose setting element 22 are in their distal position with respect to the dosing member 323. In this position, the dose setting element 22 is rotationally fixed to the dosing member 323 via the further clutch 107, which is in its closed state. Additionally, the clutch 113 is in its opened state so that the nut 38 and the dose setting element 22 are rotationally movable with respect to the piston rod 44 and the housing 332.

When rotating the dose setting element 22 during dose setting, the dosing member 323 rotates due to the closed further clutch 107 between the dose setting element 22 and the dosing member 323. Rotation of the dosing member 323 also forces a corresponding rotation of the driver 336, which is therefore moving axially relative to the piston rod guide 342 due to its threaded connection to the piston rod guide 342 via the drive thread 337. When increasing the set dose, the driver 336 moves in the distal direction away from the piston rod guide 342, and when decreasing the set dose, the driver 336 moves in the proximal direction 1 towards the piston rod guide 342.

Rotation of the driver 336 during dose setting stores energy in the spring 40. The spring 40 is thereby strained and the energy stored in the spring 40 is increased when increasing the set dose. Upon decreasing the set dose, the spring 40 is relaxed and the energy stored in the spring 40 is decreased. When no dose is set, the spring 40 may be pretensioned so that it biases the driver 336 in the proximal direction 1.

Since the nut 38 is rotationally fixed with respect to the button 318, rotation of the dose setting element 22 also causes rotation of the nut 38 during dose setting. Thereby, the nut 38 is screwed along the piston rod 44 and also moves into the distal direction. A pitch of the thread of the piston rod 44 and a pitch of the drive thread 337 are adapted so that the nut 38 and the driver 337 essentially move the same axial distance upon rotation. Thereby, a nominal pitch of the drive thread 337 is slightly higher than a nominal pitch of the thread between the piston rod 44 and the nut 38 to prevent mutual blocking of the nut 38 and driver 336 irrespective of manufacturing tolerances. In detail, the minimum tolerable pitch of the drive thread 337 is larger than the minimum tolerable pitch of the thread between the nut 38 and the piston rod 44.

During dose setting, the dosing member 323 rotates with respect to the dose selector 360. As it is detailed below, the first mechanism 354 has a dose definition mechanism 115 that acts between the dosing member 323 and the housing 332. The dose definition mechanism 115 defines distinct relative rotational positions of the dosing member 323 with respect to the housing 332 that correspond to doses settable by the first mechanism 354. At each rotational position of the dosing member 323 that corresponds to a settable dose, the dose definition mechanism 115 rotationally locks the dosing member 323 with respect to the housing 332 against the force provided by the spring 40. This allows a user of the first mechanism 354 to release the dose setting element 22 without decreasing the set dose. In between the rotational positions corresponding to settable doses, the dose definition mechanism 115 allows the dosing member 323 to freely rotate back to the next lower settable dose. This prevents unintentional setting of intermediate doses.

The dosing member 323 comprises a label 168 that is provided at an outer surface of the dosing member 323 and that is visible through the window 166 in the housing 332 when a dose has been set. The label 168 thereby indicates that the dose has been set, for example it may indicate the amount of dose that has been set.

With some embodiments of the first mechanism 354, only a single dose may be set by a user. With other embodiments, more than a single dose, such as two or more doses may be set by the user. For each settable dose, the dosing member 323 comprises a corresponding label 168 at its outer surface.

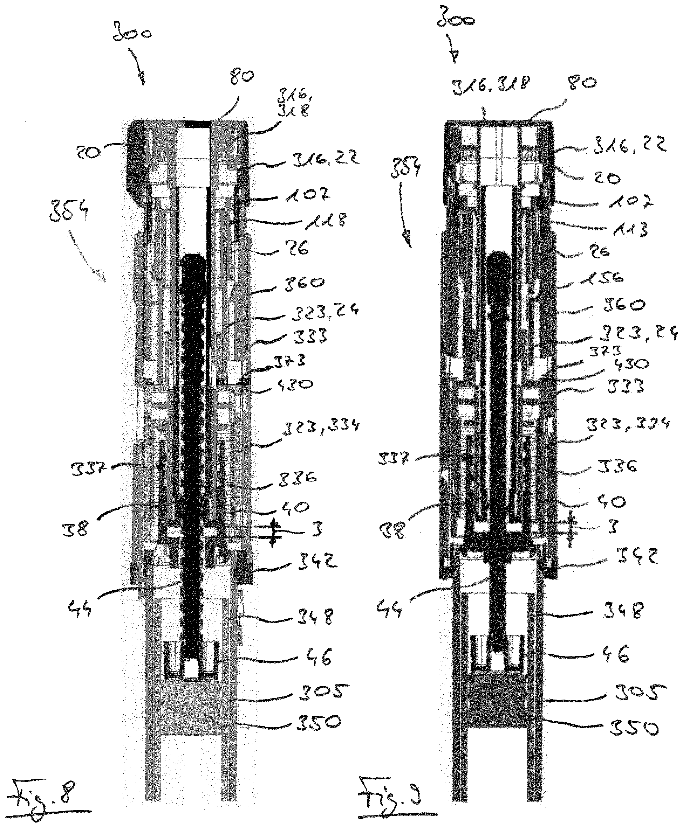

**Fig. 8** depicts a first cross-sectional cut through the medicament delivery device 300 in the first cut plane and **Fig. 9** shows a corresponding second cross-sectional cut through the medicament delivery device 300 in the second cut plane, whereby the first mechanism 354 is in the dose setting state after the setting the dose.

The nut 38 has been moved by a dose distance 3 in the distal direction away from the proximal end 14 of the first mechanism 354. The driver 336 has likewise traveled a corresponding distance in the distal direction.

To inject the set dose, the user moves the button 318 in the proximal direction 1. Thereby, also the dose setting element 22 and the dose selector 360 are moved into the proximal direction 1 with respect to the housing 332 and the dosing member 323. This opens the further clutch 107 between the dosing member 323 and the dose setting element 22 and closes the clutch 113 between the dose setting element 22 and the connector 26 that surrounds the dosing member 323.

By proximally moving the button 318, the first mechanism 354 is transferred from its dose setting state into a dose delivery state.

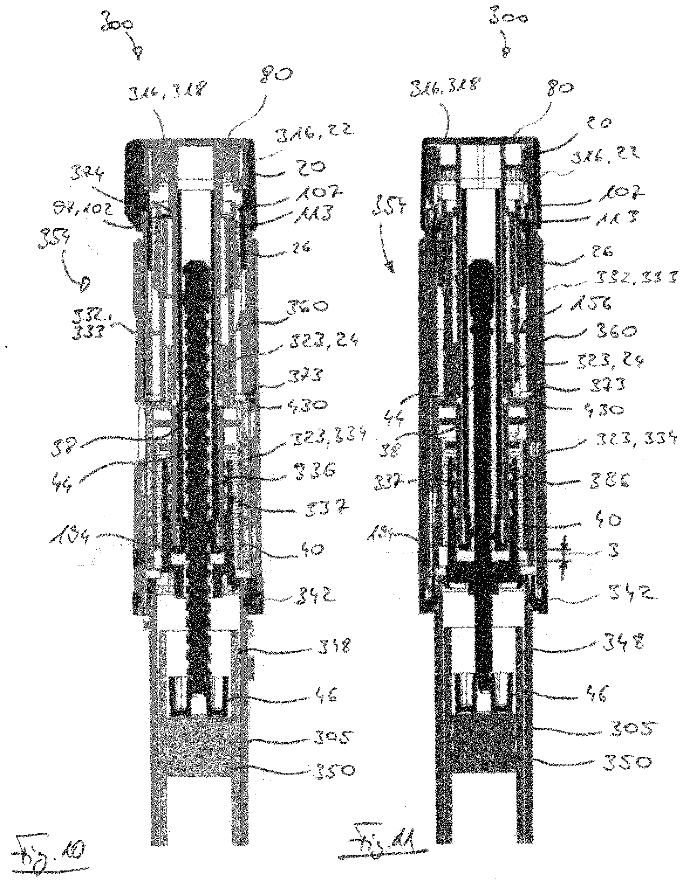

**Fig. 10** shows a first cross-sectional cut through the medicament delivery device 300 in the first cut plane and **Fig. 11** shows a corresponding second cross-sectional cut through the medicament delivery device 300 in the second cut plane, whereby the first mechanism 354 is in the dose delivery state prior to delivery of the set dose.

The closing of the clutch 113 between the dose setting element 22 and the connector 26 rotationally fixes the nut 38 to the housing 332 via the button 318, the dose setting element 22, the connector 26 and the dose selector 360. Since the piston rod 44 is rotationally fixed with respect to the housing 332, the closing of the clutch 113 also rotationally fixes the nut 38 to the piston rod 44. Closing of the clutch 113 also prevents rotation of the dose setting element 22 and the button 318 with respect to the housing 332. The opening of the further clutch 107 rotationally decouples the dose setting element 22 from the dosing member 323 and the driver 336.

The transfer of the first mechanism 354 from the dose setting state into the dose delivery state by moving the button 318 in the proximal direction 1 also disengages the dose definition mechanism 115 so that the dosing member 323 is no longer prevented from rotating with respect to the housing 332 at the rotational positions that correspond to settable doses. Generally speaking, disengagement of the dose definition mechanism 115 thus allows the driver 336 to freely rotate with respect to the housing 332, whereby the driver 337 is driven by the spring 40.

This rotation screws the driver 336 in the proximal direction 1 into the piston rod guide 342 and therefore also moves the driver 336 axially in the proximal direction 1. Upon proximal movement, the driver 336 abuts the nut 38 via an axial stop 194. This axially locks the driver 336 to the nut 38 so that the driver 336 and the nut 38 move together in the proximal direction 1.

With the present embodiment, the axial stop 194 is provided at a proximal end of the nut 38. It is exemplarily configured as an annular ring that radially protrudes from the nut 38. When moving proximally with respect to the nut 38, the driver 336 engages with the axial stop 194 at its proximal end.

Due to closure of the clutch 113, the nut 38 is rotationally fixed to the piston rod 44 and the housing 332 via the button 318, the dose setting element 22, the connector 24 and the dose selector 360. This locks the drive thread 337 so that the nut 38 and the piston rod 44 are axially fixed with respect to each other. Axial advancement of the nut 38 then causes a corresponding axial advancement of the piston rod 44. As a consequence, the spring 40 automatically drives the driver 337, the nut 38 and the piston rod 44 in the proximal direction 1 to deliver the set dose.

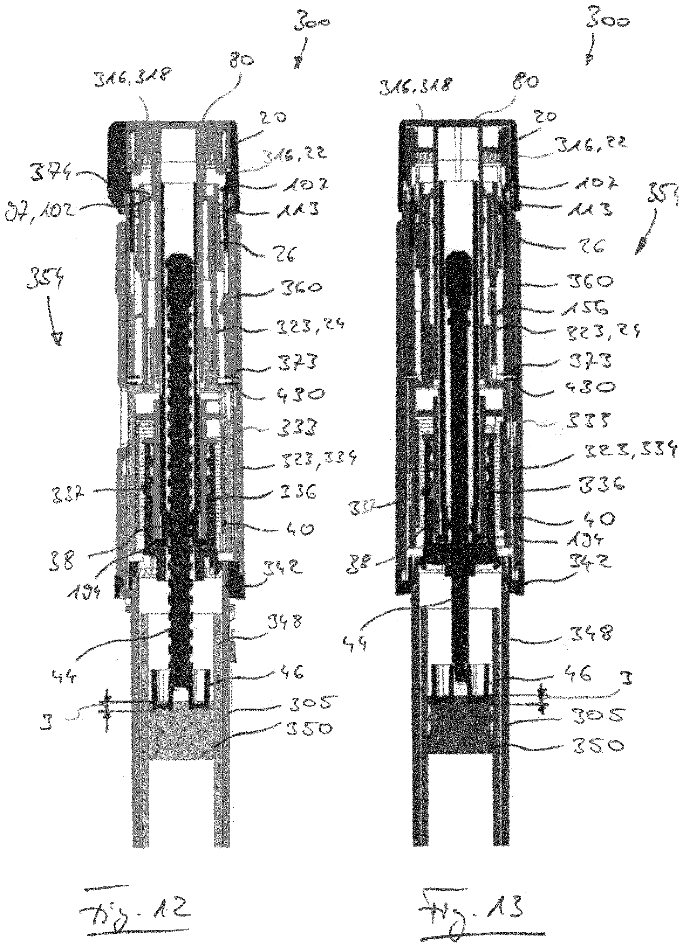

**Fig. 12** depicts a first cross-sectional cut through the medicament delivery device 300 in the first cut plane and **Fig. 13** shows a corresponding second cross-sectional cut through the medicament delivery device 300 in the second cut plane, whereby the first mechanism 354 is in the dose delivery state after delivery of the set dose. The piston 44 and the bearing 46 located at the proximal end of the piston rod 44 have been moved by the dose distance 3 in the proximal direction 1. This has also pushed the plunger 350 by the dose distance 3 in the proximal direction 1, thereby expelling an amount of medicament that corresponds to the volume covered by the dose distance 3.

As can be seen from Fig. 5, the dosing member 323 is axially held within the housing 332 by a blocker 430 that acts between the button 318 and the housing 332 and blocks the button 318 from moving axially with respect to the housing 332 in the distal direction 1. With the present embodiment, the blocker 430 acts between the housing 332 and the dosing member 323. The blocker 430 blocks distal movement of the dosing member 323 with respect to the housing 332. It is axially and rotationally fixed with respect to the housing 332 and rotatably engages with the dosing member 323, namely with the dosing element 334. In the present embodiment, the blocker 430 engages with a distal end surface of the dosing member 323. With other embodiments, the blocker 430 may also engage with other parts of the dosing member 323. The dosing member 323, namely the dosing element 333, forms a counter member of the blocker 430.

The blocker 430 is directly located at the outer housing 333 of the housing 332 and protrudes from the inside surface of the outer housing 333. With other embodiments, the blocker 430 may also be located at an additional housing component that is at least axially fixed to the outer housing 333. For example, the additional housing component may be axially and rotationally fixed to the outer housing 333.

A connection between the additional housing component and the outer housing 333 may, for example, be configured as a form-fit connection, such as a snap-fit connection or a threaded connection. The connection may be configured to assemble the additional housing component to the outer housing 333 after placing the dosing member 323 inside the outer housing 333. For example, the connection may be configured to allow assembly of the additional housing component to the outer housing 333 from the distal end of the outer housing 333.

The blocker 430 is configured as a one-way blocker that allows passage of the dosing member 323 in the proximal direction 1 but blocks passage of the dosing member 323 in the distal direction. The blocker 430 forms a flexible element that bends away from the dosing member 323 upon passage in the proximal direction 1 and interferes with the dosing member 323 upon passage in the distal direction. After passage of the dosing member 323, the blocker 430 snaps into a blocking position that blocks distal movement of the dosing member 323. As it is shown in Fig. 5, the first mechanism 354 may comprise additional blockers 430. The additional blockers 430 may be distributed around the circumference of the housing 332.

With other embodiments, the blocker 430 may also be configured as a rigid element. The blocker 430 then may be fixed to the housing 332 after having placed at least parts of the dosing member 323, such as the dosing element 333, within the housing 323.

With alternative embodiments, the blocker 430 may also be fixed to the dosing member 323. The blocker 430 then may interfere with features, such as protrusions or grooves, provided at the housing 332, such as at the inside surface of the housing 332.

Movement of the dosing member 323 in the proximal direction 1 is restrained by the housing 332, namely by the piston rod guide 342. The dosing member 323 thereby abuts a distal facing surface of the proximal outer body 387 of the piston rod guide 342.

With other embodiments of the first mechanism 354, the blocker 430 may only be configured to restrain or block movement of the dosing member 323 in the distal direction. Movement of the button 318 and/or the dose setting element 22 then may be restrained or blocked by other mechanisms.

As can also be seen from Fig. 5, the first mechanism 354 comprises a stop 373 that limits proximal movement of the button 318 during dose delivery. The stop 373 is axially fixed to the housing 332. It interferes with a component that is axially fixed with respect to the button 318. In the present embodiment, the stop 373 interferes with a housing connector that is formed by the dose selector 360. When reaching the dose delivery state, the axially fixed component formed by the dose selector 360 abuts the stop 373, thus preventing further proximal movement of the button 318. The housing 332 forms a first stop part of the stop 373 and the dose selector 360 forms a second stop part of the stop 373.

With the present embodiment, the stop 373 is located at the proximal end of one of the grooves of the rotation lock 152 formed at the inside surface of the dose selector 360. For example, the stop 373 may be formed by a proximal end of the one of the grooves.

The stop 373 is directly located at the of the outer housing 333, exemplarily it is integrally formed with the outer housing 333. With other embodiments, the outer housing 333 may also be located at a separate component that is at least axially fixed, such as axially and rotationally fixed, to the outer housing 333. With some embodiments, the separate component may also comprise the blocker 430.

As can be seen from Fig. 5, the first mechanism 354 may comprise additional stops 373. The stops 373 may be distributed circumferentially around the longitudinal axis of the first mechanism 354.

With alternative embodiments, a stop that limits proximal movement of the button 318 during dose delivery may also act between the button 318 and the dosing member 323. Such a stop 374 is also indicated in Fig. 5. It is configured as a radial protrusion that is located at the outside surface of the proximal part of the button 318. The protrusion thereby circumferentially surrounds the proximal part of the button 318. Upon proximal movement of the button 318 with respect to the dosing member 323, the stop 374 abuts against the radially inwardly protruding ring at the distal end of the dosing member 323 that also forms the second retainer element 102. This is also visible in Figs. 10 to 13. With other embodiments, the second retainer element 102 and the counter element of the stop 374 may be formed as separate features.

While both the stops 373 and the stops 374 are shown in Figures 5 to 13, the first mechanism 354 may only feature either the stops 373 or the stops 374.

With the first mechanism 354, the button 318 is the last component that is added to the first mechanism 354 during assembly. The first mechanism 354 comprises a priming mechanism that allows to adjust the axial position of the piston rod 44 and the bearing 46 with respect to the housing 332 in a preassembled state of the first mechanism 354. Within this preassembled state, the button 318 has been inserted into the dosing member 323 but not been pushed fully in the proximal direction 1 to engage with the intermediate member 20 to become axially and rotationally fixed to the dose setting element 22. In this position, a further first retainer element 98b that is located proximally from the first retainer element 98a engages with the second retainer element 102. The button 318 then is axially restrained in the distal direction with respect to the housing 332 and can rotationally move with respect to the dose setting element 22. This allows to rotate the nut 38 by rotating the button 318 and to thereby axially move the piston rod 44 due to the threaded connection between the nut 38 and the piston rod 44. Details of this priming mechanism are described in application US 17/981231, which is incorporated by reference in its entirety.

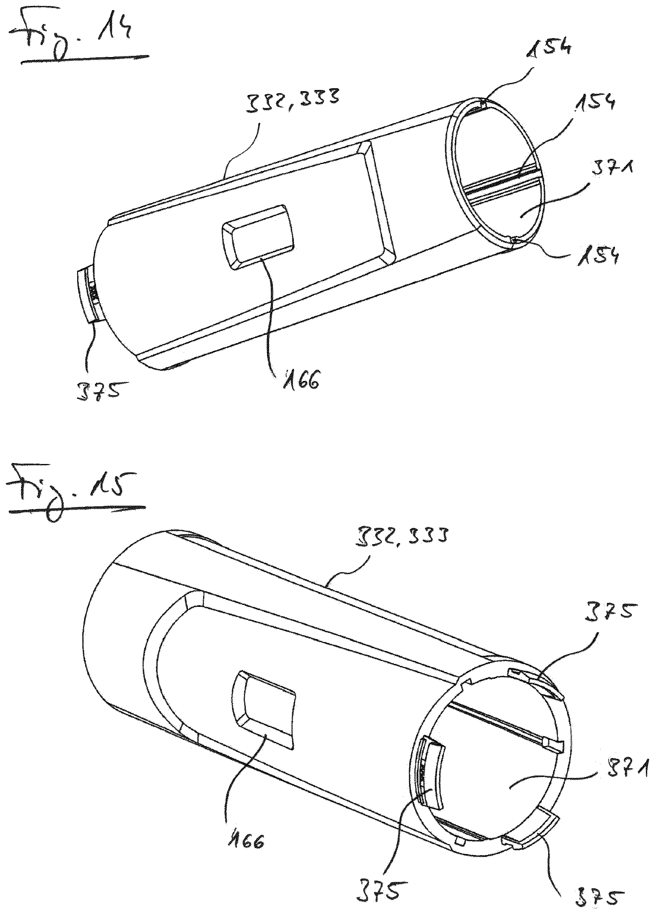

**Fig. 14** shows a perspective view of the outer housing 333 of the first mechanism 354 from a distal end and **Fig. 15** shows a perspective view of the outer housing 333 from a proximal end. At the inside surface 371 of the outer housing 333, the grooves of the rotation lock 154 are formed. Furthermore, three of the connectors 375 are protruding from the proximal end of the outer housing 333.

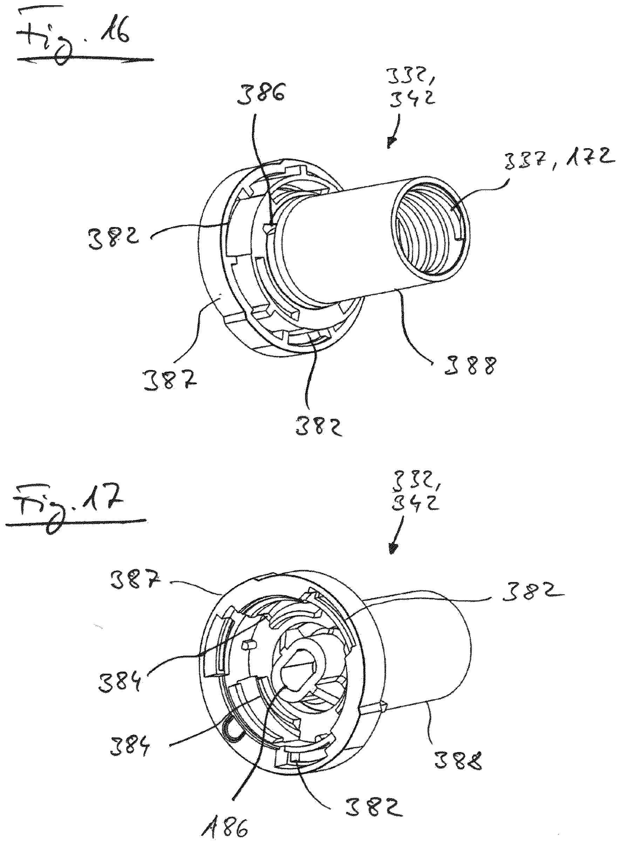

**Fig. 16** shows a perspective view of the piston rod guide 342 of the first mechanism 354 from a distal end and **Fig. 17** shows a perspective view of the piston rod guide 342 from a proximal end. At the inside surface of the inner body 388, an inner thread 172 of the drive thread 337 is formed. At the proximal end of the inner body 388 and on the outer surface of the inner body 388, a spring connector 386 is formed that fixes the second end 462 of the spring 40 to the piston rod guide 342. The spring connector 386 is exemplarily formed as an opening within the outer surface of the inner body 388 and the second end 462 of the spring 40 is bent to engage with this opening.

As can be seen from Fig. 16, a housing connector 382 is formed at the distal side of the outer body 387 of the piston rod guide 342. The housing connector 382 is configured as several recesses in which corresponding snap hooks of the connector 375 formed at the proximal end of the outer housing 333 engage upon assembly of the outer housing 333 to the piston rod guide 342.

The piston rod 44 is guided within an opening 186 of the piston rod guide 342, which is visible in Fig. 17. The opening 186 is formed as an out of round axial opening that corresponds to an out of round cross-section of the piston rod 44. Therefore, the piston rod 44 is axially movable relative to the piston rod guide 342, but cannot rotate relative to the piston rod guide 342.

A container connector 384 is located next to the opening 186. The container connector 384 is formed by openings that receive corresponding snap-fit hooks of the connector 307 that are formed at the distal end of the container holder 305.

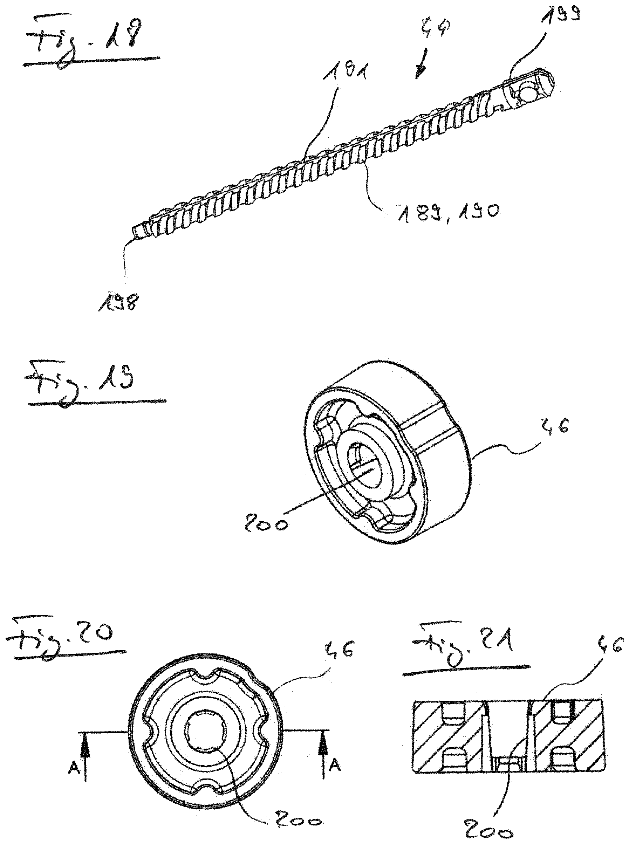

**Fig. 18** shows a perspective view of the piston rod 44 of the first mechanism 354. On its outer surface, the piston rod 44 has an outer thread 190 that forms part of the threaded connection 189 between the piston rod 44 and the nut 38. In addition, the piston rod comprises two opposing flattened sections 191 that provide the out-of-round cross-section of the piston rod 44 that corresponds to the out-of-round cross-section of the opening 186 of the piston rod guide 342. These out-of-round cross-sections rotationally lock the piston 44 to the piston rod guide 342 while allowing axial relative movement.

At its proximal end, the piston rod 44 comprises a proximal connector 198 that engages with the bearing 46 to hold the bearing 46 at the piston rod 44. At its distal end, the piston rod 44 comprises a stop 199 that is configured as a thickened and non-threaded section of the piston rod 44.

**Fig. 19** shows a perspective view of the bearing 46 of the first mechanism 354, **Fig. 20** shows a top view of the bearing 46 and **Fig. 21** shows a section view of the bearing 46 along a line A-A shown in Fig. 20. The bearing 46 comprises a bearing connector 200 that is configured as a central opening. The bearing connector 200 features angled side surfaces that snap over the proximal connector 198 of the piston rod 44 when the proximal connector 198 is inserted into the central opening 200.

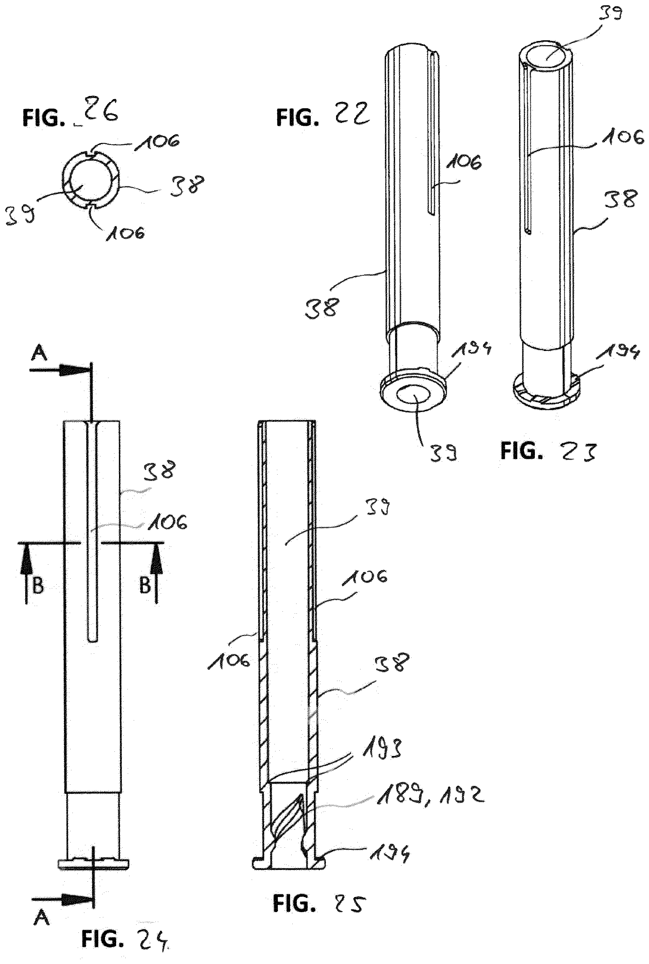

**Figs. 22** to **26** depict the nut 38 of the first mechanism 354. On an inner surface of a central opening 39, the nut 38 comprises an inner thread 192 of the threaded connection 189 between the piston rod 44 and the nut 38. The inner thread 192 is thereby located at the proximal end of the nut 38. The piston rod 44 and the nut 38 can move relative to each other in a compulsory guided combined axial and rotational movement.

On the outside surface, the nut 38 features the axial stop 194 that is formed as an annular protrusion. The axial stop 194 is located in a proximal end section of the nut 38. The axial stop 194 abuts a front surface 196 of the driver 336 during dose delivery. In order to reduce friction during dose delivery, the axial stop 194 is formed as protrusions extending distally from the annular protrusion. This reduces the contact area between the driver 376 and the nut 38. With alternative embodiments, a bearing element, such as a ball bearing and/or a glide disc made of low-friction material, may be arranged between the axial stop 194 and the front surface 196 of the driver 336.

The first mechanism 354 comprises a last dose mechanism that prevents dialling a dose that is larger than a remaining amount of medicament within the medicament container 348. The last dose mechanism is formed by the stop 199 at the distal end of the piston rod 44 and an axial stop 193 provided on the inside surface of the opening 39 of the nut 38.

Upon repeatedly setting and delivering a dose, the piston rod 44 successively moves in the proximal direction 1 with respect to the nut 38. This successively reduces a distance between the stop 199 provided at the piston rod 44 and the axial stop 198 provided on the inside surface of the nut 38 after each dose delivery. Prior to delivering a first dose from the medicament container, the distance between the stop 199 and the axial stop 198 has an initial value. The initial value is adapted to reduce upon repeated delivery of set doses by an amount that prevents further distal movement of the nut with respect to the piston rod 44 if a user sets a dose that is larger than a remaining amount of medicament within the medicament container 348.

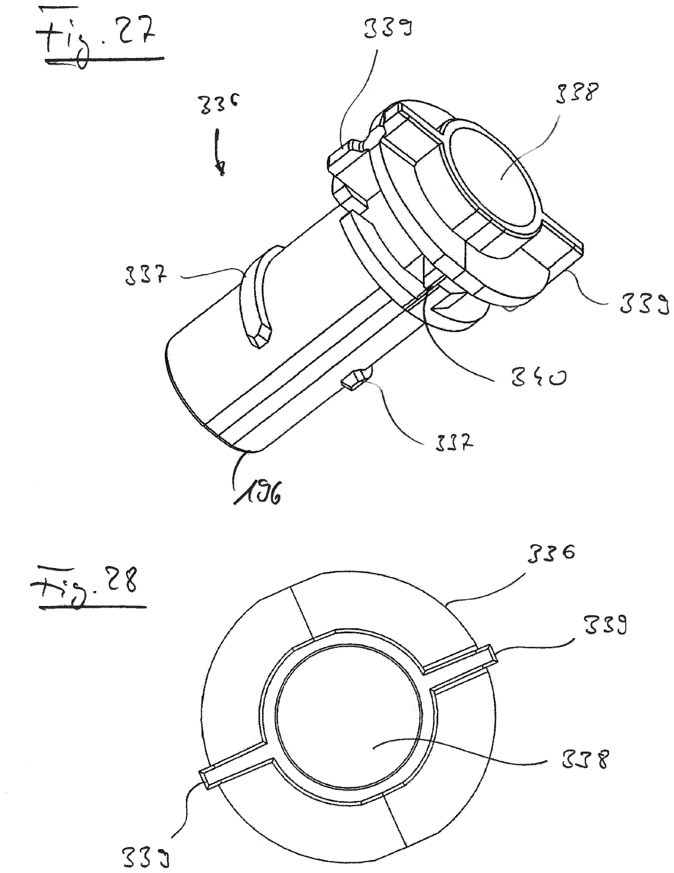

**Fig. 27** shows a perspective view of the driver 336 of the first mechanism 354 and **Fig. 28** shows a top view of the driver 336. The driver 336 has an opening 338 that receives the nut 38. The opening 338 is configured as a central axial opening. In its distal end section, the driver 336 comprises a spring connector 340 that is configured to connect to the first end 461 of the spring 40. Furthermore, the driver 336 comprises the connector 339 in its distal end section. The connector 339 comprises two radially outwardly directed ribs that engage with the dosing element 334 of the dosing member 323.

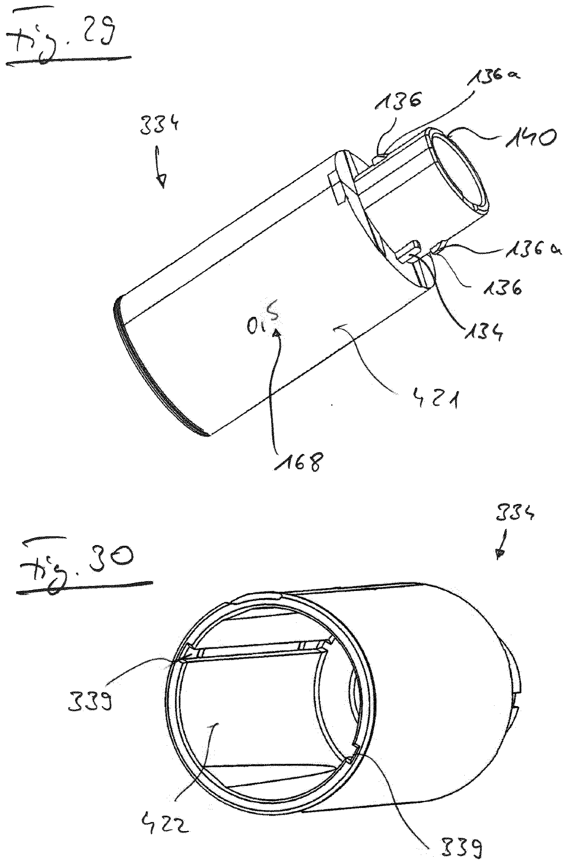

**Fig. 29** shows a perspective view of the dosing element 334 of the dosing member 332 of the first mechanism 354 from the distal end and **Fig. 30** shows a perspective view of the dosing element 334 from the proximal end.

On an outer surface 421, the dosing element 334 has the label 168 that indicates setting of one of the settable doses. On an inner surface 422, the dosing element 334 comprises longitudinal grooves of the connector 339 that connects rotationally fixed and axially movable to the driver 336. The longitudinal grooves engage with the outwardly directed ribs provided on the outside surface of the driver 336.

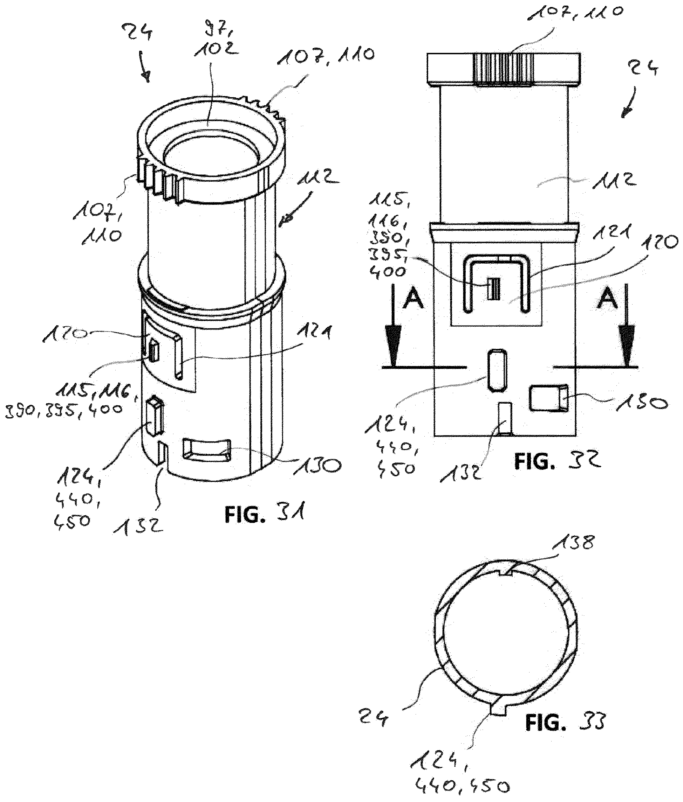

**Figs. 31** to **33** depict the carrier 24 of the dosing member 323 of the first mechanism 354. The carrier 24 comprises axial and rotational fixation means to axially and rotationally fix the carrier 24 to the dosing element 334. The axial and rotational fixation means are provided at a proximal end of the carrier 24. They are configured as a radially extending opening 130 and an axially extending slot 132.

As can be seen in Fig. 29, the dosing element 334 has an axially extending rib 134 that is configured to engage with the slot 132 of the carrier 24 to rotationally lock the dosing element 334 to the carrier 24. Furthermore, the dosing element 334 has a protrusion 136 with a chamfered surface 136a that engages with the opening 130 of the carrier 24. While the opening 130 and the protrusion 136 form the axial fixation means, the slot 132 and the rib 134 form the rotational fixation means. Due to the axial and rotational fixation means, the carrier 24 and the dosing element 334 can be connected to each other in one defined relative rotational position. In order to strengthen the rotational fixation between the carrier 24 and the dosing element 334, an axially extending rib 138 is formed on an inner circumferential surface of the carrier 24 (cf. Fig. 33) that engages with an axially extending groove 140 (cf. Fig. 29) on an outer circumferential surface of the dosing element.

On its outer surface, the carrier 24 forms an axial section with a reduced circumference that forms the coupling section 112 for the connector 26.

The carrier 24 further comprises a counter element 116 of the dose definition mechanism 115 that defines the rotational positions of the dosing member 323 that correspond to settable doses. The counter element 116 is located on an outer surface of the carrier 24. It is exemplarily configured as an axially extending radial projection. Furthermore, the counter element 116 forms an axially extending rib. The counter element 116 may have a symmetrical cross section in a radial plane perpendicular to the longitudinal axis of the first mechanism 354 or an asymmetrical cross section.

The counter element 116 is resiliently fixed to the carrier 24. It is configured to deflect in the radial direction. With the present embodiment, the counter element 116 is located on an elastically deformable section 120 of the carrier 24. The elastically deformable section 120 is formed by an axially extending arm partially surrounded by a cut-out 121. The elastically deformable section 120 bends inwardly when the counter element 116 passes a dose stop.

The carrier 24 further comprises a blocking part 124. The blocking part 124 radially extends from the outer surface of the carrier 24. The blocking part 124 is configured as a hard stop. It is formed by an axially extending rib that extends along the longitudinal axis of the first mechanism 354. The blocking part 124 is axially distanced from the counter element 116. Furthermore, it is rotationally and axially aligned with the counter element 116.

Furthermore, a further first engaging part 110 of the further clutch 107 is located at the carrier 24. The further first engaging part 110 is configured as radially projecting teeth at the outer surface of the carrier 24. It is located at a distal end of the carrier 24. The radially projecting teeth thereby only cover sections of the circumference of the outer surface of the carrier 24, whereby the sections are separated by sections having no teeth. With other embodiments, the teeth may also cover the complete circumference of the outer surface of the carrier 24.

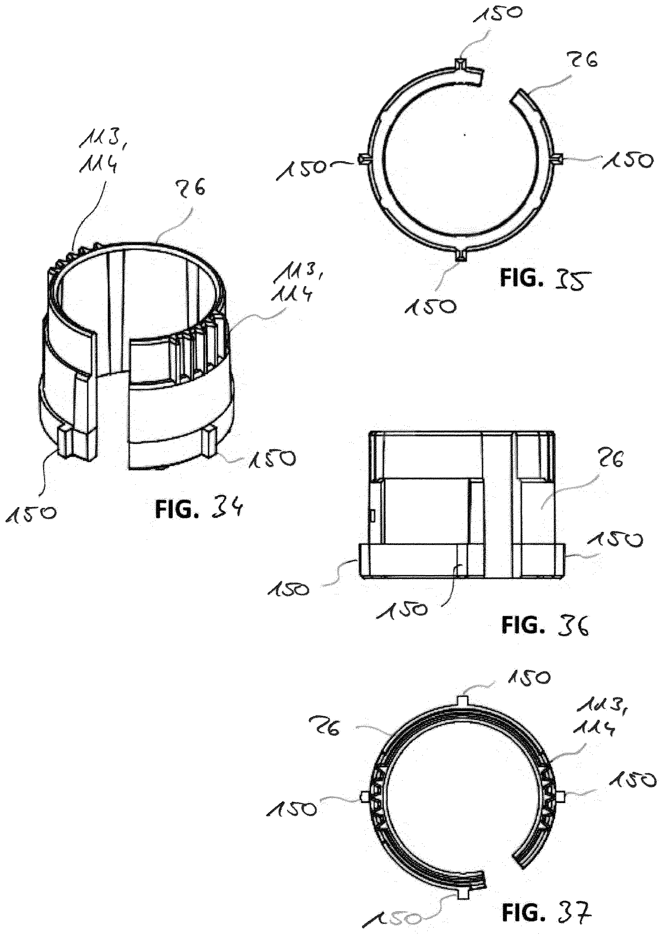

**Figs. 34** to **37** depict the connector 26 of the first mechanism 354. The connector 26 is configured as a hollow generally cylindrical member. It has a longitudinal cut that extends along the entire length of the connector 26. The connector 26 has an open cross section along its entire longitudinal length. This allows to clip the connector over the coupling section 112 of the carrier 24 during assembly of the first mechanism 354.

The connector 26 is axially fixedly connected to the carrier 24 in both directions due to the connector 26 having a length in the axial direction that corresponds to a length in the axial direction of the reduced circumference of the coupling section 112. At the same time, the connector 26 is rotatable relative to the carrier 24.

The connector 26 comprises a first engaging part 114 of the clutch 113. The first engaging part 114 is configured as radially extending teeth that are located on an outer surface of the connector 26. Like with the further first engaging part 110 of the further clutch 107, the teeth of the first engaging part 114 of the clutch 113 only cover sections of the circumference of the connector 26, which sections are separated by sections that have no teeth. With other embodiments, the teeth may also cover the complete circumference of the outer surface of the connector 26.

The first engaging part 114 is located at a distal end of the connector 26. At a proximal end, the connector 26 comprises the longitudinal ribs of the rotation lock 150.

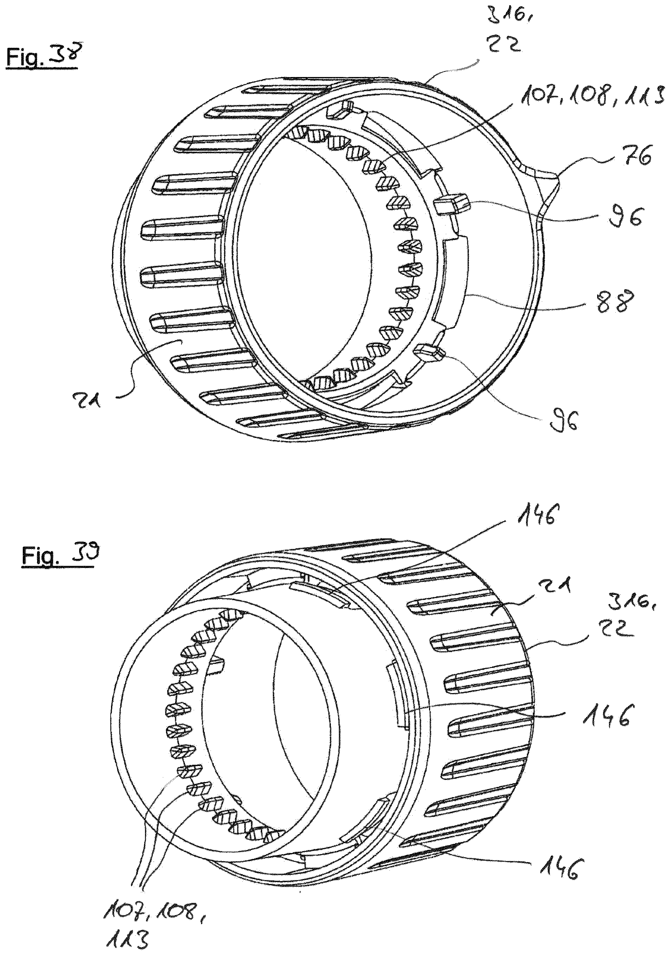

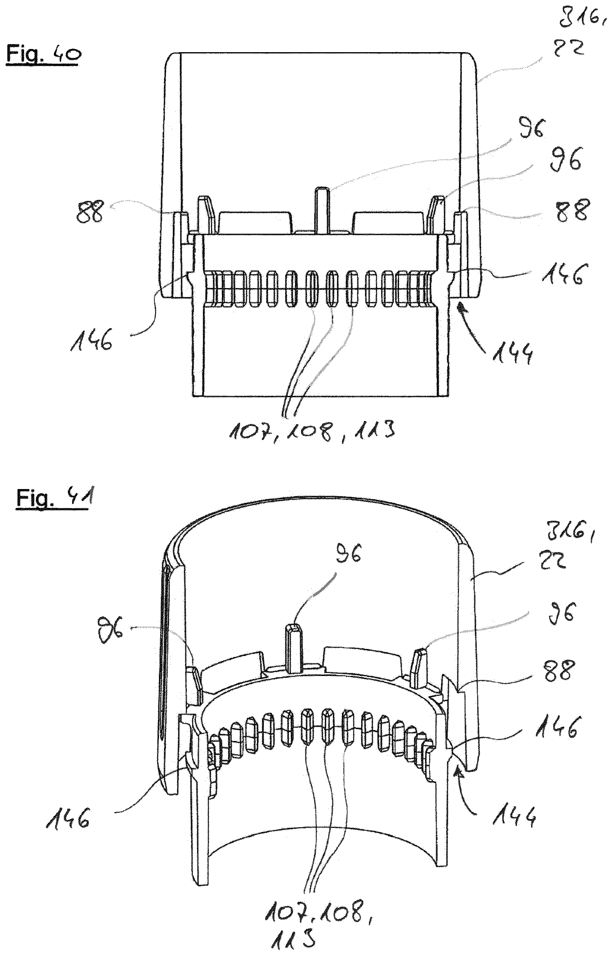

**Figs. 38** to **41** depict the dose setting element 22 of the first mechanism 354. The dose setting element 22 is configured as a generally cylindrical element that longitudinally extends along the longitudinal axis of the first mechanism 354. Furthermore, it is configured as a hollow member.

The dose setting element 22 comprises a second engaging part 108 of the clutch 113. The second engaging part 108 is configured as radially extending teeth that are disposed around a circumference of a cylindrical surface of the dose setting element 22. The second engaging part 108 thereby is located at an inside surface of the dose setting element 22. The teeth radially extend inwardly from the inside surface.

The dose setting element 22 also comprises a further second engaging part of the further clutch 107. The further second engaging part is thereby formed by the second engaging part 108 of the clutch 113.

On its outer surface, the dose setting element 22 comprises a gripping surface 21 and a radially protruding rib 76 that extends in the axial direction. The rib 76 forms an anti-rolling feature that prevents the first mechanism 354 from rolling when being put on a flat surface.

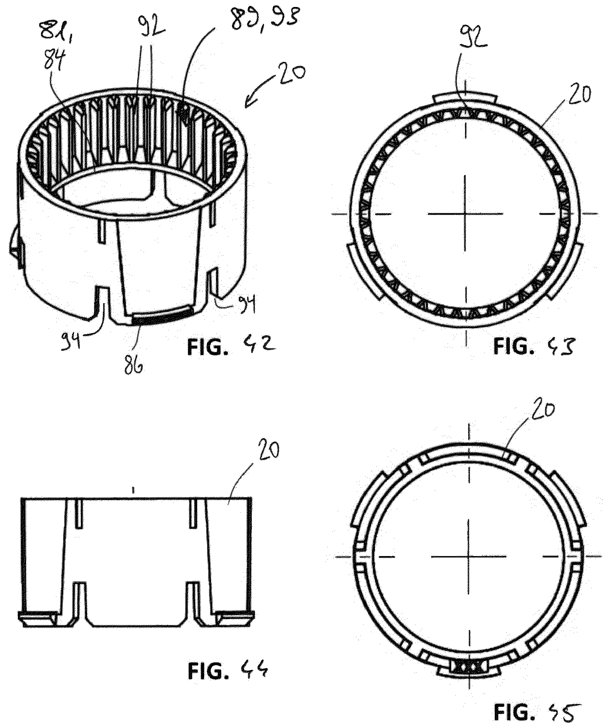

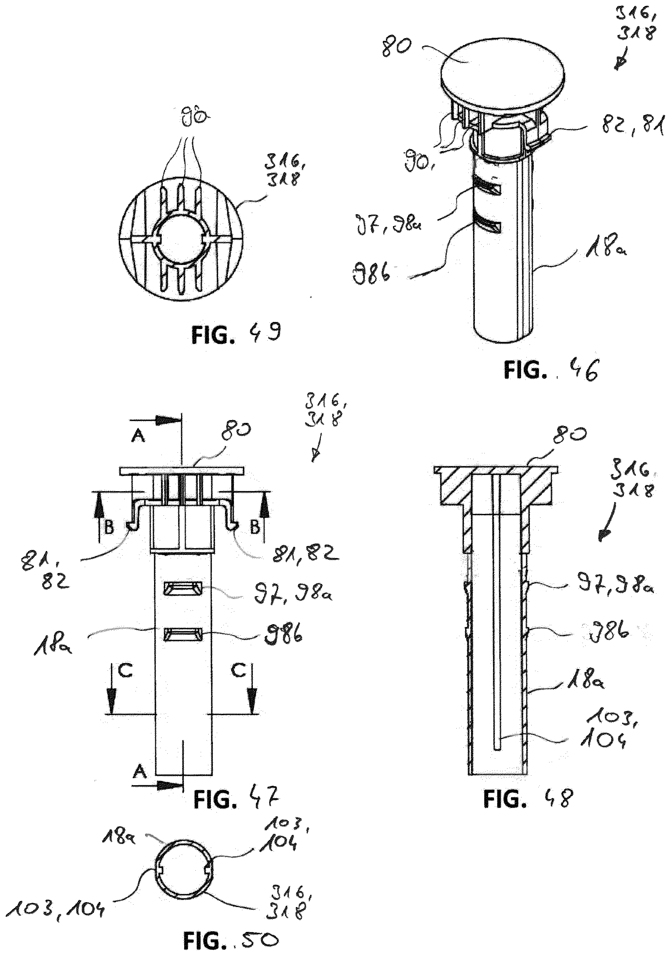

**Figs. 42** to **45** depict the intermediate member 20 of the first mechanism 354 and **Figs. 46** to **50** depict the button 318 of the first mechanism 354. The button 318 has the distal end surface 80 to apply a force to the button 318 to inject a set dose. The button 318 comprises axial fixation means 82 to axially attach the button 318 to the intermediate member 20 which is axially fixed to the dose setting element 22. The axial fixation means 82 comprise two elastically deformable hooks 82 which engage with a circumferentially extending rib 84 on the intermediate member 20. The intermediate member 20 also comprises axial fixation means 86 in the form of elastically deformable bendable hooks that engage with an undercut 88 formed in the dose setting element 22. The button 318, the intermediate member 20 and the dose setting element 22 are permanently axially fixed to each other in an assembled state of the first mechanism 354. With other embodiments, the button 318 may also be axially fixed with respect to the dose setting element 22 by other means. For example, the button 318 may directly engage with the dose setting element 22 without the intermediate member 20.

The button 318 also has rotation fixation means 90 in the form of radially extending ribs. The ribs 90 are form-fittingly engaged with rotation fixation means 92 of the intermediate member 20, whereby the rotation fixation means 92 are exemplarily formed as teeth arranged on an inner circumferential surface of the intermediate member 20. The rotation fixation means 90, 92 rotationally fix the button 318 to the intermediate member 20. The rotation fixation means 92 of the intermediate member 20 form a toothed part 93 of the intermediate member 20 and the ribs 90 form an engaging part of the button 318. The intermediate member 20 comprises rotation fixation means 94 in the form of axially extending recesses that define side surfaces of the elastically deformable bendable hooks 86 and that engage with rotation fixation means 96 in the form of axially extending ribs on the inner circumferential surface of the dose setting element 22.

After assembly and in an assembled state of the dose delivery mechanism 54, the button 318, the intermediate member 20 and the dose setting element 22 are rigidly connected with each other and form the actuation unit 316 of the mechanism 354.

The button 318 further has a cylindrical portion 18a. The cylindrical portion 18a is configured as a hollow member. It also forms the proximal part of the button 318. With the first mechanism 354, the cylindrical portion 18a is integrally formed with a distal part of the button 318 that forms the distal end surface 80. With other embodiments, the part forming the end surface 80 and the cylindrical portion 18a may be configured as separate elements that are rigidly connected to each other and axially and rotationally fixed with respect to each other.

On the cylindrical portion 18a, the first retainer element 98a of the retainer 97 is located. Furthermore, the cylindrical portion 18a also comprises the further first retainer element 98b.

As can be best seen in Figs. 48 and 50, the button 318 comprises an axially extending rib 104 on its inner circumferential surface. The axially extending rib 104 engages in the axially extending groove 106 of the nut 38 to form a rotation lock 103. Due to the rotation lock 103, the button 318 and the nut 38 can move axially relative to each other but are rotationally fixed to each other.

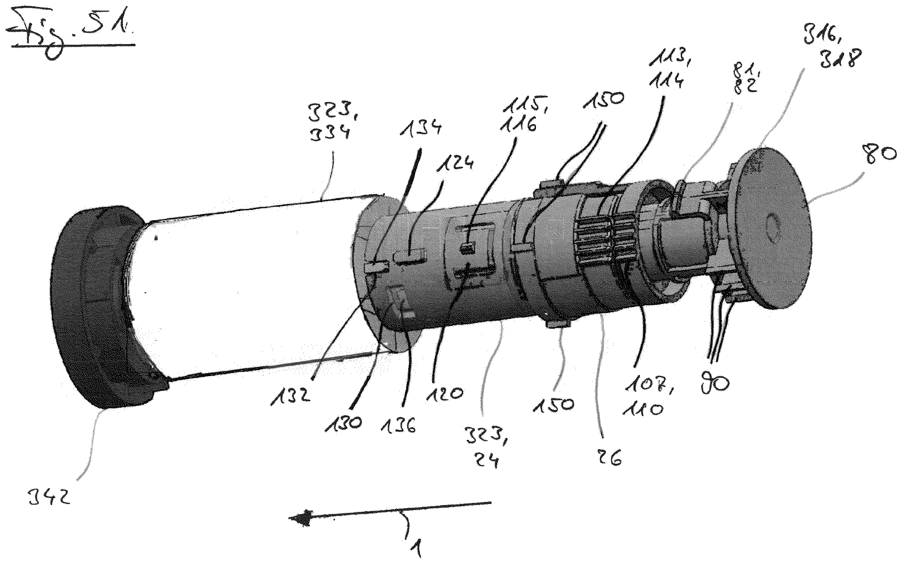

**Fig. 51** depicts a subassembly of the first mechanism 354 that comprises the piston rod guide 342, the dosing member 323, the connector 26 and the button 318.

The first engaging part 114 of the clutch 113 and the further first engaging part 110 of the further clutch 107 are located next to each other. Thereby, the first engaging part 114 is located proximally from the further first engaging part 110. When the first mechanism 354 is in the dose setting state, the actuation unit 316 with the button 318 and the dose setting element 22 is in its distal position. The further second engaging part 108 of the dose setting element 22 engages with the further first engaging part 110 of the further clutch 107 to rotationally fix the dose setting element 22 to the dosing member 323.

Upon transfer of the first mechanism 354 from the dose setting state to the dose delivery state, the actuation unit 316 and the button 318 move proximally. This disengages the further first and second engaging parts 108, 110 of the further clutch 107 and engages the first engaging part 108 and the second engaging part 114 of the clutch 113. This rotationally fixes the dose setting element 22 carrying the first engaging part 108 to the connector 26 carrying the second engaging part. As a consequence, the nut 38 is rotationally fixed with respect to the piston rod 44.

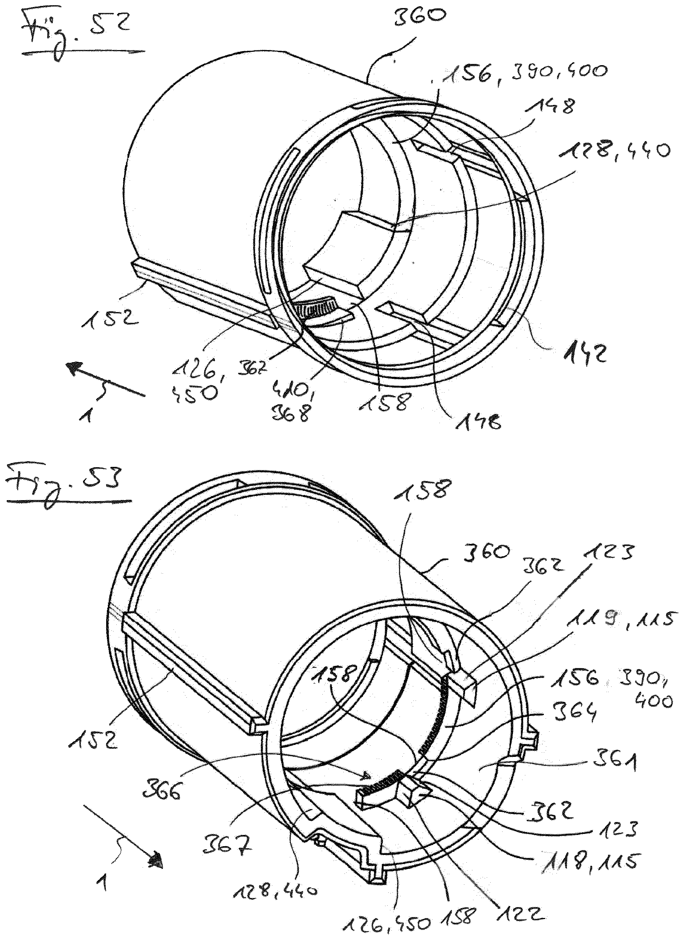

**Fig. 52** depicts a perspective view of a first embodiment of the dose selector 360 of the first mechanism 354 from a distal end and **Fig. 53** depicts a perspective view of the first embodiment of the dose selector 360 from a proximal end.

The dose selector 360 comprises axial fixation means 142 in the form of circumferentially extending projections on an inner surface 361 of a distal section of the dose selector 360. The dose selector 360 is axially fixed to the dose setting element 22 by inserting the distal section with the axial fixation means 142 into a circumferentially extending intake 144 of the dose setting element 22. In the intake 144, the dose setting element 22 has axial fixation means 146 in the form of circumferentially extending protrusions on an outer circumferential surface which get engaged with the axial fixation means 142 of the dose selector 360 to form an axial connection that allows relative rotational movement between the dose selector 360 and the dose setting element 22.

As can be seen from Fig. 52, rotation fixation means 148 in the form of axially extending grooves are located on the inner surface 361 of the dose selector 360. The rotation fixation means 148 form part of the rotation lock 150 between the dose selector 360 and the connector 26. The grooves of the rotation fixation means 148 thereby engage with the protrusions of the rotation lock 150 formed on the outer surface of the connector 26. The rotation lock 150 allows axial movement between the dose selector 28 and the connector 26.

The dose selector 360 further comprises the axially extending ribs of the rotation lock 152 formed on an outer circumferential surface of the dose selector 360. The ribs of the rotation lock 152 engage with the corresponding grooves formed on the inner circumferential surface of the housing 332. The rotation lock 152 thereby is configured to define one single possible rotational alignment that allows insertion of the dose selector 360 into the housing 322.

As it is shown in Fig. 53, the dose selector 360 comprises a dose stop 118 of the dose definition mechanism 115 on the inner surface 361. The dose stop 118 is configured as a radial protrusion that extends from the inner surface 361. Besides the dose stop 118, the dose definition mechanism 115 comprises a further dose stop 119 that is configured as it is disclosed for the dose stop 118. The dose stop 118 and the further dose stop 119 are located at the same axial position on the dose selector 360. They are spaced from each other in the circumferential direction. With other embodiments, the dose selector 360 may only comprise a single dose stop 118.

The circumferential positions of the individual dose stops 118, 119 relative to the housing 332 define individual relative rotational positions of the dosing member 323 and the driver 336 with respect to the housing 332 that correspond to settable doses.

When the first mechanism 354 is in the dose setting state, the dose stop 118 of the dose definition mechanism 115 and the counter element 116 provided at the carrier 24 are axially aligned with each other along the longitudinal axis of the first mechanism 354. The counter element 116 then engages with the individual dose stops 118, 119 upon rotating past the individual dose stops 118, 119. While passing over one of the dose stops 118, 119, the counter element 116 deflects radially inward and snaps over the respective dose stop 118, 119.

The first mechanism 354 further comprises a zero dose mechanism 450 that defines a zero dose position of the dosing member 323, in which no dose is set. The zero dose position also corresponds to the rotational position of the dosing member 323 with respect to the housing 332 that the dosing member 323 takes at the end of dose delivery.

The zero dose mechanism 450 comprises a zero dose stop 126 that is located on the inside surface 361 of the dose selector 360. The zero dose stop 126 is formed by a longitudinal edge that radially protrudes inwardly from the inner surface 361.

The zero dose stop 126 is configured to engage with the blocking part 124 formed on the outer surface of the carrier 24. When reaching the zero dose position, the blocking part 124 rotationally abuts the zero dose stop 126. This prevents further rotational movement of the dosing member 323 with respect to the housing 332.

An angular distance between the zero dose stop 126 and the individual dose stops 118, 119 defines the amount of medicament of the individual doses corresponding to the respective dose stop 118, 119.

Dialing of intermediate doses in between the individual dose stops 118, 119 is prevented by the spring 40 that is provided between the piston rod guide 342 and the driver 336. Since the spring 40 is loaded when increasing the set dose, it causes the dosing member 323 to rotate back to the last set dose in cases where the dose setting element 22 is released while the counter element 116 on the carrier 24 is positioned in between two dose stops 118, 119.

The dose selector 360 further comprises a maximum dose stop 128 of a maximum dose mechanism 440. The maximum dose stop 128 is also located on the inside surface 361 of the dose selector 360. It is formed by a longitudinal edge that radially protrudes inwardly from the inner surface 361. Like the zero dose stop 126, the maximum dose stop 128 is configured as a hard stop.

The maximum dose mechanism 440 further comprises the blocking part 124 that is configured to engage with the maximum dose stop 128 after dialing past a maximum dose setting, which is given by the further dose stop 119 with the embodiment shown in Figures 52 and 53. In general, the blocking part 124 of the carrier 24 is configured to abut the maximum dose stop 128 upon rotation of the dosing member 323 into a rotational position that corresponds to or exceeds a maximum settable dose and thereby prevents further rotation of the dosing member 323.

With the embodiment shown in Figs. 52 and 53, the maximum dose stop 128 and the zero dose stop 126 are configured as opposing side surfaces of a step located on the inner surface 361 of the dose selector 360.

The first mechanism 354 further comprises a blocking mechanism 400 that prevents a transfer of the first mechanism 354 from the dose delivery state to the dose setting state during dose delivery. The blocking mechanism 400 comprises a first element 156 that is located at the dose selector 360 and a second element that is formed by the counter element 116 of the carrier 24.

The first element is configured as a rib 156 that longitudinally extends around the longitudinal axis of the first mechanism 354. It is located on the inside surface 361 of the dose selector 360. Furthermore, it is located adjacent to the dose stops 118, 119 on a distal side from the dose stops 118, 119.

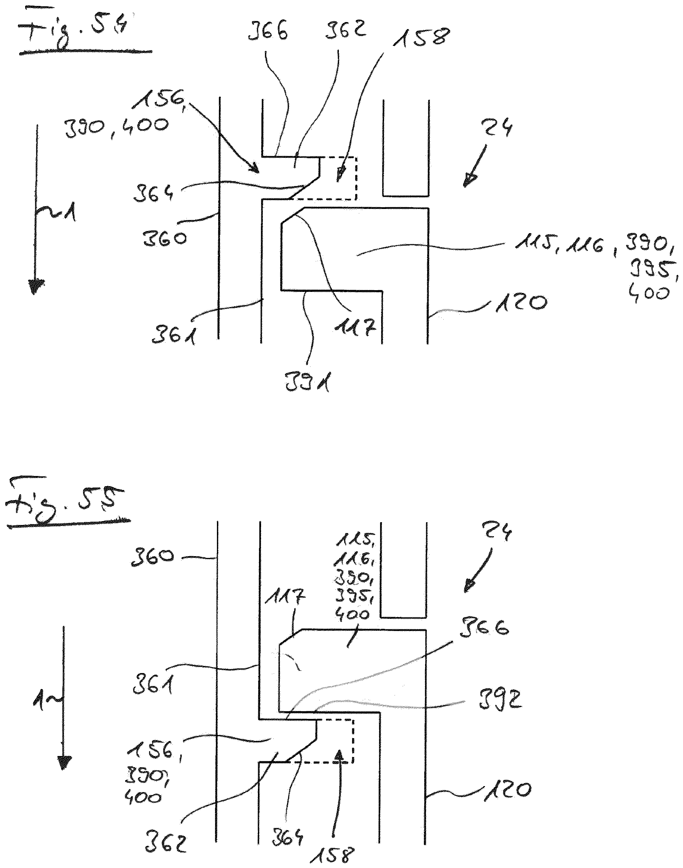

**Fig. 54** shows a longitudinal cut through the dose selector 360 and the carrier 24 of the dosing member 323 in a plane parallel to a longitudinal axis of the first mechanism 354 in the dose setting state. **Fig. 55** shows a longitudinal cut through the dose selector 360 and the carrier 24 of the dosing member 323 in the plane parallel to a longitudinal axis of the first mechanism 354 in the dose delivery state.

During dose setting, the counter element 116 is located in the proximal direction 1 from the rib 156. Upon transfer of the first mechanism 354 from the dose setting state to the dose delivery state, the dose selector 360 is moved in the proximal direction 1 with respect to the dosing member 323. This moves the counter element 116 in the distal direction to the opposite side of the rib 156. Generally speaking, the counter element 116 located at opposing sides of the rib 156 in the dose delivery state and in the dose setting state.

The rib 156 is configured to allow axial movement of the counter element 116 past the rib 156 upon transfer of the first mechanism 354 from the dose setting state to the dose delivery state and to block axial movement of the counter element 116 past the circumferential rib 156 during dose delivery. To this end, the rib 156 exemplarily comprises openings 158 located at the dose stops 118, 119 that allow to pass the counter element 116 from one side of the rib 156 to the opposite side of the rib 156. Thereby, the counter element 116 passes through the openings 158.

To prevent the counter element 116 from moving back towards the dose stops 118, 119 through the openings 158 upon release of the button 318, the rib 156 comprises recessed sections 362 that are formed at the openings 158. The recessed sections 362 radially extend inwardly on the inside surface 361 by a distance that is less than a radial extent of the rib 156. The counter element 116 is configured to snap over the recessed section 362 when transferring the first mechanism 354 from the dose setting state into the dose delivery state and to interfere with the recessed sections 362 upon release of the button 318 in the dose delivery state. This then blocks the counter element 116 from moving back towards the dose stops 118, 119

As can be seen from Figures 54 and 55, the recessed sections 362 each comprise a chamfer 364 that faces towards the counter element 116 in the dose setting state. The counter element 116 comprises a corresponding chamfer 117. Upon axial movement of the counter element 116 against the recessed section 362, the chamfers 117, 364 glide along each other and thus facilitates deflection of the counter element 116 in the radial direction.

Corresponding end surfaces of the rib 156 and the counter element 116 that are located opposite the chamfers 117, 364 in the axial direction are orientated perpendicular to the longitudinal axis and thus do not feature a chamfer. As can be seen from Fig. 55, the counter element 116 then becomes blocked by the rib 156 when moving in the proximal direction 1. With other embodiments, the end surfaces of the rib 156 and the counter element 116 that engage upon blocking movement of the counter element 116 in the proximal direction 1 respect to the dose selector 360 may also be angled to deflect the counter element 116 radially outwards. This further prevents disengagement of the counter element 116 from the rib 156 upon release of the button 318 during dose delivery.

With alternative embodiments, the rib 156 and the counter element 116 may only comprise a single one of the chamfers 117, 364 or none of the chamfers 116, 364. Furthermore, the circumferential rib 156 may also comprise none of the openings 158 located adjacent to the dose stops 118, 119. With these embodiments, a deflection of the counter element 116 upon transfer of the first mechanism 354 from the dose setting state into the dose delivery state may only be caused by one or both of the chamfers 117, 364.

At the zero dose position, the rib 156 comprises an opening 158 that does not feature a recessed section 362. This allows the counter element 116 to pass the rib 156 in the axial direction and thus allows the first mechanism 354 to transfer from the dose delivery state into the dose setting state. With embodiments, that are configured as single-use mechanisms and that only allow to set and deliver a dose once, the rib 156 may also comprise no opening 158 at the zero dose position. This prevents a return to the dose setting state after a user has set and delivered the first dose with the first mechanism 354. Alternative embodiments of a single-use device may also comprise coupling means that acts between the button 318 and the dosing member 323 and that axially lock the button 318 to the dosing member 323 after proximal movement of the button 318 to transfer the first mechanism 354 from the dose setting state into the dose delivery state. Such coupling means are, inter alia, described in application US 17/981231.

The axial movement of the counter element 116 with respect to the dose stops 118, 119 upon transfer of the first mechanism 354 from the dose setting state into the dose delivery state also disengages the counter element 116 from the dose stops 118, 119. As long as the user pushes the button 318 in the proximal direction 1, the dosing member 323 is allowed to freely rotate back to the zero dose position and the set dose is automatically delivered by the force stored in the spring 40.

The first mechanism 354 also comprises a break 390 that is configured to stop movement of the nut 38 and the piston rod 44 upon release of the apartment 318 during dose delivery. The brake 390 comprises a first break part that is formed by the rib 156 and a second break part that is formed by the counter element 116.

During dose delivery, the counter element 116 rotates around the rib 156 back to the zero dose position. If the button 318 is released during dose delivery, the biasing element 250 biases the rib 156 provided at the dose selector 360 towards the counter element 116 of the carrier 24. This pushes an engaging section 392 of the counter element 116 against a break surface 366 of the rib 156. The engaging section 392 is thereby formed by the proximal end face of the counter element 116 and the break surface 366 is formed by the distal end surface of the rib 156.

As can be seen from Fig. 53, the rib 156 comprises a plurality of grooves 367 that are formed on the break surface 366. Upon release of the button 318 during dose delivery, the counter element 116 engages with one of the grooves 367. This locks the dosing member 323 to the dose selector 360 by a form fit.

With alternative embodiments, the rib 156 may not comprise the grooves 367. The dosing member 323 may then lock to the dose selector 360 by a friction-lock.

Other embodiments of the first mechanism 354 may not comprise the brakes 390. With these embodiments, automatic injection will continue after release of the button 318 during dose delivery.

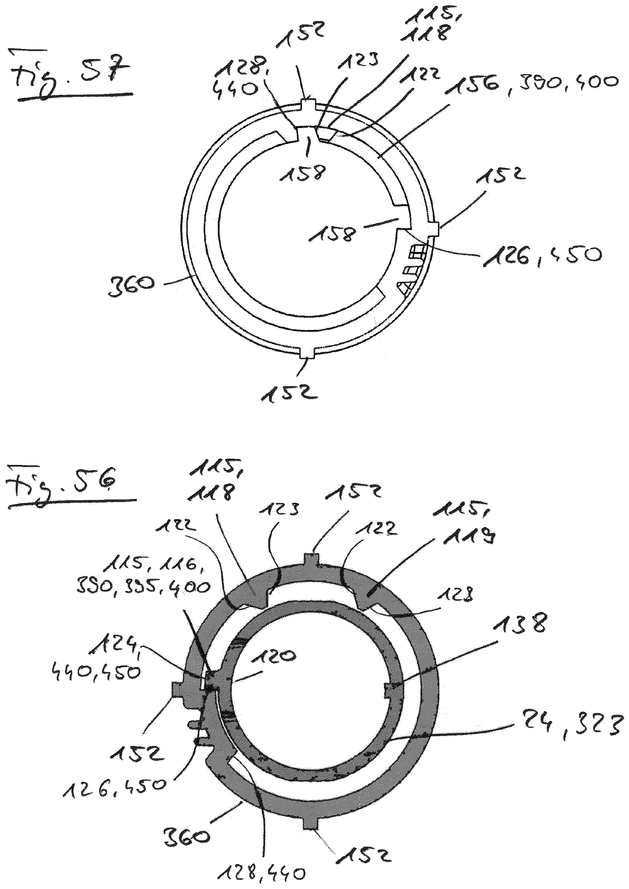

**Fig. 56** shows a radial cut perpendicular to the longitudinal axis through the dose selector 360 with the dose stops 118, 119 and the carrier 24 of the dosing member 323. The first mechanism 354 thereby is in the dose setting state with the dosing member 323 in the zero dose position. The blocking part 126 then abuts the zero dose stop 126.

In order to reduce the force needed to rotate the dose setting element 22 and the carrier 24 relative to the dose selector 360 to enlarge or decrease the set dose, the dose stops 118, 119 have chamfered side surfaces 122 and 123. According to the embodiment shown in Fig. 56, the dose stops 118, 119 have an asymmetrical cross section in the radial plane perpendicular to the longitudinal axis of the first mechanism 354. Thereby, first side surfaces 122 that get in contact with the counter element 116 when the set dose is increased have a smaller pitch than second side surfaces 123 that get in contact with the counter element 116 when the dose is decreased. The second side surfaces 123 then also generate a higher counter force than the first side surfaces 122. This helps to prevent the dosing member 323 to rotate back to the zero dose position due to the torque of the spring 40. With other embodiments, the first side surface 122 and the second side surface 123 may be symmetric and may have pitches that are equal to each other.

The embodiment of the dose selector 360 shown in Figures 52 to 66 is a first embodiment of the dose selector 360.

**Fig. 57** shows a bottom view of a second embodiment of the dose selector 360 from a proximal end. The dose selector 360 only comprises a single dose stop 118. Furthermore, the rib 156 does not feature a recessed section 362 at the opening 158 formed at the dose stop 118. In addition, the maximum dose stop 128 is located directly next to the dose stop 118. Finally, the first side surface 122 and the second side surface 123 are symmetric and have pitches that are equal to each other.

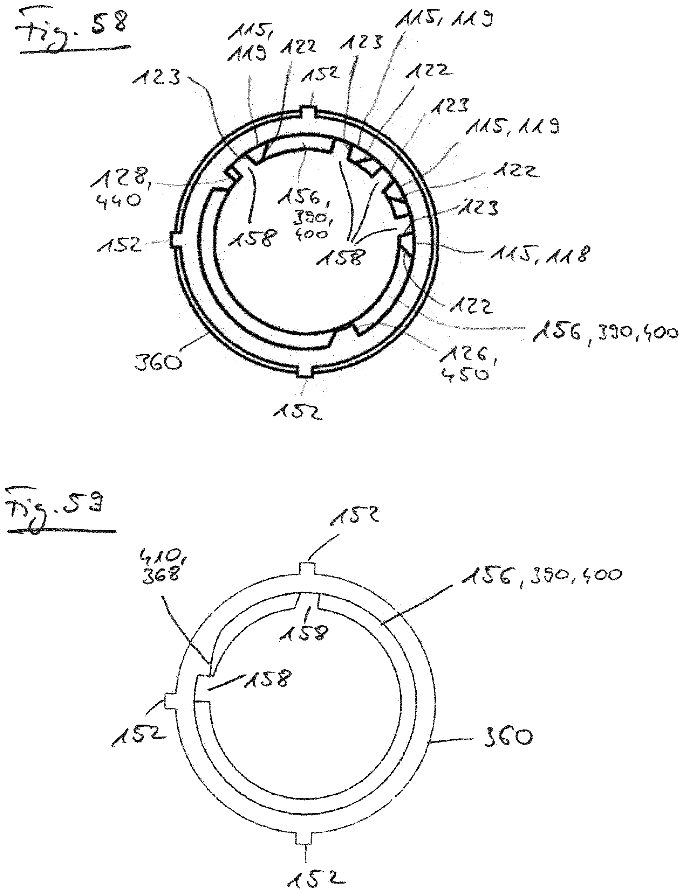

**Fig. 58** shows a bottom view of a third embodiment of the dose selector 360 from a proximal end. The dose selector 360 comprises four dose stops 118, 119. The dose stops 118 are configured as asymmetric dose stops with first side surfaces 122 that have a smaller angle than second side surfaces 123. Furthermore, the dose selector 360 does not feature an opening 158 at the zero dose position next to the zero dose stop 126. This prevents a transfer of the first mechanism 354 from the dose delivery state back to the dose setting state at the end of dose delivery. The first mechanism 354 then is configured as a single-use mechanism.

**Fig. 59** shows a top view of the first embodiment of the dose selector 360 from a distal end. As can also be seen from Fig. 52, the first mechanism comprises a feedback mechanism 410 that indicates an end of dose delivery. The feedback mechanism 410 thereby is configured to give an audible and/or tactile feedback to a user of the device.

The feedback mechanism 410 comprises the counter element 116 and a feedback element 368. At the end of injection, the counter element 116 passes over the feedback element 368 and becomes deflected radially inwards. Upon disengagement from the feedback element 368, the counter element 116 relaxes radially outwards and produces an audible and/or sensible click.

The feedback element 368 is located next to the zero dose stop 126. It is configured as a ramp having a shallow side surface facing away from the zero dose stop and a steep side surface facing towards the zero dose stop. Upon returning to the zero dose position, the counter element 116 gets gradually deflected when it travels over the shallow side surface. Subsequently, the counter element 116 rapidly passes over the steep side surface and thereby produces the audible and/or tactile feedback.

With first mechanism 354, the dose setting element 22 and the button 318 of the actuation unit 316 are rigidly connected to each other and axially and rotationally fixed with respect to each other. With other embodiments, the dose setting element 22 and the button 318 may also be movable with respect to each other.

With alternative embodiments, the rib 156 may also be engaged by the blocking part 124 of the carrier 24. The rib 156 then may be distanced from the dose stops 118, 119 towards the proximal direction. With these embodiments, the openings 158 do not comprise the recessed sections 362.

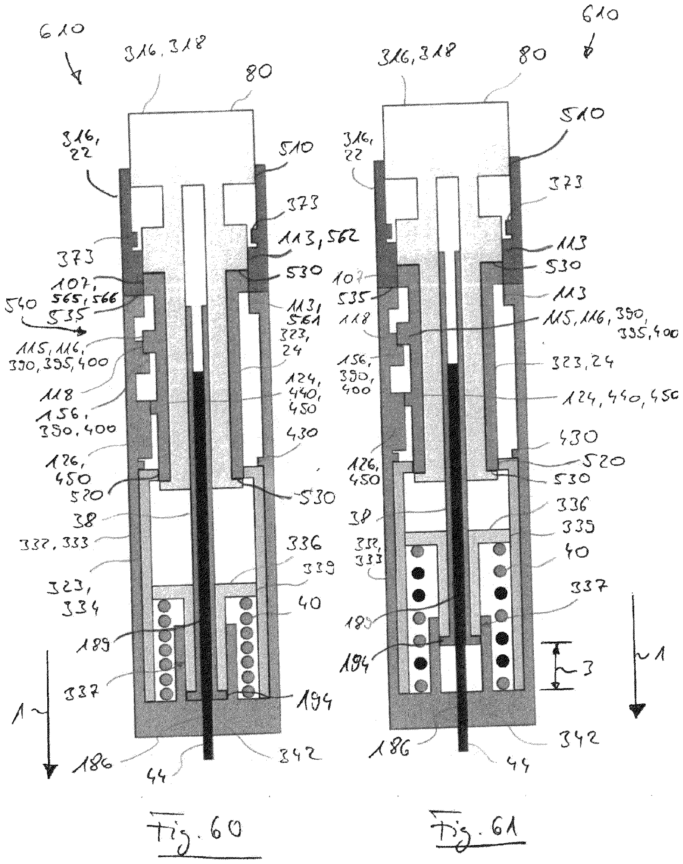

**Fig. 60** shows a section view of a second mechanism 610 for an automatic medicament delivery device according to the present disclosure in a dose setting state prior to setting a dose. As far as no differences are disclosed, the second mechanism 610 is configured as it is disclosed for the first mechanism 354 and vice versa.

With the second mechanism 610, the button 318 is axially movable with respect to the dose setting element 22. Furthermore, the button 318 is rotationally fixed to the dose setting element 22. An axial lock 510 acts between the button 318 and the dose setting element 22 and rotationally locks the button 318 to the dose setting element 22 while allowing relative axial movement. In an exemplary configuration, the axial lock 510 may be configured as a splined connection having interacting longitudinal ridges and grooves. The axial lock 510 may be formed at the dose setting element 22 and the button 318. Alternatively, the axial lock 510 may be formed at one or more intermediate members located in between the button 318 and the dose setting element 22.

The dose setting element 22 is axially fixed to the housing 332 of the second mechanism 610. Furthermore, the dose setting element 22 is rotationally movable with respect to the housing 332. The dose setting element 22 is connected to the housing 332 by an axial lock 535 that prevents relative rotational movement and allows relative axial movement between the dose setting element 22 and the housing 332. The axial lock 535 may be located at a distal end of the housing 332. Additionally or alternatively, the axial lock 535 may be located at a proximal end of the dose setting element 22. The axial lock 535 may exemplarily be configured as a snap-fit connection. For example, the axial lock 535 may be configured as it is described for the axial lock having the axial fixation means 142, 146 between the dose setting element 22 and the dose selector 360 of the first mechanism 354.

The carrier 24 of the dosing member 323 is rotationally fixed and axially movable with respect to the dosing element 334 of the dosing member 323. A rotation lock 520 acts between the carrier 24 and the dosing element 334 and rotationally fixes the carrier 24 to the dosing element 334. The rotation lock 520 may be configured as a splined connection having longitudinal ridges and corresponding longitudinal grooves engaging with the longitudinal ridges. With other embodiments, the rotation lock 520 may also be configured as matching out-of-round cross-sections of two lock elements of the rotation lock 520 that engage with each other.

The rotation lock 520 may be formed by the carrier 24 and the dosing element 334. With alternative embodiments, the rotation lock 520 may formed by one or more intermediate members coupled between the carrier 24 and the dosing element 334.

Like with the first mechanism 354, the dosing element 334 of the second mechanism 610 is rotationally movable and axially fixed with respect to the housing 332. The blocker 430 thereby axially restraints the dosing element 334 in the distal direction, as it is disclosed in connection with the first mechanism 354.

The carrier 24 of the dosing member 323 is axially fixed and rotationally movable with respect to the button 318. An axial lock 530 acts between the carrier 24 and the button 318 and prevents relative axial movement while allowing relative rotational movement. The axial lock 530 may be formed by the carrier 24 and the button 318. With alternative embodiments, the axial lock 530 may be formed by one or more intermediate members coupled between the carrier 24 and the button 318. For example, one of these intermediate members may be axially and/or rotationally fixed to the button 318.

The axial lock 530 is exemplarily formed by two radially extending portions of the button 318 that are axially spaced from each other to form an axially confined section and that receive the carrier 24 in the axially confined section.

With the embodiment shown in Fig. 60, the button 318 comprises a generally cylindrical proximal part that is configured as a hollow cylinder. The carrier 24 is thereby disposed around an outer surface, namely around an outer circumferential surface, of the cylindrical proximal part. The cylindrical proximal part may be integrally formed with a distal part of the button 318 that comprises the end surface 80. With alternative embodiments, the cylindrical proximal part may be formed as a member separate from the distal part and the cylindrical proximal part and the distal part may be configured to be joined axially and rotationally fixed to each other during assembly. For example, the distal part and the proximal part may be configured to be joined to each other after the carrier 24 has been disposed around one of the distal part and the proximal part.

The button 318 receives the nut 38 axially movable and rotationally fixed within the proximal part, as it is described for the cylindrical portion 18a of the button 318 of the first mechanism 354.

The second mechanism 610 comprises a dose selector 540 that is axially and rotationally fixed with respect to the housing 332. As far as no further differences are disclosed, the dose selector 540 is configured as it is disclosed for the dose selector 360 of the first mechanism 354.

The dose selector 540 is exemplarily integrally formed with the housing 332. With other embodiments, the dose selector 540 may also be configured as a separate component that is rotationally and axially fixed to the housing 332 during assembly of the second mechanism 610.

As can be seen from Fig. 60, the rib 156 that forms parts of the brake 390 and the blocking mechanism 400 is located in the proximal direction from the counter element 116. With the second mechanism 610, the interaction between the counter element 116 and the components 118, 119, 156, 362, 367, 368 located at the inside surface 361 of the dose selector 540 and the relative movements of the counter element 116 with respect to said components occur in the same manner as described in connection with the first mechanism 354, but with the axial directions of movement reversed.

The clutch 113 that rotationally fixes the nut 38 to the piston rod 44 during dose delivery acts between the button 318 and the housing 332. A first engaging part 561 of the clutch 113 is axially and rotationally fixed to the housing 332. It may be integrally formed with the housing 332 or attached to the housing 332 as a separate part. A second engaging part 562 of the clutch 113 is axially and rotationally fixed to the button 318. It may be integrally formed with the button 318 or attached to the button 318 as a separate part. The first engaging part 561 and the second engaging part 562 are disengaged from each other in the dose setting state depicted in Fig. 60.

The further clutch 107 that rotationally fixes the dose setting element 22 to the dosing member 323 during dose setting acts between the dose setting element 22 and the carrier 24, as it is disclosed for the clutch 107 of the first mechanism 354. The dose setting element 22 thereby exemplarily comprises a further second engaging part 566 of the further clutch 107 and the carrier 24 exemplarily comprises a further first engaging part 565 of the further clutch 107. As it is disclosed for the further clutch 107 of the first mechanism 354, the further first and second engaging parts of the further clutch 107 of the second mechanism 610 may be configured as radially extending longitudinal teeth. The further first engaging part 565 may be integrally formed with the carrier 24 or it may be attached to the carrier 24 as an axially and rotationally fixed separate part. Likewise, the further second engaging part 566 may be integrally formed with the dose setting element 22 or it may be attached to the dose setting element 22 is an axially and rotationally fixed separate part.

In the dose setting state depicted in Fig. 60, the further first engaging part 565 engages with the further second engaging part 566 and the further clutch 107 is in its closed state rotationally locking the dose setting element 22 to the dosing member 323 and the driver 336.

During dose setting, rotation of the dose setting element 22 is transferred to the nut 38 via the rotational lock 510 and the button 318. Furthermore, rotation of the dose setting element 22 is transferred to the driver 336 and the spring 40 via the closed further clutch 107, the carrier 24, the rotational lock 520 and the dosing element 334.

With the second mechanism 610, the stop 373 that limits proximal movement of the button 318 during dose delivery acts between the dose setting element 22 and the bottom 318. It thereby directly acts between the dose setting element 22 and the button 318 and the dose setting element 22 forms a first stop part and the button 318 forms a second stop part of the stop 373. The stop 373 is exemplarily located at the dose setting element 22 and is configured to engage with the button upon proximal movement of the button 318.

With other embodiments, the stop 373 may also act directly between the button 318 and the housing 332 or it may act between the button 318 and the dosing element 334.

**Fig. 61** shows the section view of the second mechanism 610 in the dose setting state after setting a dose by rotating the dose setting element 22 with respect to the housing 332. Both the clutch 38 and the driver 336 have moved in the distal direction, whereby rotation of the driver 336 has strained the spring 40 storing energy in the spring 40. The clutch 38 has moved by the dose distance 3.

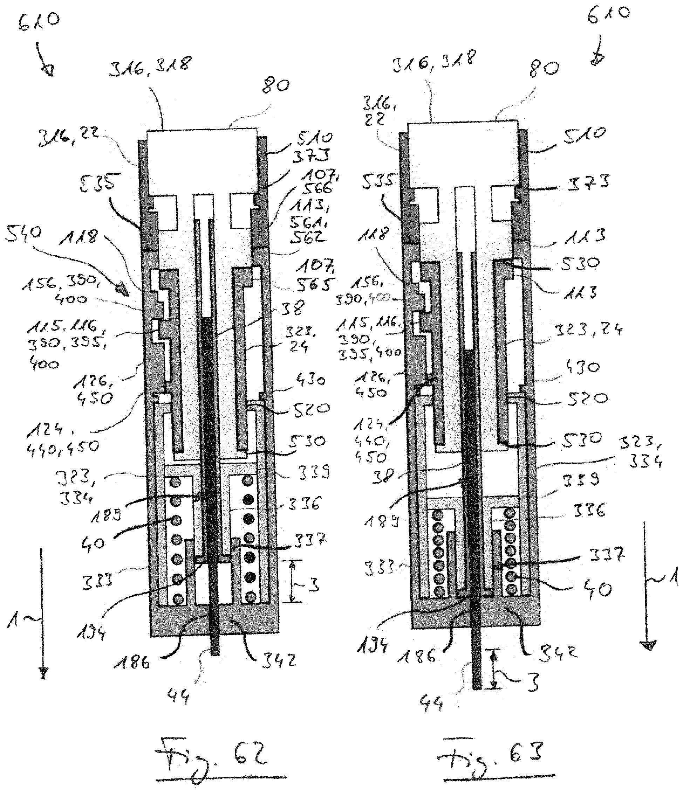

**Fig. 62** shows the section view of the second mechanism 610 in the dose delivery state prior to delivering the set dose. The button 318 has being moved in the proximal direction 1 until the stop 373 limits further proximal movement. The button 318 then engages with the stop 373.

Proximal movement of the button 318 has also moved the carrier 24 in the proximal direction 1. Thereby, the further first engaging part 565 of the further clutch 107 has disengaged from the further second engaging part 566 due to relative axial movement with respect to each other. The dosing member 323 then is free to rotate with respect to the housing 332.

Axial movement of the button 318 has additionally closed the clutch 113 so that the first engaging part 561 engages with the second engaging part 562. This rotationally locks the button 318 to the housing 332 and thus also the nut 38 to the piston rod 44.

The counter element 116 has been moved from the distal side of the rib 156 to the proximal side of the rib 156. At the proximal side, the rib 156 comprises the break surface 366 that interacts with the counter element 116 upon release of the button 318 during dose delivery. The dose stops 118, 119 are located at the distal side of the rib 156, while the feedback element 368 is located at the proximal side of the rib 156.

Generally speaking, the break surface 366 and the dose stops 118, 119 are located at opposing sides from the rib 156 in the longitudinal direction.

**Fig. 63** shows the section view of the second mechanism 610 in the dose delivery state after delivering the set dose. Driven by the spring 40, the driver 336 and the nut 38 have moved by the dose distance 3 in the proximal direction 1. Due to the axially fixed connection between the nut 38 and the piston rod 44, the piston rod 44 is also moved in the proximal direction 1 by the dose distance 3. Rotation of the driver 336 during dose delivery has rotated the carrier 24 back to its zero dose position, whereby a torque is transferred to the carrier 24 via the dosing element 334 and the rotation lock 520.

Like the first mechanism 354, the second mechanism 610 may comprise a biasing member that biases the button 318 in the distal direction with respect to the housing 332.

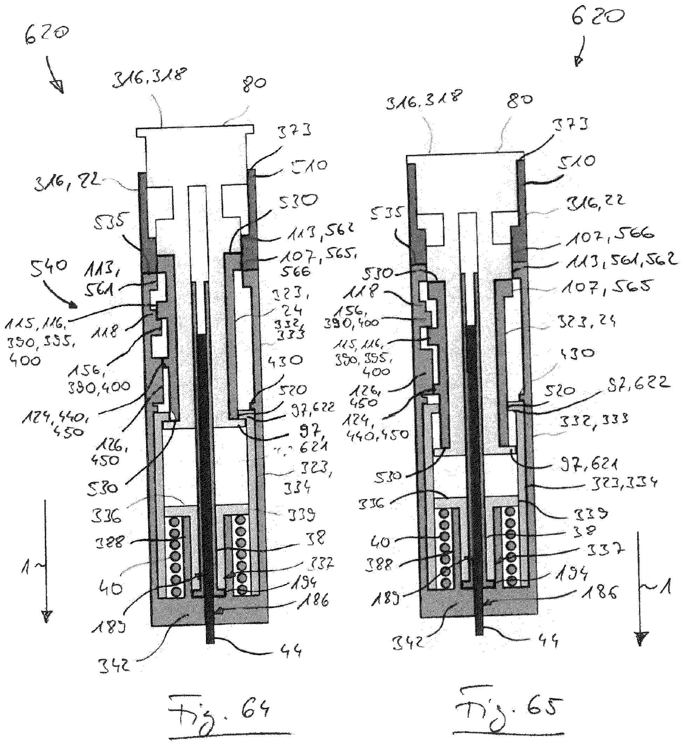

**Fig. 64** depicts a third mechanism 620 for an automatic medicament delivery device according to the present disclosure in a dose setting state prior to setting a dose and **Fig. 65** depicts the third mechanism 620 in a dose delivery state after delivering a dose. As far as no differences are disclosed, the third mechanism 620 is configured as it is disclosed for the second mechanism 610 and vice versa.

While the stop 373 of the second mechanism 610 is located at an inside surface of the dose setting element 22, the stop 373 of the third mechanism 620 is located at a distal end of the dose setting element 22. It is exemplarily formed by the distal end surface of the dose setting element 22. In the dose delivery state, the button 318 is configured to abut the stop 373 with a radial protrusion that is directed radially outwards and located at a distal end of the button 318.

The third mechanism 620 also comprises a retainer 96 that prevents detachment of the button 318 from the housing 332. The retainer 96 acts between the dosing member 323 and the button 318, namely between the dosing element 334 and the button 318. A first retainer element 621 is fixed to the button 318 and a second retainer element 622 of the retainer 97 is fixed to the dosing member 323, namely to the dosing element 334. The first retainer element 621 is exemplarily located at the proximal end of the button 318 and the second retainer element 622 is exemplarily located at the distal end of the dosing element 334.

In the dose setting state shown in Fig. 64, the first retainer element 621 and the second retainer element 622 axially engage with each other, while the first retainer element 621 has moved axially away from the second retainer element 622 in the dose delivery state shown in Fig. 65.

With alternative embodiments, the stop 373 of the third mechanism 620 may also be configured like the stop 373 of the second mechanism 610.

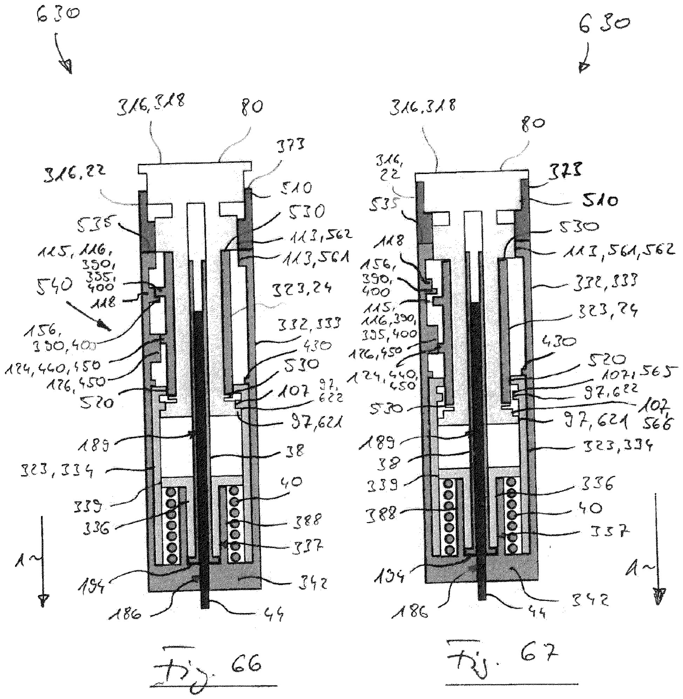

**Fig. 66** depicts a section view of a fourth mechanism 630 for an automatic medicament delivery device according to the present disclosure in a dose setting state prior to setting a dose and **Fig. 67** depicts the section view of the fourth mechanism 630 in a dose delivery state after delivering a set dose. As far as no differences are disclosed, the fourth mechanism 630 is configured as it is disclosed for the third mechanism 620 and vice versa.

With the fourth mechanism 630, the further clutch 107 that rotationally locks the dose setting element 22 to the dosing member 323 is coupled to the dose setting element 22 via the button 318. The further clutch 107 is exemplarily formed between the dosing member 323 and the button 318. The further first engaging part 565 is fixed to the dosing member 323 and the further second engaging part 566 is fixed to the button 318. With the embodiment shown in Figures 66 and 67, the further first engaging part 565 is fixed to the dosing element 334. The further second engaging part 566 is exemplarily located at a proximal end of the button 318. Furthermore, the further clutch 107 is located within the dosing member 323, namely within the dosing element 334.

With the fourth mechanism 630, a force exerted by the user upon rotation of the dose setting element 22 during dose setting is transferred via the rotation lock 510, the button 318 and the further clutch 107 to the dosing member 323 and the driver 336. An axial distance that is travelled by the button 318 upon transfer of the fourth mechanism 630 from the dose setting state to the dose delivery state is reduced compared to the second and third mechanisms 610, 620.

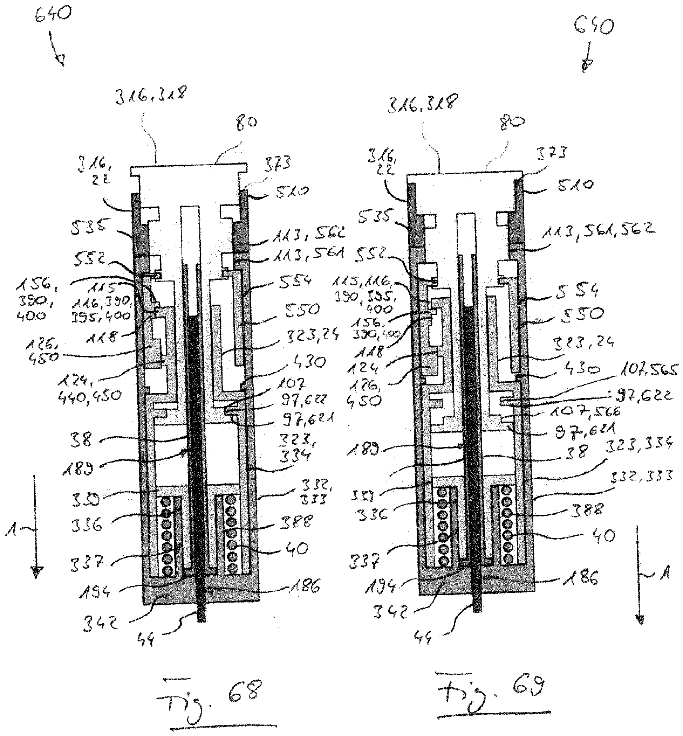

**Fig. 68** depicts a section view of a fifth mechanism 640 for an automatic medicament delivery device according to the present disclosure in a dose setting state prior to setting a dose and **Fig. 69** depicts the section view of the fifth mechanism 640 in a dose delivery state after delivering a set dose. As far as no differences are disclosed, the fifth mechanism 640 is configured as it is disclosed for the fourth mechanism 630 and vice versa.

The fifth mechanism 640 comprises a dose selector 550 that is axially movable and rotationally fixed with respect to the housing 332. The dose selector 550 thereby is coupled to the housing 332 by a rotation lock 554 that prevents relative rotation and allows axial movement between the dose selector 550 in the housing 332. The rotation lock 554 may be configured as a splined connection, for example analogously to the rotation lock 152 between the dose selector 360 and the housing 332 of the first mechanism 354. As far as no further differences are disclosed, the dose selector 550 is configured as it is disclosed for the dose selector 360 of the first mechanism 354 and vice versa.

The dose selector 550 is axially fixed and rotationally movable with respect to the button 318 and coupled to the button 318 via an axial lock 552. The axial lock 552 is formed between the dose selector 550 and the button 318. With alternative embodiments, the axial lock 552 may also be formed at one or more intermediate members.

In general, the dose selector 550 may be configured to follow the axial movement of the button 318. With some embodiments, the dose selector 550 then may only abut against the button 318, for example in a distal direction. The dose selector 550 additionally may be biased in the distal direction, for example by a biasing element that acts between the dose selector 550 in the housing 332 or between the dosing member 332 and the dose selector 550. The biasing element may, for example, be located within the dose selector 550. It may, for example, be configured as a compression spring. When moving the button 318 in the proximal direction 1, it will push against the dose selector 550 and move it against the biasing force directed in the distal direction.

The carrier 24 of the dosing member 323 is axially and rotationally fixed to the dosing element 334. The carrier 24 and the dosing element 334 of the fifth mechanism 640 may be configured as it is disclosed for the carrier 24 and the dosing element 334 of the first mechanism 354. Furthermore, the layout of the elements on the inside surface of the dose selector 550 of the fifth mechanism 640 may correspond to the layout of the elements on the inside surface of the dose selector 360 of the first mechanism 354.

With alternative embodiments of the fifth mechanism 640, the stop 373 may also act between the dose selector 550 and the housing 332, as it is described for the stop 373 of the first mechanism 354. Furthermore, the further clutch 107 of the fifth mechanism 640 may also be configured like the further clutch 107 of the second and third mechanisms 610, 620.

### Reference signs:

- 1: proximal direction
- 3: dose distance
- 12: distal end
- 14: proximal end
- 18a: cylindrical portion
- 20: intermediate member
- 21: gripping surface
- 22: dose setting element
- 24: carrier
- 26: connector
- 38: nut
- 39: opening
- 40: spring
- 44: piston rod
- 46: bearing
- 76: rib
- 80: end surface
- 81: axial lock
- 82: axial fixation means
- 84: rib
- 86: axial fixation means
- 88: undercut
- 90: rotation fixation means
- 92: rotation fixation means
- 93: toothed part
- 94: rotation fixation means
- 96: rotation fixation means
- 97: retainer
- 98a: first retainer element
- 98b: further first retainer element
- 102: second retainer element
- 103: rotation lock
- 104: rib
- 106: groove
- 107: further clutch
- 108: second engaging part, further second engaging part
- 110: further first engaging part
- 112: coupling section
- 113: clutch
- 114: first engaging part
- 115: dose definition mechanism
- 116: counter element
- 117: chamfer
- 118: dose stop
- 119: further dose stop
- 120: elastically deformable section
- 121: cut-out
- 122: first side surface
- 123: second side surface
- 124: blocking part
- 126: zero dose stop
- 128: maximum dose stop
- 130: opening
- 132: slot
- 134: rib
- 136: protrusion
- 136a: chamfered surface
- 138: rib
- 140: groove
- 142: axial fixation means
- 144: intake
- 146: axial fixation means
- 148: rotation fixation means
- 150: rotation lock
- 152: rotation lock
- 156: rib
- 158: opening
- 166: window
- 168: label
- 172: inner thread
- 186: opening
- 189: threaded connection
- 190: outer thread
- 191: flattened section
- 192: inner thread
- 193: axial stop
- 194: axial stop
- 196: front surface
- 198: proximal connector
- 199: stop
- 200: bearing connector
- 250: biasing member
- 300: medicament delivery device
- 301: cap
- 302: longitudinal axis
- 305: container holder
- 306: needle connector
- 307: connector
- 316: actuation unit
- 318: button
- 323: dosing member
- 332: housing
- 333: outer housing
- 334: dosing element
- 336: driver
- 337: drive thread
- 338: opening
- 339: connector
- 340: spring connector
- 342: piston rod guide
- 348: medicament container
- 349: needle end
- 350: piston
- 354: first mechanism
- 360: dose selector
- 361: inner surface
- 362: recessed section
- 364: chamfer
- 366: break surface
- 367: groove
- 368: feedback element
- 371: inside surface
- 373: stop
- 374: stop
- 375: connector
- 382: housing connector
- 384: container connector
- 386: spring connector
- 387: outer body
- 388: inner body
- 390: break
- 391: first part
- 392: engaging section
- 395: second part
- 400: blocking mechanism
- 401: first element
- 403: second element
- 410: feedback mechanism
- 421: outer surface
- 422: inner surface
- 424: connector
- 430: blocker
- 440: maximum dose mechanism
- 450: zero dose mechanism
- 461: first end
- 462: second end
- 510: rotation lock
- 520: rotation lock
- 530: axial lock
- 535: axial lock
- 540: dose selector
- 550: dose selector
- 552: axial lock
- 554: rotation lock
- 561: first engaging part
- 562: second engaging part
- 565: further first engaging part
- 566: further second engaging part
- 610: second mechanism
- 620: third mechanism
- 621: first retainer element
- 622: second retainer element
- 630: fourth mechanism
- 640: fifths mechanism

The present disclosure is also directed the following enumerated embodiments:
1. A mechanism (354, 610, 620, 630, 640) for an automatic medicament delivery device (300) comprising:
   a housing (332);
   a dose setting element (22);
   a button (318);
   a piston rod (44) that is rotationally fixed and axially movable with respect to the housing (332);
   a nut (38); and
   a spring (40),
   wherein the dose setting element (22) is configured to be gripped by a user of the device to set a dose to be delivered by rotation of the dose setting element (22) with respect to the housing (332) when the mechanism (354, 610, 620, 630, 640) is in a dose setting state,
   wherein the rotation of the dose setting element (22) stores energy in the spring (40),
   wherein the rotation of the dose setting element (22) causes the nut (38) to move by a dose distance (3) in a proximal direction (1) with respect to the piston rod (44), the dose distance (3) being proportional to the dose,
   wherein the mechanism (354, 610, 620, 630, 640) is configured to switch from the dose setting state into a dose delivery state upon moving the button (318) [in an axial direction] with respect to the housing (332),
   wherein a transfer into the dose delivery state couples the spring (40) to the nut (38) to cause automatic movement of the nut (38) in a proximal direction (1) by releasing the energy stored during dose setting,
   wherein the piston rod (44) is configured to move together with the nut (38) by the dose distance (3) in the proximal direction (1) to deliver the dose when the mechanism (354, 610, 620, 630, 640) is in the dose delivery state.
2. The mechanism (354, 610, 620, 630, 640) of embodiment 1,
   wherein the dose setting element (22) and the button (318) are movable with respect to each other.
3. The mechanism (354, 610, 620, 630, 640) of at least one of the preceding embodiments,
   wherein the button (318) is axially movable with respect to the dose setting element (22), wherein the button (318) is rotationally fixed with respect to the dose setting element (22).
4. The mechanism (354, 610, 620, 630, 640) of embodiment 1,
   wherein the dose setting element (22) and the button (318) are fixed with respect to each other during dose setting and dose delivery.
5. The mechanism (354, 610, 620, 630, 640) of at least one of the preceding embodiments,
   wherein the dose setting element (22) is rotationally fixed with respect to the housing (332) when the dose is being delivered.
6. The mechanism (354, 610, 620, 630, 640) of at least one of the preceding embodiments,
   wherein the dose setting element (22) is configured to remain axially stationary with respect to the housing (332) when the piston rod (44) moves in the proximal direction (1) to deliver the dose.
7. The mechanism (354, 610, 620, 630, 640) of at least embodiment 6,
   wherein the dose setting element (22) is axially fixed with respect to the housing (332) during dose setting and during dose delivery.
8. The mechanism (354, 610, 620, 630, 640) of at least one of the preceding embodiments,
   wherein the button (318) is configured to remain axially stationary with respect to the housing (332) when the piston rod (44) moves in the proximal direction (1) to deliver the dose.
9. The mechanism (354, 610, 620, 630, 640) of at least one of the preceding embodiments,
   further comprising a dosing member (323),
   wherein the dosing member (323) is configured to rotate with respect to the housing (332) during dose setting and to rotate with respect to the housing (332) during dose delivery,
   wherein the dosing member (323) is configured to remain axially stationary with respect to the housing (332) during dose delivery.
10. The mechanism (354, 610, 620, 630, 640) of embodiment 9,
   wherein the dosing member (323) comprises a first part (24) and a second part (334),
   wherein the first part (24) is axially movable and rotationally fixed with respect to the second part (334).
11. The mechanism (354, 610, 620, 630, 640) of at least one of embodiments 9 and 10,
   wherein the first part (24) is axially fixed with respect to the button (318) and/or wherein the second part (334) is axially fixed with respect to the housing (332).
12. The mechanism (354, 610, 620, 630, 640) of at least one of embodiments 9 to 11,
   wherein a rotational position of the dosing member (323) defines the dose, wherein the first part (24) comprises one of a dose stop (118, 119) and a counter element (116) of a dose definition mechanism (115).
13. The mechanism (354, 610, 620, 630, 640) of at least one of embodiments 9 to 12,
   wherein the dosing member (323) is configured to remain axially stationary with respect to the housing (332) while storing the energy in the spring (40) during dose setting.
14. The mechanism (354, 610, 620, 630, 640) of at least one of embodiments 9 to 13,
   wherein the dosing member (323) is coupled between the spring (40) and the dose setting element (22) during dose setting to transfer the energy from the dose setting element (22) to the spring (40).
15. The mechanism (354, 610, 620, 630, 640) of at least one of embodiments 9 to 14,
   wherein the dosing member (323) comprises a label (168) to visually indicate setting of the dose.
16. The mechanism (354, 610, 620, 630, 640) of at least one of embodiments 9 to 15,
   wherein the dose setting element (22) is configured to remain axially stationary with respect to the dosing member (323) during dose delivery.
17. The mechanism (354, 610, 620, 630, 640) of at least one of embodiments 9 to 16,
   wherein the dose setting element (22) is configured to remain axially stationary with respect to the dosing member (323) during dose setting.
18. The mechanism (354, 610, 620, 630, 640) of at least one of the preceding embodiments,
   further comprising a blocker (430),
   wherein the blocker (430) acts between the button (318) and the housing (332),
   wherein the blocker (430) blocks the button (318) from moving axially with respect to the housing (332) in the distal direction (1).
19. The mechanism (354, 610, 620, 630, 640) of at least embodiment 18 and at least one of embodiments 9 to 17,
   wherein the blocker (430) acts between the dosing member (323) and the housing (332),
   wherein, for example, the blocker (430) is provided at one of the housing (332) and the dosing member (323) and engages with the other one of the housing (332) and the dosing member (323).
20. The mechanism (354, 610, 620, 630, 640) of at least embodiment 18 and 19,
   wherein the blocker (430) is configured as an axial stop.
21. The mechanism (354, 610, 620, 630, 640) of at least embodiment 18 to 20,
   wherein the blocker (430) is configured as a one-way blocker,
   wherein the blocker (430) allows relative axial movement between the housing (332) and a counter member (323, 334) in a first direction and blocks relative axial movement between the housing (332) and the counter member (323, 334) in a second direction opposite the first direction.
22. The mechanism (354, 610, 620, 630, 640) of embodiment 21,
   wherein the blocker (430) is configured as a flexible element that is configured to snap into a blocking position upon assembly of the counter member (323, 334) to the housing (332).
23. The mechanism (354, 610, 620, 630, 640) of at least one of embodiments 21 and 22 and at least embodiment 19,
   wherein the counter member (323, 334) is part of the dosing member (323).
24. The mechanism (354, 610, 620, 630, 640) of at least one of the preceding embodiments,
   wherein the mechanism (354, 610, 620, 630, 640) comprises a stop (373, 374) to limit proximal movement of the button (318) during dose delivery.
25. The mechanism (354, 610, 620, 630, 640) of at least embodiment 24 and at least one of embodiments 9 to 17,
   wherein the dosing member (323) is coupled in between the stop (374) and the housing (332).
26. The mechanism (354, 610, 620, 630, 640) of embodiment 24,
   wherein the stop (373) is provided at the housing (332), such as an inside surface (371) of the housing (332).
27. The mechanism (354, 610, 620, 630, 640) of at least one of embodiments 24 to 26,
   wherein the stop (373) acts between the housing (332) and a housing connector (360) that is axially fixed to the button (318).
28. The mechanism (354, 610, 620, 630, 640) of embodiment 24,
   wherein the stop (373) is provided at the dose setting element (22).
29. The mechanism (354, 610, 620, 630, 640) of at least one of embodiments 24 to 28,
   wherein the stop (373, 374) is configured as an axial stop.
30. The mechanism (354, 610, 620, 630, 640) of at least one of the preceding embodiments,
   further comprising a retainer (97) to prevent detachment of the button (318) and/or the dose setting element (22) from the housing (332),
   wherein the retainer (97) comprises a first retainer element (98a, 621) and a second retainer element (102, 622),
   wherein the first retainer element (98a, 621) and the second retainer element (102, 622) are configured to engage with each other to prevent the detachment of the button (318) and/or the dose setting element (22) from the housing (332).
31. The mechanism (354, 610, 620, 630, 640) of at least embodiment 30 and at least one of embodiments 9 to 17,
   wherein the retainer (97) acts between, on the one hand, the dosing member (323) and, on the other hand, the button (318) and/or the dose setting element (22).
32. The mechanism (354, 610, 620, 630, 640) of at least embodiment 30,
   wherein the dose setting element (22) is axially fixed with respect to the housing (332),
   wherein the retainer (97) is configured to prevent detachment of the button (318) from the housing (332),
   wherein the retainer (97) acts between the dose setting element (22) and the button (318).
33. The mechanism (354, 610, 620, 630, 640) of at least one of embodiments 30 to 32 ,
   wherein the first retainer element (98a, 621) and the second retainer element (102, 622) are configured to move away from each other to allow axial movement of the button (318) and/or the dose setting element (22) with respect to the housing (332) in the proximal direction (1).
34. The mechanism (354, 610, 620, 630, 640) of at least one of embodiments 30 to 33,
   wherein the retainer (97) is configured as an axial stop.
35. The mechanism (354, 610, 620, 630, 640) of at least one of the preceding embodiments,
   further comprising a driver (336),
   wherein the driver (336) is coupled between the spring (40) and the nut (38) to transfer the energy stored in the spring (40) to the nut (38) when the mechanism (354, 610, 620, 630, 640) is in the dose delivery state,
   wherein the driver (336) is configured to move in the distal direction when the mechanism (354, 610, 620, 630, 640) is in the dose setting state and to move in the proximal direction (1) when the mechanism (354, 610, 620, 630, 640) is in the dose delivery state.
36. The mechanism (354, 610, 620, 630, 640) of embodiment 35,
   wherein the spring (40) is coupled between the driver (336) and the housing (332).
37. The mechanism (354, 610, 620, 630, 640) of at least one of embodiments 35 and 36,
   wherein the driver (336) is rotationally driven by the spring (40) during dose delivery.
38. The mechanism (354, 610, 620, 630, 640) of at least one of embodiments 35 to 37,
   wherein the driver (336) is coupled between the spring (40) and the dose setting element (22) during dose setting to transfer the energy from the dose setting element (22) to the spring (40).
39. The mechanism (354, 610, 620, 630, 640) of at least one of embodiments 35 to 38,
   wherein the driver (336) is rotationally fixed with respect to the nut (38) when the mechanism (354, 610, 620, 630, 640) is in the dose setting state and rotationally movable with respect to the nut (38) when the mechanism (354, 610, 620, 630, 640) is in the dose delivery state.
40. The mechanism (354, 610, 620, 630, 640) of at least one of embodiments 35 to 39,
   wherein the driver (336) is rotationally fixed with respect to the dose setting element (22) when the mechanism (354, 610, 620, 630, 640) is in the dose setting state and rotationally movable with respect to the dose setting element (22) when the mechanism (354, 610, 620, 630, 640) is in the dose delivery state.
41. The mechanism (354, 610, 620, 630, 640) of at least one of embodiments 35 to 40,
   wherein the driver (336) is rotationally movable with respect to the housing (332) during dose setting and dose delivery.
42. The mechanism (354, 610, 620, 630, 640) of at least one of embodiments 35 to 41,
   wherein the mechanism (354, 610, 620, 630, 640) comprises a drive thread (337) that couples the driver (336) to the housing (332),
   wherein the drive thread (337) converts a torque provided by the spring (40) into axial movement of the driver (336).
43. The mechanism (354, 610, 620, 630, 640) of embodiment 42,
   wherein the driver (336) threadedly engages the housing (332) via the drive thread (337).
44. The mechanism (354, 610, 620, 630, 640) of at least one of embodiments 35 to 43 and at least one of embodiments 9 to 17,
   wherein the driver (336) is coupled between the nut (38) and the dosing member (323),
   wherein, for example, the driver (336) engages with the dosing member (323).
45. The mechanism (354, 610, 620, 630, 640) of at least one of embodiments 35 to 44 and at least one of embodiments 9 to 17,
   wherein the driver (336) is rotationally fixed and/or axially movable with respect to the dosing member (323).
46. The mechanism (354, 610, 620, 630, 640) of at least one of the preceding embodiments,
   further comprising a dose definition mechanism (115),
   wherein the dose definition mechanism (115) acts between the dose setting element (22) and the housing (332) during dose setting,
   wherein the dose definition mechanism (115) has at least one dose stop (118,119) and a counter element (116),
   wherein the counter element (116) is configured to rotate with respect to the dose stop (118,119) when the dose setting element (22) rotates during dose setting,
   wherein the counter element (116) is configured to engage the dose stop (118,119) when the dose has been set.
47. The mechanism (354, 610, 620, 630, 640) of embodiment 46,
   wherein engagement of the counter element (116) with the dose stop (118,119) prevents the spring (40) from releasing the energy stored upon rotation of the dose setting element (22).
48. The mechanism (354, 610, 620, 630, 640) of at least one of embodiments 46 and 47,
   wherein the counter element (116) is configured to disengage from the dose stop (118,119) upon transfer of the mechanism (354, 610, 620, 630, 640) from the dose setting state into the dose delivery state.
49. The mechanism (354, 610, 620, 630, 640) of at least embodiment 48,
   wherein the counter element (116) is configured to disengage from the dose stop (118,119) by axially moving with respect to the dose stop (118,119).
50. The mechanism (354, 610, 620, 630, 640) of at least one of embodiments 46 to 49,
   wherein one of the dose stop (118,119) and the counter element (116) is rotationally fixed with respect to the housing (332).
51. The mechanism (354, 610, 620, 630, 640) of at least embodiment 50,
   wherein the one of the dose stop (118,119) and the counter element (116) is axially movable with respect to the dose setting element (22).
52. The mechanism (354, 610, 620, 630, 640) of at least one of embodiments 50 and 51,
   wherein the one of the dose stop (118,119) and the counter element (116) is axially fixed with respect to the button (318).
53. The mechanism (354, 610, 620, 630, 640) of at least one of embodiments 50 and 51,
   wherein the one of the dose stop (118,119) and the counter element (116) is axially fixed with respect to the housing (332).
54. The mechanism (354, 610, 620, 630, 640) of at least one of embodiments 50 to 53,
   wherein the one of the dose stop (118,119) and the counter element (116) is fixed to an outer housing part (360, 540) of the mechanism (354, 610, 620, 630, 640).
55. The mechanism (354, 610, 620, 630, 640) of at least one of embodiments 50 to 54 and at least one of embodiments 9 to 17,
   wherein the other one of the dose stop (118,119) and the counter element (116) is rotationally fixed with respect to the dosing member (323).
56. The mechanism (354, 610, 620, 630, 640) of at least embodiment 55,
   wherein the other one of the dose stop (118,119) and the counter element (116) is axially fixed to the housing (332).
57. The mechanism (354, 610, 620, 630, 640) of at least embodiment 56,
   wherein the other one of the dose stop (118,119) and the counter element (116) is axially movable with respect to the button (318).
58. The mechanism (354, 610, 620, 630, 640) of at least embodiment 55,
   wherein the other one of the dose stop (118,119) and the counter element (116) is axially movable with respect to the housing (332).
59. The mechanism (354, 610, 620, 630, 640) of at least embodiment 58,
   wherein the other one of the dose stop (118,119) and the counter element (116) is axially fixed with respect to the button (318).
60. The mechanism (354, 610, 620, 630, 640) of at least one of the preceding embodiments,
   wherein the mechanism (354, 610, 620, 630, 640) comprises a blocking mechanism (400) having a first element (156) and a second element (116),
   wherein the first element (156) engages the second element (116) upon release of the button (318) during dose delivery to prevent a transfer of the mechanism (354, 610, 620, 630, 640) from the dose delivery state to the dose setting state.
61. The mechanism (354, 610, 620, 630, 640) of at least embodiment 60,
   wherein the first element (156) rotates with respect to the second element (116) in a first direction during dose setting and rotates in a second direction opposite the first direction during dose delivery.
62. The mechanism (354, 610, 620, 630, 640) of at least one of embodiments 60 and 61,
   wherein the first element (156) is configured as a circumferential rib that longitudinally extends around an axis of the housing (332),
   wherein the second element (116) is configured as a stop that travels along the circumferential rib during dose delivery.
63. The mechanism (354, 610, 620, 630, 640) of at least one of embodiments 60 to 62,
   wherein the second element (116) passes the first element (156) upon release of the button (318) at the end of dose delivery.
64. The mechanism (354, 610, 620, 630, 640) of at least one of embodiments 60 to 63,
   wherein the second element (116) passes the first element (156) upon transfer of the mechanism (354, 610, 620, 630, 640) from the dose setting state into the dose delivery state.
65. The mechanism (354, 610, 620, 630, 640) of embodiment 64,
   wherein the second element (116) passes through an opening (158) within the first element (156) upon transfer of the mechanism (354, 610, 620, 630, 640) from the dose setting state into the dose delivery state.
66. The mechanism (354, 610, 620, 630, 640) of embodiment 65,
   wherein the first element (156) comprises a recessed section (362) at the opening (158),
   wherein the second element (116) engages with the recessed section (362) upon release of the button (318) after having passed through the opening (158) during transferring the mechanism (354, 610, 620, 630, 640) from the dose setting state into the dose delivery state,
   wherein engagement of the second element (116) with the recessed section (362) prevents transition of the mechanism (354, 610, 620, 630, 640) back into the dose setting state.
67. The mechanism (354, 610, 620, 630, 640) of at least one of embodiments 60 to 66 and at least one of embodiments 46 to 59,
   wherein the first element (156) of the blocking mechanism (400) and one of the dose stop (118,119) and the counter element (116), such as the dose stop (118,119), are fixed to the same member (360, 540, 550) of the mechanism (354, 610, 620, 630, 640) and
   wherein the second element (116) of the blocking mechanism (400) and the other one of the dose stop (118,119) and the counter element (116), such as the counter element (116), are fixed to the same further member (24) of the mechanism (354, 610, 620, 630, 640).
68. The mechanism (354, 610, 620, 630, 640) of at least embodiment 67,
   wherein one of the first element (156) and second element (116) of the blocking mechanism (400) and one of the dose stop (118,119) and the counter element (116) are formed by a single element,
   wherein, for example, the second element (116) of the blocking mechanism (400) and the counter element (116) of the dose definition mechanism (115) are formed by the single element.
69. The mechanism (354, 610, 620, 630, 640) of at least one of the preceding embodiments,
   wherein the mechanism (354, 610, 620, 630, 640) comprises a maximum dose mechanism (440) that restrains further rotation of the dose setting element (22) upon dialing past a maximum dose setting,
   wherein the maximum dose mechanism (440) comprises a maximum dose stop (128) and a blocking part (124),
   wherein the blocking part (124) is configured to engage the maximum dose stop (128) upon dialing past the maximum dose setting.
70. The mechanism (354, 610, 620, 630, 640) of at least embodiment 69,
   wherein the maximum dose stop (128) and the blocking part (124) are configured to rotate with respect to each other during dose setting.
71. The mechanism (354, 610, 620, 630, 640) of at least embodiment 70,
   wherein the maximum dose stop (128) is configured as a radial stop and the blocking part (124) is configured to rotate against the maximum dose stop (128) upon dialing past the maximum dose.
72. The mechanism (354, 610, 620, 630, 640) of at least one of embodiments 69 to 71,
   wherein one of the maximum dose stop (128) and the blocking part (124) is rotationally fixed with respect to the housing (332).
73. The mechanism (354, 610, 620, 630, 640) of at least embodiment 72,
   wherein the one of the maximum dose stop (128) and the blocking part (124) is fixed to an outer housing part (333, 360, 540) of the mechanism (354, 610, 620, 630, 640).
74. The mechanism (354, 610, 620, 630, 640) of at least one of embodiments 72 and 73 and at least one of embodiments 9 to 17,
   wherein the other one of the maximum dose stop (128) and the blocking part (124) is rotationally fixed with respect to the dosing member (323).
75. The mechanism (354, 610, 620, 630, 640) of at least one of embodiments 74,
   wherein the other one of the maximum dose stop (128) and the blocking part (124) is fixed to a coupling member (24) that rotationally couples the dosing member (323) to the dose setting element (22) during dose setting.
76. The mechanism (354, 610, 620, 630, 640) of at least one of embodiments 69 to 75 and at least one of embodiments 46 to 59,
   wherein one of the dose stop (118,119) and the counter element (116), such as the dose stop (118,119), and one of the maximum dose stop (128) and the blocking part (124), such as the maximum dose stop (128), are fixed to the same member (333, 360, 540, 550) of the mechanism (354, 610, 620, 630, 640) and/or wherein the other one of the dose stop (118,119) and the counter element (116), such as the counter element (116), and the other one of the maximum dose stop (128) and the blocking part (124), such as the blocking part (124), are fixed to the same further member (24) of the mechanism (354, 610, 620, 630, 640).
77. The mechanism (354, 610, 620, 630, 640) of at least one of the preceding embodiments,
   wherein the mechanism (354, 610, 620, 630, 640) comprises a zero dose mechanism (450) that prevents further axial movement of the nut (38) at the end of dose delivery,
   wherein the zero dose mechanism (450) comprises a zero dose stop (126) and a further blocking part (124),
   wherein the further blocking part (124) is configured to engage the zero dose stop (126) at the end of dose delivery.
78. The mechanism (354, 610, 620, 630, 640) of at least embodiment 77,
   wherein the zero dose stop (126) and the further blocking part (124) are configured to rotate with respect to each other during dose delivery.
79. The mechanism (354, 610, 620, 630, 640) of at least embodiment 78,
   wherein the zero dose stop (126) is configured as a radial stop and the further blocking part (124) is configured to rotate against the zero dose stop (126) at the end of dose delivery.
80. The mechanism (354, 610, 620, 630, 640) of at least one of embodiments 77 to 79,
   wherein one of the zero dose stop (126) and the further blocking part (124) is rotationally fixed with respect to the housing (332).
81. The mechanism (354, 610, 620, 630, 640) of at least embodiment 80,
   wherein the one of the zero dose stop (126) and the further blocking part (124) is fixed to an outer housing part (360, 540) of the mechanism (354, 610, 620, 630, 640).
82. The mechanism (354, 610, 620, 630, 640) of at least one of embodiments 80 and 81 and at least one of embodiments 9 to 17,
   wherein the other one of the zero dose stop (126) and the further blocking part (124) is rotationally fixed with respect to the dosing member (323).
83. The mechanism (354, 610, 620, 630, 640) of at least one of embodiments 82,
   wherein the other one of the zero dose stop (126) and the further blocking part (124) is fixed to a coupling member (24) that rotationally couples the dosing member (323) to the dose setting member during dose setting.
84. The mechanism (354, 610, 620, 630, 640) of at least one of embodiments 77 to 83 and at least one of embodiments 46 to 59,
   wherein one of the dose stop (118,119) and the counter element (116), such as the dose stop (118,119), and one of the zero dose stop (126) and the further blocking part (124), such as the zero dose stop (126), are fixed to the same member (360, 540, 550) of the mechanism (354, 610, 620, 630, 640) and/or
   wherein the other one of the dose stop (118,119) and the counter element (116), such as the counter element (116), and the other one of the zero dose stop (126) and the blocking part (124), such as the blocking part (124), are fixed to the same further member of the mechanism (354, 610, 620, 630, 640).
85. The mechanism (354, 610, 620, 630, 640) of at least one of embodiments 77 to 84 and at least one of embodiments 69 to 76,
   wherein one of the maximum dose stop (128) and the blocking part (124) of the maximum dose mechanism (440) and one of the zero dose stop (126) and the further blocking part (124) of the zero dose mechanism (450), such as the maximum dose stop (128) and the zero dose stop (126), are fixed to the same member (360, 540, 550) of the mechanism (354, 610, 620, 630, 640),
   wherein the other one of the maximum dose stop (128) and the blocking part (124) of the maximum dose mechanism (440) and the other one of the zero dose stop (126) and the further blocking part (124) of the zero dose mechanism (450), such as the blocking part (124) and the further blocking part (124), are fixed to the same further member (24) of the mechanism (354, 610, 620, 630, 640).
86. The mechanism (354, 610, 620, 630, 640) of at least one of embodiments 77 to 85 and at least one of embodiments 69 to 76,
   wherein the blocking part (124) of the maximum dose mechanism (440) forms the further blocking part (124) of the minimum dose mechanism (450).
87. The mechanism (354, 610, 620, 630, 640) of at least one of the preceding embodiments,
   wherein the mechanism (354, 610, 620, 630, 640) comprises a break (390) for stopping movement of the nut (38) upon release of the button (318) during dose delivery.
88. The mechanism (354, 610, 620, 630, 640) of at least embodiment 87,
   wherein the break (390) comprises a first break part (156) and a second break part (124) that engages the first break part (156) upon release of the button (318) during dose delivery.
89. The mechanism (354, 610, 620, 630, 640) of at least embodiment 88,
   wherein the first break part (156) rotates with respect to the second break part (124) in a first direction during dose setting and rotates in a second direction opposite the first direction during dose delivery.
90. The mechanism (354, 610, 620, 630, 640) of at least embodiment 88 and 89,
   wherein the first break part (156) is configured as a circumferential rib that longitudinally extends around an axis of the housing (332),
   wherein the second break part (124) is configured as a stop that travels along the circumferential rib during dose delivery.
91. The mechanism (354, 610, 620, 630, 640) of at least embodiment 88 to 90,
   wherein the second break part (124) frictionally locks to the first break part (156) upon release of the button (318) during dose delivery.
92. The mechanism (354, 610, 620, 630, 640) of at least embodiment 88 to 91,
   wherein the first break part (156) comprises a plurality of grooves (367),
   wherein the second break part (124) is configured to engage with at least one of the grooves (367) upon release of the button (318) during dose delivery.
93. The mechanism (354, 610, 620, 630, 640) of at least one of embodiments 60 to 68 and at least one of embodiments 88 to 92,
   wherein the first element (156) of the blocking mechanism (400) forms the first break part (156) of the break (390) and/or
   wherein the second element (116) of the blocking mechanism (400) forms the second break part (124) of the break (390).
94. The mechanism (354, 610, 620, 630, 640) of at least one of the preceding embodiments,
   further comprising a clutch (113),
   wherein the clutch (113) rotationally locks the nut (38) to the piston rod (44) during dose delivery and rotationally releases the nut (38) from the piston rod (44) during dose setting.
95. The mechanism (354, 610, 620, 630, 640) of at least embodiment 94,
   wherein the clutch (113) rotationally fixes the nut (38) to the piston rod (44) during dose delivery via the housing (332) and, for example, via the dose setting element (22) and/or the button (318).
96. The mechanism (354, 610, 620, 630, 640) of at least one of embodiments 94 and 95,
   wherein the clutch (113) acts between the button (318) and the housing (332).
97. The mechanism (354, 610, 620, 630, 640) of at least one of embodiments 94 to 96,
   wherein the clutch (113) has a first engaging part (114, 561) that is rotationally fixed to the housing (332) and a second engaging part (108, 562) that is rotationally fixed to the button (318),
   wherein the first engaging part (114, 561) is configured to move into engagement with the second engaging part (108, 562) to rotationally lock the nut (38) to the piston rod (44).
98. The mechanism (354, 610, 620, 630, 640) of at least one of the preceding embodiments,
   comprising a further clutch (107),
   wherein the further clutch (107) rotationally locks the dose setting element (22) to one end of the spring (40) during dose setting and decouples the dose setting element (22) from the one end of the spring (40) during dose delivery,
   wherein the further clutch (107) has a further first engaging part (110, 565) and a further second engaging part (108, 566),
   wherein the further first engaging part (110, 565) is configured to move into engagement with the further second engaging part (108, 566) to rotationally lock the dose setting element (22) to the one end of the spring (40).
99. The mechanism (354, 610, 620, 630, 640) of at least embodiment 98,
   wherein one of the further first engaging part (110, 565) and the further second engaging part (108, 566) is rotationally and axially fixed to the button (318).
100. The mechanism (354, 610, 620, 630, 640) of at least one of embodiments 98 and 99,
   wherein one of the further first engaging part (110, 565) and the further second engaging part (108, 566) is rotationally and axially fixed to the dose setting element (22).
101. The mechanism (354, 610, 620, 630, 640) of at least one of embodiments 98 to 100,
   wherein the dose setting element (22) is coupled to the further clutch (107) via the button (318).
102. The mechanism (354, 610, 620, 630, 640) of at least one of embodiments 98 to 101 and at least one of embodiments 9 to 17,
   wherein the further clutch (107) acts between the dosing member (323) and the dose setting element (22).
103. The mechanism (354, 610, 620, 630, 640) of at least embodiment 102,
   wherein one of the further first engaging part (110, 565) and the further second engaging part (108, 566) is rotationally fixed to the dosing member (323).
104. The mechanism (354, 610, 620, 630, 640) of at least embodiment 103,
   wherein the one of the further first engaging part (110, 565) and the further second engaging part (108, 566) is axially fixed to the dosing member (323).
105. The mechanism (354, 610, 620, 630, 640) of at least one of embodiments 102 to 104,
   wherein the further clutch (107) is located within the dosing member (323).
106. The mechanism (354, 610, 620, 630, 640) of at least one of embodiments 94 to 97 and at least one of embodiments 98 to 105,
   wherein the clutch (113) comprises a first engaging part (114, 561) that engages a second engaging part (108, 562) to rotationally fix the nut (38) to the piston rod (44) during dose delivery,
   wherein the second engaging part (108, 562) of the clutch (113) forms the further second engaging part (108, 566) of the further clutch (107).
107. The mechanism (354, 610, 620, 630, 640) of at least one of the preceding embodiments,
   wherein the nut (38) is threadedly connected to the piston rod (44), such as threadedly engaged with the piston rod (44).
108. The mechanism (354, 610, 620, 630, 640) of at least one of the preceding embodiments,
   wherein the nut (38) is rotationally movable with respect to the piston rod (44) when the mechanism (354, 610, 620, 630, 640) is in the dose setting state,
   wherein the nut (38) is rotationally fixed with respect to the piston rod (44) when the mechanism (354, 610, 620, 630, 640) is in the dose delivery state.
109. The mechanism (354, 610, 620, 630, 640) of at least one of the preceding embodiments,
   wherein the nut (38) is axially movable with respect to the piston rod (44) when the mechanism (354, 610, 620, 630, 640) is in the dose setting state,
   wherein the nut (38) is axially fixed with respect to the piston rod (44) when the mechanism (354, 610, 620, 630, 640) is in the dose delivery state.
110. The mechanism (354, 610, 620, 630, 640) of at least one of the preceding embodiments,
   wherein the dose setting element (22) is rotatable over less than a full revolution during dose setting.
111. The mechanism (354, 610, 620, 630, 640) of at least one of the preceding embodiments,
   wherein the spring (40) is configured as a torsion spring (40).
112. A medicament delivery device (300) comprising the mechanism (354, 610, 620, 630, 640) of at least one of the preceding embodiments.

## Claims

1. A mechanism (354, 610, 620, 630, 640) for an automatic medicament delivery device (300) comprising:
a housing (332);
a dose setting element (22);
a button (318);
a piston rod (44) that is rotationally fixed and axially movable with respect to the housing (332);
a nut (38); and
a spring (40),
wherein the dose setting element (22) is configured to be gripped by a user of the device to set a dose to be delivered by rotation of the dose setting element (22) with respect to the housing (332) when the mechanism (354, 610, 620, 630, 640) is in a dose setting state,
wherein the rotation of the dose setting element (22) stores energy in the spring (40),
wherein the rotation of the dose setting element (22) causes the nut (38) to move by a dose distance (3) in a proximal direction (1) with respect to the piston rod (44), the dose distance (3) being proportional to the dose,
wherein the mechanism (354, 610, 620, 630, 640) is configured to switch from the dose setting state into a dose delivery state upon moving the button (318) with respect to the housing (332),
wherein a transfer into the dose delivery state couples the spring (40) to the nut (38) to cause automatic movement of the nut (38) in a proximal direction (1) by releasing the energy stored during dose setting,
wherein the piston rod (44) is configured to move together with the nut (38) by the dose distance (3) in the proximal direction (1) to deliver the dose when the mechanism (354, 610, 620, 630, 640) is in the dose delivery state,
wherein the dose setting element (22) is configured to remain axially stationary with respect to the housing (332) when the piston rod (44) moves in the proximal direction (1) to deliver the dose.

2. The mechanism (354, 610, 620, 630, 640) of claim 1,
further comprising a dosing member (323),
wherein the dosing member (323) is configured to rotate with respect to the housing (332) during dose setting and to rotate with respect to the housing (332) during dose delivery,
wherein the dosing member (323) is configured to remain axially stationary with respect to the housing (332) during dose delivery.

3. The mechanism (354, 610, 620, 630, 640) of claim 2,
wherein the dosing member (323) is configured to remain axially stationary with respect to the housing (332) while storing the energy in the spring (40) during dose setting.

4. The mechanism (354, 610, 620, 630, 640) of at least one of claims 2 and 3,
wherein the dosing member (323) is coupled between the spring (40) and the dose setting element (22) during dose setting to transfer the energy from the dose setting element (22) to the spring (40).

5. The mechanism (354, 610, 620, 630, 640) of at least one of claims 2 to 4,
wherein the dosing member (323) comprises a label (168) to visually indicate setting of the dose.

6. The mechanism (354, 610, 620, 630, 640) of at least one of the preceding claims,
further comprising a blocker (430),
wherein the blocker (430) acts between the button (318) and the housing (332),
wherein the blocker (430) blocks the button (318) from moving axially with respect to the housing (332) in the distal direction.

7. The mechanism (354, 610, 620, 630, 640) of at least claim 6 and at least one of claims 2 to 5,
wherein the blocker (430) acts between the dosing member (323) and the housing (332),
wherein, for example, the blocker (430) is provided at one of the housing (332) and the dosing member (323) and engages with the other one of the housing (332) and the dosing member (323).

8. The mechanism (354, 610, 620, 630, 640) of at least claim 6 and 7,
wherein the blocker (430) is configured as a one-way blocker (430),
wherein the blocker (430) allows relative axial movement between the housing (332) and a counter element (116) in a first direction and blocks relative axial movement between the housing (332) and the counter element (116) in a second direction opposite the first direction.

9. The mechanism (354, 610, 620, 630, 640) of claim 8,
wherein the blocker (430) is configured as a flexible element that is configured to snap into a blocking position upon assembly of the counter element (116) to the housing (332).

10. The mechanism (354, 610, 620, 630, 640) of at least one of claims 8 and 9 and at least one of claims 2 to 5,
wherein the counter element (116) is part of the dosing member (323).

11. The mechanism (354, 610, 620, 630, 640) of at least one of the preceding claims,
wherein the mechanism (354, 610, 620, 630, 640) comprises a stop (373, 374) to limit proximal movement of the button (318) during dose delivery.

12. The mechanism (354, 610, 620, 630, 640) of at least claim 11 and at least one of claims 2 to 5,
wherein the dosing member (323) is coupled in between the stop (374) and the housing (332).

13. The mechanism (354, 610, 620, 630, 640) of claim 11,
wherein the stop (373) is provided at the housing (332), such as an inside surface (371) of the housing (332).

14. The mechanism (354, 610, 620, 630, 640) of claim 13,
wherein the stop (373) acts between the housing (332) and a housing connector (360) that is axially fixed to the button (318).

15. The mechanism (354, 610, 620, 630, 640) of at least one of claims 11 to 14,
wherein the stop (373, 374) is configured as an axial stop.
